# EUROPÄISCHE PATENTSCHRIFT

(11) **EP 1 149 167 B1**
(45) Veröffentlichungstag und Bekanntmachung des Hinweises auf die Patenterteilung: **03.03.2004**
(21) Anmeldenummer: 99963478.5
(22) Anmeldetag: 03.12.1999
(51) Int. Cl.: C12N 15/49, C12N 15/85, C12N 1/21, C12N 5/10, C07K 14/16, A61K 39/12, A61K 39/21, A61P 31/18, A61P 31/12

(54) **VIRUS-VAKZINE**
VIRAL VACCINE
VIRUS-VACCINS

(30) Priorität: 12.02.1999 DE 19907485
(43) Veröffentlichungstag der Anmeldung: 31.10.2001
(73) Patentinhaber: Strathmann AG & Co., 22459 Hamburg (DE)
(72) Erfinder: SCHREIBER, Michael, D-22301 Hamburg (DE)
(74) Vertreter: UEXKÜLL & STOLBERG
(86) Internationale Anmeldenummer: PCT/EP1999/009759
(87) Internationale Veröffentlichungsnummer: WO 2000/047223

(56) Entgegenhaltungen:
- EP-A- 0 327 180
- WO-A-95/04147
- FR-A- 2 677 363
- US-A- 5 846 546
- US-A- 5 851 813
- NEURATH A R ET AL: "Antibody responses of chimpanzees immunized with synthetic peptides corresponding to full-length V3 hypervariable loops of HIV-1 envelope glycoproteins." AIDS RESEARCH AND HUMAN RETROVIRUSES, (1991 OCT) 7 (10) 813-23. , XP000938405
- SCHREIBER M ET AL: "The V3 -directed immune response in natural human immunodeficiency virus type 1 infection is predominantly directed against a variable, discontinuous epitope presented by the gp120 V3 domain." JOURNAL OF VIROLOGY, (1997 DEC) 71 (12) 9198-205. , XP002145193
- FOMSGAARD A: "HIV-1 DNA vaccines." IMMUNOLOGY LETTERS, (1999 JAN) 65 (1-2) 127-31. REF: 36 , XP000857026

## Beschreibung

Die vorliegende Erfindung betrifft eine pharmazeutische Zusammensetzung bzw. eine Vakzine, die eine Mischung viraler Protein-Moleküle umfaßt, die Sequenzvarianten eines einzigen viralen Proteins oder eines Teils desselben sind, wobei die Mischung ≥ 10² Sequenzvarianten enthält, die durch Expression einer Plasmid-DNA-Mischung erhältlich ist, die aufgrund der Variation von Nukleotidpositionen zufallsverteilte Sequenzkombinationen aufweist. Die Erfindung betrifft ferner u.a. eine DNA-Vakzine, die für eine Mischung strukturell unterschiedlicher Virus-Proteine kodiert, wobei die Vakzine eine Mischung von Sequenzvarianten eines viralen DNA-Moleküls oder eines Teils desselben enthält, die Sequenzvarianten eines viralen Proteins oder eines Teils kodieren, wobei die Mischung ≥ 10² DNA-Moleküle enthält, die sich in ihrer Nukleinsäuresequenz voneinander unterscheiden, wobei die Mischung aufgrund der Variation von Nukleotidpositionen zufallsverteilte Sequenzkombinationen aufweist. Gemäß einer bevorzugten Ausführungsform der Erfindung handelt es sich bei den viralen Proteinen um Sequenzvarianten des GP120-Proteins des Humanen Immundefizienzvirus (HIV), die sich jeweils in ihrer Aminosäuresequenz im Bereich der V2-Scheife und/oder der V3-Schleife, vorzugsweise sowohl der V2- als auch der V3-Schleife, voneinander unterscheiden. Die Erfindung betrifft ferner die Herstellung der Virus-Vakzinen einschließlich der damit im Zusammenhang stehenden Zwischenstufen bzw. Konstrukte, Herstellungsverfahren und Verwendungen.

Bei vielen viralen Infektionen, insbesondere bei der HIV-1-Infektion, beobachtet man eine starke Immunabwehr, die in der Lage ist, das Virus über einen Zeitraum von mehreren Jahren zu kontrollieren. Den Zeitraum, in dem das virus kontrolliert wird und keine Krankheitssymptome beobachtet werden, bezeichnet man als die asymptomatische Phase der (HIV-) Erkrankung. Im Verlauf der Erkrankung entstehen immer wieder neue Virusvarianten. Dadurch ist es dem Virus möglich, der Immunabwehr des Menschen zu entkommen und immer wieder neue Abwehrzellen des Immunsystems zu infizieren (vgl. M. Schreiber et al., J. Virol. 68 Nr. 6 (1994) 3908-3916; J. Gen. Virol. 77 (1996) 2403-2414; Clin. Exp. Immunol. 107 (1997) 15-20; J. Virol. 71 Nr. 12 (1997) 9198-9205).

Im Stand der Technik sind zur Behandlung viraler Erkrankungen, wie z. B. der Poliovirus- (Horaud F et al., Biologicals, 1993, 21:311-316), Hantavirus- (Ulrich R et al., 1998 Vaccine 16:272-280; Schmaljohn CS et al., 1992 Vaccine 10:10-13), Lassavirus- (Morrison HG et al., 1989 Virology 171:179-188; Clegg JC et al., 1987 Lancet 2:186-188), Hepatitis-A-Virus- (Clemens et al., 1995 J. Infect. Dis. 171:Suppl1:S44-S49; Andre et al., 1990 Prog. Med. Virol. 37:72-95) und Hepatitis-B-Virus-Infektion (McAleer et al., 1984 Nature 307:178-180) aber auch der HIV-Infektion (Egan et al., 1995 J. Infect. Dis. 171:1623-1627, Kovac et al., 1993 J. Clin. Invest. 92:919-928) nur einzelne, genetisch unveränderte, spezifische Virus-Antigene oder einzelne inaktivierte Virusstämme zu Untersuchungen geeigneter Vakzinierungsstrategien eingesetzt worden. Im Falle von HIV wurden bisher beispielsweise die externen Hüllproteine von zwei Virusstämmen für Impfstoffexperimente am Menschen hergestellt (MN und SF2) (Zolla-Pazner et al., J. Infect. Dis. 178 (1998) 1502-1506). Beide GP120-Moleküle unterscheiden sich in der Aminosäuresequenz, besonders aber in der Aminosäuresequenz der variablen Bereiche, wie z.B. der V3-Schleife (V3-Loop). Die beiden Virusstämme haben aufgrund der verschiedenen V3-Loop-Sequenzen unterschiedliche phänotypische Eigenschaften. Der HIV-1 MN-Stamm ist eine Virusvariante, die bevorzugt Makrophagen und Zellen, die den viralen Korezeptor CCR5 besitzen, infiziert. Viren des SF2-Typs dagegen vermehren sich bevorzugt in T-Zellen und benutzen den viralen Korezeptor CXCR4. Man nennt solche Viren daher auch T-Zelltrophe (z.B. HIV-Stamm SF2) oder Makrophagotrophe (z.B. HIV-Stamm MN) Virusvarianten. Im Schimpansen sind mit diesen beiden GP120-Varianten Impfexperimente durchgeführt worden. In diesen Experimenten ist gezeigt worden, daß man eine Immunantwort induzieren kann, die nicht nur gegen die beiden HIV-Stämme MN und SF2 schützt, sondern auch in der Lage ist, die Infektion mit anderen Virusstämmen, aber auch mit HIV-1-Patientenisolaten zu verhindern. Im Unterschied zum Schimpansen hat man beim Menschen bisher nur eine der beiden GP120-Varianten für Impfexperimente eingesetzt. Sowohl MN GP120 als auch SF2 GP120 schützen nicht mit Sicherheit vor einer Infektion durch eine Virusvariante wie sie in einem HIV-1-Infizierten vorkommt (Patienten-Isolat oder Wildtyp-Virus). Einen Überblick über den Stand der Forschung im Zusammenhang mit der Vakzinierung mit GP120-Hüllprotein bietet J.A. Levy in "*HIV and the Pathogenesis of AIDS*", Herausgeber: Jay A. Levy, Kapitel 15, 2. Auflage, ASM Press Washington, D.C., 1998).

Der Nachteil der im Stand der Technik bislang diskutierten bzw. untersuchten Vakzinierungsstrategien besteht unter anderem darin, daß man mit den eingesetzten Vakzinen nicht in der Lage ist, die Entstehung immer neuer Virusvarianten im Verlauf der Virus-Erkrankung zu verhindern.

Aufgabe der vorliegenden Erfindung ist es daher, eine Vakzine zur verfügung zu stellen, die insbesondere in der-Lage ist, die Entstehung neuer Virusvarianten im Verlauf einer Virus-Erkrankung zu verhindern bzw. deutlich zu vermindern und die Möglichkeiten einzuschränken bzw. zu verhindern, daß das Virus der Immunabwehr des Menschen entkommen und immer wieder neue Abwehrzellen des Immunsystems infizieren kann.

Zur Lösung der Aufgabe werden erfindungsgemäß die in den nachfolgenden Ansprüchen zum Ausdruck kommenden Gegenstände vorgeschlagen.

Gegenstand der vorliegenden Erfindung ist somit unter anderem eine Protein-Vakzine, die eine Mischung viraler Protein-Moleküle umfaßt, wobei die Moleküle Sequenzvarianten eines einzigen viralen Proteins oder eines Teils desselben sind, wobei die Mischung ≥ 10² Sequenzvarianten enthält, die durch Expression einer Plasmid-DNA-Mischung erhältlich ist, die aufgrund der Variation von Nukleotidpositionen zufallsverteilte Sequenzkombinationen aufweist.

Unter Sequenzvarianten eines Proteins werden erfindungsgemäß solche Moleküle verstanden, die eine gegenüber einem nativen viralen Protein oder einem Teil (Fragment) desselben abgeleitete Aminosäuresequenzen aufweisen, wobei sich die Varianten dadurch voneinander unterscheiden, daß mindestens eine Aminosäure an beliebigen Stellen der Sequenz bzw. Teilen derselben ausgetauscht sein kann. vorzugsweise weisen die Sequenzvarianten mehrere Aminosäureaustausche an verschiedenen Stellen der Sequenz auf, die für die Produktion, aber auch die Bindung virusneutralisierender Antikörper verantwortlich sind. Die Zahl und Lage der ausgetauschten Aminosäuren hängt dabei von der Aminosäurevariabilität der Regionen des gp120 ab, die bei Zellkultur adaptierten und Wildtyp HIV-Isolaten beobachtet wird. Erfindungsgemäß weisen die Sequenzvarianten eine Heterogenität an mindestens zwei Aminosäurepositionen der Sequenz oder Teilen derselben auf. Besonders bevorzugt ist eine Heterogenität an drei bis acht und vorzugsweise an mehr als acht Aminosäurepositionen, wobei alle vorkommenden Aminosäuren an diesen Positionen möglich sind. Aus der Kombination der verschiedenen Aminosäuren, die je Position möglich sind, ergibt sich die Anzahl der möglichen Sequenzvarianten, die in ihrer Gesamtheit die Vakzine darstellen.

Die Erfindung betrifft eine Vakzine, die eine Mischung von ≥ 10² Sequenzvarianten umfaßt, d.h. eine Mischung von mehr als 10² Molekülen unterschiedlicher Aminosäuresequenz (Homologen). Besonders bevorzugt ist eine Vakzine, die ≥ 10³ und vorzugsweise ≥ 10⁴ Sequenzvarianten umfaßt.

Im Rahmen der vorliegenden Erfindung wird davon ausgegangen, daß die Vakzine neben den Sequenzvarianten auch das Protein als solches umfassen kann, von dem die Sequenzvarianten abgeleitet sind.

Erfindungsgemäß wird somit ein Wirkstoff gegen Viren bereitgestellt, der virusspezifische Proteine umfaßt, die sich alle in ihren Sequenzen bzw. Teilen derselben unterscheiden. Um dies zu erreichen, wird das Protein-kodierende Gen neu synthetisiert, um neue Schnittstellen für DNA-schneidende Enzyme zu erzeugen, um den Austausch spezifischer Regionen zu erlauben. Durch chemische Synthese von DNA-Fragmenten, die für den betreffenden Proteinabschnitt kodieren, wird dann eine Genbank für die Protein-kodierende Sequenz hergestellt. Die Expressionsvektoren, die das Protein kodieren, werden dann als Mischung in Zellen transfiziert. Aus diesen Protein-produzierenden Zellen kann dann die Mischung der verschiedenen viralen Proteine isoliert werden, die erfindungsgemäß den bevorzugten Wirkstoff darstellt.

Im Falle von HIV als viraler Erkrankung kommt es durch die chronische Infektion und die damit verbundene kontinuierliche Schädigung des Immunsystems im Verlauf der Erkrankung zum vollständigen Verlust der spezifischen Virusabwehr. Zur spezifischen Virusabwehr gehören neutralisierende Antikörper, die die Eigenschaft besitzen, mit bestimmten antigenen Strukturen eine spezifische, sehr feste Bindung einzugehen. Dadurch werden fremde Antigene markiert und deren Interaktion mit z.B. Virus-Rezeptoren blockiert. Eine solche spezifische Virusabwehr sind neutralisierende Antikörper gegen den V3-Loop des externen GP120-Hüllproteins. Solche anti-V3-Loop-Antikörper sind in der Lage, die Infektion von Zellen zu verhindern. Im Tiermodell ist gezeigt worden, daß sich durch die Verabreichung eines bestimmten monoklonalen V3-Loop-Antikörpers eine Infektion mit HIV-1 verhindern lässt. Durch die Gabe des gleichen monoklonalen Antikörpers war es ebenfalls möglich, eine bestehende Infektion zu heilen.

Im Unterschied zu experimentellen Systemen beobachtet man jedoch im natürlichen Verlauf der HIV-Infektion die Entwicklung immer neuer Virusvarianten. So finden sich zu einem Zeitpunkt in einem einzigen Patienten hunderte von verschiedenen V3-Loop-Varianten, da die Variation des HIV-1 gerade im V3-Loop besonders hoch ist. Der V3-Loop ist eine wichtige dominante antigene Domäne des GP120. Daher wird gegen jeden V3-Loop eine sehr spezifische humorale Immunantwort gebildet. Resultat der Induktion einer hoch spezifischen Immunantwort gegen den V3-Loop ist, daß neutralisierende Antikörper gegen den V3-Loop der HIV-1-Variante A nicht in der Lage sind, die Variante B zu neutralisieren und umgekehrt (Schreiber et al., J. Virol. 68 (1994) 3908-16, Abrahamsson et al., 4 (1990) 107-12).

In der asymptomatischen Phase der HIV-Erkrankung werden die zellfreien Virusvarianten im Serum durch Antikörper neutralisiert. Erst zu einem späteren Zeitpunkt können Virusvarianten im Serum beobachtet werden, die sich der autologen Neutralisation entziehen. Diese V3-Loop-Escape-Varianten werden nicht mehr durch V3-Loop-Antikörper erkannt und daher auch nicht mehr neutralisiert. Alle anderen Virusvarianten, die im Serum der Patienten gefunden werden, werden aber durch autologe V3-Loop-Antikörper erkannt. Dies deutet darauf hin, daß im Verlauf der Erkrankung Virusvarianten auftreten, gegen die keine V3-Loop-Antikörper existieren. Überpüft man den Antikörpergehalt von Serumproben eines Patienten über einen Zeitraum von mehreren Jahren, so kann man in den Serumproben, die am Anfang der asymptomatischen Phase entnommen wurden, die V3-Loop-Antikörper gegen die im späteren Stadium auftretende V3-Loop-Escape-Variante nachweisen. Es kommt daher nicht wie bei anderen Infektionserkrankungen zum klassischen Escape des Erregers durch immer neue antigene Variation, sondern zum Abschalten der neutralisierenden Immunantwort gegen eines der im Patienten replizierenden Viren. Das beoachtete Fehlen der neutralisierenden V3-Loop-Antikörper ist daher das Resultat eines kontinuierlichen Verlusts der typenspezifischen V3-Loop-Antikörper. Durch den Verlust der neutralisierenden Antikörper kann sich das entsprechende Virus vermehren, was zum Anstieg der Viruslast im Serum der Patienten führt. Im Verlauf der Erkrankung beobachtet man ebenfalls den Anstieg der Viruslast im Lymphknoten (Chun et al., Nature 387 (1997) 183-188).

Ein Virus, welches sich im Patienten gegen das Immunsystem durchsetzt und sich zur dominierenden Virusvariante entwickelt, muß zwangsläufig alle antiviralen Barrieren überwinden, die das Immunsystem bereitstellt. Neben neutralisierenden Antikörpern sind cytotoxische T-Zellen ebenfalls in der Lage die Virusvermehrung zu unterdrücken. Cytotoxische T-Zellen bewirken den Tod HIV infizierter CD4+ T-Zellen. Neben dem Verlust der neutralisierenden Antikörper wird auch der Verlust solcher cytotoxischer T-Zellen gegen HIV infizierte Zellen beobachtet (Zerhoui et al., Thymus 24 (1997) 203-219; Gould et al., Semin Cell Dev Biol 9 (1998) 321-328; Wagner et al., J Gen Virol 74 (1993) 1261-1269; O'Toole et al., AIDS Res Hum Retroviruses 8 (1992) 1361-1368; Shearer et al., 137 (1986) 2514-2521). Daher ist die Aufgabe einer heterologen Vakzine, der Mischung von vielen verschiedenen GP120 Hüllproteinen des HIV, sowohl neutralisierende Antikörper als auch cytotoxische T-Zellen gegen viele verschiedenen Virusvarianten zu induzieren bzw. zu aktivieren.

Wie schon ausgeführt, zeichnet sich die HIV-Erkrankung dadurch aus, daß sich das Virus ständig verändert. Dies wird durch die Fehlerrate des viralen Enzyms Reverse-Transkriptase bewirkt, welches bei der Vermehrung der viralen Erbinformation Fehler macht. Dadurch werden Mutanten erzeugt, die einerseits aufgrund ihrer biologischen Eigenschaften und der antiviralen Wirkung des humanen Immunsystems selektiert werden. Wie bei HIV so gibt es auch weitere Krankheitserreger, die ihre Erbinformation ohne Fehlerkorrektur umschreiben, was zur Ausbildung von vielen Varianten führen kann. Dazu zählen neben Hepatitis A-, B- und C-Viren auch Hanta- und Lassa-Viren. So beobachtet man bei Hepatitis B geimpften Personen, daß es bei ca 2% zum Impfversagen kommt. Bei diesen 2% werden Hepatitis B "escape" Varianten gefunden, die sich nicht durch die durch den Impfstoff induzierte Immunantwort unterdrücken lassen.

Das Entstehen von Impfstoff-, Therapie- und Immun-Escape-Varianten beruht auf der genetischen Variabilität dieser Viren (Blum, Int J Clin Lab Res 27 (1997) 213-214; Jongerius et al., Transfusion 38 (1998) 56-59).

Im Stand der Technik ist bislang nicht bekannt, wie einem solchen Verlust Virus-neutralisierender Antikörper entgegengewirkt werden kann. Die bisher eingeschlagenen Strategien waren jeweils nur punktuell erfolgreich, d.h. in Bezug auf die Bildung und den Erhalt einzelner variantenspezifischer Antikörper gegen bestimmte Viren, dem Verlust typenspezifischer Antikörper gegen die Viel-falt der vom Virus im Verlauf der Erkrankung gebildeten Proteinvarianten können die bislang vorgeschlagenen Behandlungsmethoden in Form von antiviralen Medikamenten oder Impfstoffen auf der Basis einer Memo-Substanz jedoch nicht entgegenwirken.

Durch die vorliegende Erfindung einer heterogenen Vakzine ist es nunmehr erstmals möglich, den Verlust neutralisierender V3-Antikörper zu verhindern oder diesem Verlust zumindest deutlich entgegenzuwirken.

Auf der Basis der vorliegenden Erfindung wird erstmals eine immunrekonstitutive Behandlung von Virus-infizierten Patienten, insbesondere von HIV-1-Infizierten, zur Verfügung gestellt, bei der man das Immunsystem in einer Art und Weise aktiviert, daß die natürlich erworbene Immunabwehr gegen die Virus-Population der verschiedenen Virusvarianten regeneriert und neu stimuliert wird, um so die im Patienten vorhandenen Virusvarianten an ihrer Vermehrung zu hindern.

Die Erfindung basiert kurz gesagt auf dem Konzept, für einen Wirkstoff gegen HIV-1 ein Gemisch von GP120-Proteinen herzustellen, wobei sich diese GP120-Proteine z.B. entweder in der V2- oder der V3-Schleife oder gleichzeitig in den beiden variablen Domänen, d.h. dem V2-Loop und dem V3-Loop, unterscheiden. Um dies zu erreichen, muß das GP120-kodierende Gen neu synthetisiert werden unter Bildung neuer (monovalenter) Schnittstellen für DNA-schneidende Enzyme, die den spezifischen Austausch der z.B. V2- und V3-Region erlauben. Durch chemische Synthese von DNA-Fragmenten, die für den V2-Loop und den V3-Loop kodieren, wird dann eine V2/V3-Genbank für GP120 hergestellt. Die GP120-Expressionsvektoren werden schließlich als Mischung in Zellen transfiziert, und aus diesen GP120-produzierenden Zellen kann dann die Mischung der verschiedenen GP120-Proteine isoliert werden, die als Wirkstoff zur Prophylaxe und/oder Therapie der HIV-Erkrankung bzw. AIDS eingesetzt werden kann.

Die der vorliegenden Erfindung zugrundeliegende Überlegung besteht somit darin, für eine Immuntherapie oder einen Impfstoff gegen HIV-1 viele verschiedene GP120-Moleküle herzustellen, die V3-Loop-Sequenzen (und/oder V2-Loop-Sequenzen) tragen, wie sie auch bei Virusvarianten in Patienten identifiziert werden können.

Die Produktion einer Mischung von natürlichen Virusvarianten im Zellkultursystem ist sehr aufwendig. Beonders zu beachten ist, daß sich die Zusammensetzung einer Virus-Mischung verändert, da man Zellen in der Zellkultur einsetzen muß, die von HIV-1 infiziert werden können. Einige Virusvarianten, die selektive Vorteile besitzen, z.B. eine schnellere Wachstumskinetik, werden daher im Zellkultursystem schon nach wenigen Tagen dominieren und die anderen langsameren Virusvarianten verdrängen. Dies ist vor allen Dingen dann gegeben, wenn die verschiedenen Virusvarianten, die in den peripheren mononukleären Blutzellen (PBMC) oder im Serum von Patienten vorkommen, isoliert werden sollen. Da es nicht möglich ist, eine gleichbleibende Mischung von HIV-Varianten zu kultivieren, kann auch auf diese Weise keine entsprechende Mischung von GP120-Proteinen aus Viruskulturen produziert bzw. isoliert werden.

Daher wurde im Rahmen der vorliegenden Erfindung ein gentechnischer Ansatz für die Herstellung von HIV-1-Varianten und GP120-Varianten gewählt. Zur Herstellung von Virusvarianten und rekombinantem GP120 wurde ein Klonierungsystem konstruiert, das aus zwei Vektoren besteht. Zentraler Bestandteil beider Vektoren ist ein neues, chemisch synthetisiertes HIV-1 Hüllprotein-Gen (HIV-1 *env*-Gen), welches für das GP120-Protein kodiert. Ein Vektor enthält das gesamte Genom des HIV-1. Nach Transfektion dieses Vektors in Zellen produzieren die Zellen infektiöse Viruspartikel. Dies ermöglicht die Produktion einer Mischung von Virusvarianten, da Zellen verwendet werden können, die resistent gegen eine HIV-1-Infektion sind. Daher kann sich in einem solchen Zellkultursystem die Zusammensetzung der Virusmischung nicht aufgrund der biologischen Eigenschaften der Virusvarianten ändern. Der zweite Vektor ermöglicht die Expression von GP120-Varianten in eukaryontischen Zellen. Dieser Vektor dient direkt zur Herstellung des Impfstoffs (der Vakzine).

Für die gentechnische Herstellung und Expression der V3-Loop-GP120-Varianten in eukaryotischen Zellen wurde ein spezielles Genkonstrukt für HIV-1-*env* hergestellt, welches im nachfolgenden als Genkassette bezeichnet wird. Zur Herstellung der Genkassette wurde erfindungsgemäß die gesamte kodierende Sequenz des *env*-Gens chemisch synthetisiert. Mit Hilfe dieser Methode ist es möglich, die kodierende Sequenz des Gens beliebig zu verändern, wobei neue DNA-Erkennungssequenzen für DNA-schneidende Restriktionsenzyme in die *env*-Gensequenz eingebaut werden können. Dabei muß darauf geachtet werden, daß sich die Aminosäuresequenz des ursprünglichen GP120 (vorzugsweise der Stämme NL4-3 und PI-932) nicht ändert, während in der DNA-Sequenz des *env*-Gens hingegen neue Restriktionsschnittstellen entstehen. Gleichzeitig werden erfindungsgemäß Schnittstellen für Enzyme, die in der *env*-Sequenz mehrfach vorkommen, entfernt, so daß jeweils nur eine Schnittstelle für ein bestimmtes Restriktionsenzym, wie z.B. BglII, vorhanden ist. Gemäß einer bevorzugten Ausführungsform der Erfindung werden im *env*-Gen zehn neue, einmal-vorkommende (monovalente) Schnittstellen in Abständen von ca. 150 Basenpaaren eingefügt. Das so erzeugte neue *env*-Gen wird im Rahmen der vorliegenden Erfindung auch als Genkassette bezeichnet. Im Prinzip ist das neue *env*-Fragment einem Polylinker ähnlich.

Die Schnittstellen für die Restriktionsenzyme BstEII und BamHI begrenzen die erfindungsgemäß bevorzugte Genkassette (SEQ ID NO: 9). Um den Bereich des env-Gens austauschen zu können, sind beide Schnittstellen nur einmal im pBSCenvATG Expressionsvektor (SEQ ID NO: 10) für gp120 und im retroviralen Vektor pNL4-3 vorhanden. Der Vektor mit dem vom Hinterleger zugeteilten Bezugszeichen pBSCenv-V3 wurde am 6. Januar 1999 bei der DSMZ - Deutschen Sammlung von Mikroorganismen und Zellkulturen GmbH, Mascheroder Weg 1b, 38124 Braunschweig, Deutschland, unter der Zugriffsnummer DSM 12612 nach dem Budapester Vertrag hinterlegt.

Durch den Austausch des durch BstEII und BamHI begrenzten Genabschnitts lassen sich die neu hergestellten Abschnitte des *env*-Gens, die ebenfalls durch BstEII und BamHI begrenzt sind, in beide Vektoren klonieren. Das PstI/BclI V2-Loop und das BglII/ XbaI V3-Loop env-Fragment werden chemisch bzw. enzymatisch synthetisiert und anschließend in einen Standardvektor wie z.B. pUC 18 oder 19 kloniert. In diesem vektor wird das Fragment durch Sequenzierung überprüft und anschließend durch einen Pst/BclI oder BglII/XbaI Verdau aus dem Vektor geschnitten und in den pBSCenvATG und dann in den pNL4-3 Vektor überführt. Anstelle der Zwischenklonierung in einen Standardvektor (pUC 18, pUC 19 etc.) können die PstI/BclI V2-Loop- und BglII/XbaI V3-Loop-Fragmente auch direkt in pBSCenvATG kloniert werden.

Da sich in der Genkassette alle ca. 100 Basenpaare eine Schnittstelle für ein Restriktionsenzym befindet, können alle Bereiche des *env*-Gens, insbesondere der V3-Loop oder V2-Loop, gegen beliebige DNA-Fragmente ausgetauscht werden. Der für den V3-Loop kodierende Abschnitt läßt sich durch ein BglII-XbaI-Fragment, das eine Größe von 244 Basenpaaren besitzt (vgl. SEQ ID NO: 9; Nukleotide 708 bis 955), austauschen. Ziel ist es, die Genabschnitte, die für die variablen Loops des GP120-Proteins kodieren, gegen neue DNA-Fragmente auszutauschen. Die Fragmente werden synthetisch hergestellt und anschliessend in die envGenkassette kloniert. Wenn man bei der Synthese der V2-Loop- und V3-Loop-DNA-Fragmente an bestimmten Positionen die Sequenz variiert, d.h. alle vier Nukleotide gleichberechtigt einbaut oder an dieser Position das Nukleotid Inosin verwendet, lassen sich env-Genvarianten mit einer vorgegebenen Variation der dadurch kodierten Aminosäuresequenz herstellen. Die V3-Loop-Sequenzen von Patientenisolaten sind Ausgangsbasis für die Herstellung der GP120-Mischung. Werden diese Varianten in die *gp120*-Genkassette kloniert, so können die entsprechenden GP120-Proteine mit den variablen antigenen Domänen in eukaryontischen Zellen exprimiert werden. Für die variation der V3-Loop Region des *env*-Gens stehen Sequenzdaten von HIV-1 Patientenisolaten zur Verfügung. Patientenisolate sind Virusvarianten, die direkt aus Patientenmaterial, Serum oder infizierten Zellen aus dem Blut im Labor angezüchtet worden sind. Diese Viren zeichnen sich, im Unterschied zu HIV-1 Viren, die lange im Labor gezüchtet wurden, durch besondere Eigenschaften aus. Diese Viren sind resistenter gegen neutralisierende Antikörper und Chemokine. Im Unterschied zu Zellkultur-adaptierten Viren benutzen Patientenisolate verschiedene Corezeptoren für die Infektion von Zellen. Da sich Patientenisolate sehr von Laboradaptierten Viren unterscheiden, sollen die gesammelten V2-Loop- bzw. V3-Loop-Sequenzdaten der env-Gene solcher Viren für die Herstellung der GP120-Mischung benutzt werden. Im Rahmen der Erfindung sind zusätzliche Basenaustausche in Bereichen außerhalb der für V2 und V3 kodierenden Sequenzabschnitte möglich.

Gemäß einer besonderen Ausführungsform der Erfindung wird eine Protein-Vakzine zur Verfügung gestellt, die eine Mischung von GP120-Proteinen des Humanen Immundefizienzvirus (HIV) umfaßt, die sich jeweils in ihrer Aminosäuresequenz im Bereich der V3-Scheife und/oder der V2-Schleife voneinander unterscheiden.

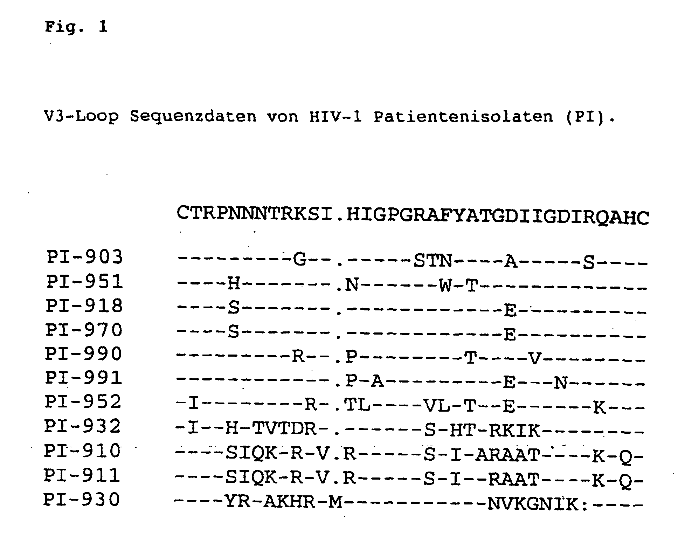

Im Rahmen der vorliegenden Erfindung werden bei der gentechnischen Herstellung der Protein-Mischung bzw. der Protein-Vakzine Sequenzvariationen vorzugsweise in Bereichen des V3-Loops eingeführt, die außerhalb von Konsensussequenzen liegen (vgl. z.B. M. Schreiber et al., J. Virol. 71 Nr. 12 (1997) 9198-9205). Bei den Konsensussequenzen handelt es sich um Sequenzabschnitte, die sowohl zwischen verschiedenen Virusstämmen als auch bei der Bildung von Virusvarianten bei fortschreitender Virus-Erkrankung (HIV-Erkrankung) im Körper im wesentlichen erhalten bleiben. Ein solcher Abschnitt ist im Falle des HIV-1 vom B Subtyp die Sequenz Gly-Pro-Gly-Arg-Ala-Phe (GPGRAF). Links und rechts von diesem Sequenzmotiv liegen kurze 4-10 Aminosäure lange Bereiche, die stark variieren können. In Fig. 1 ist dies am Beispiel des V3-Loop Sequenzvariationen von Patientenisolaten dargestellt.

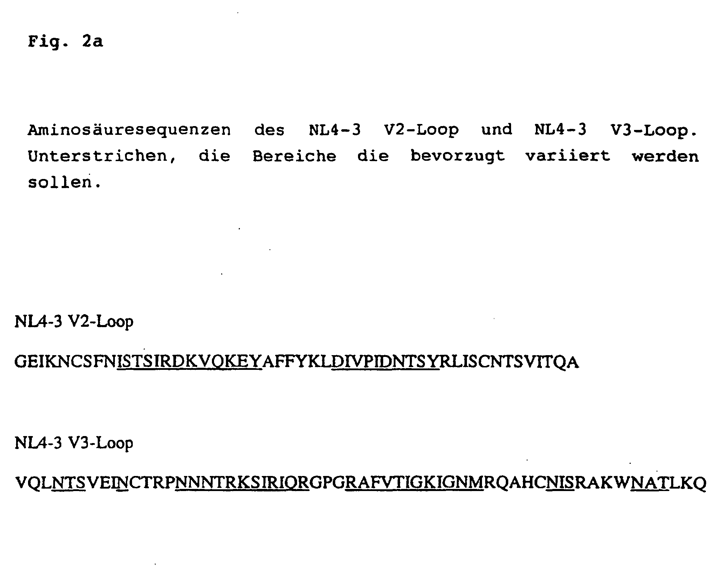

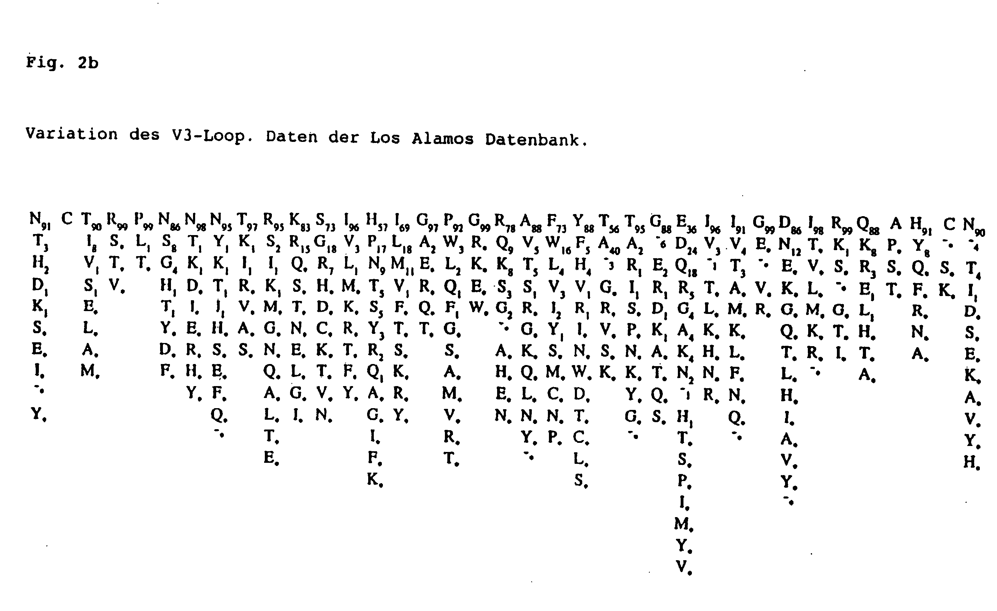

Zusätzlich zu Variationen im Bereich des V3-Loops können die Moleküle der erfindungsgemäßen Protein-Vakzine auch im Bereich des V2-Loops Sequenzvariationen aufweisen. Auch in diesem Fall sind Änderungen in der Aminosäuresequenz außerhalb von Konsensussequenz-Bereichen bevorzugt (siehe Fig. 2). Ferner sind zusätzliche Aminosäureaustausche in Bereichen außerhalb der V2und V3-Loops möglich.

In Zusammenhang mit der vorliegenden Erfindung werden stets die üblichen Ein- bzw. Dreibuchstabencodes zur Bezeichnung der Aminosäuren verwendet. Bei der Variation der Aminosäuresequenzen innerhalb der erfindungsgemäßen Protein-Vakzine sind beliebige Aminosäureaustausche möglich. In jedem Fall werden aber Aminosäuren der viralen GP120 Sequenz bzw. der Sequenzen des V2-Loop und V3-Loop vorzugsweise durch Aminosäuren ausgetauscht, die auch bei anderen Virusvarianten an den entsprechenden Sequenzpositionen vorkommen (siehe Fig. 2, Stand der Sequenzdaten 1997 in: Human Retroviruses and AIDS, A compilation and analysis of nucleic acid and amino acid sequences, Los Alamos National Laboratory, Los Alamos, NM 87545, U.S.A., Editors: B. Korber, B. Hahn, B. Foley, J.W. Mellors, T. Leitner, G. Myers, F. McCutchan, C. Kuiken).

Im Rahmen der vorliegenden Erfindung wird erstmals eine Protein-Vakzine zur Verfügung gestellt, die auf gentechnischem Wege hergestellt wird und Sequenzvarianten spezifischer viraler Antigene (Proteine) umfaßt. Die Herstellung der Vakzine wird nachfolgend am Beispiel des V3-Loops von HIV beschrieben. Für den Fachmann versteht es sich, daß das nachfolgend beschriebene Synthese-Prinzip auf andere Viren bzw. virale Proteine oder Teile von deren Sequenzen übertragen werden kann.

Kurz gesagt wird als Basis für die Vakzine eine Mischung aus GP120-Proteinen mit variablen V3-Sequenzen hergestellt, die der Variabilität des V3-Loops des GP120-Proteins von HIV nachempfunden sind.

Dafür wird zunächst die für das GP120-Protein kodierende Sequenz stückweise in das Plasmid pUC 18/19 hineinkloniert. Dies erfolgt durch einen Polylinkeraustausch, so daß mit den möglichen Restriktionssites, die vorher in der *gp120*-Sequenz durch stille Mutationen eingefügt wurden, alle interessanten Abschnitte mit monovalenten Restriktionssites flankiert sind und somit später herausgeschnitten und ausgetauscht werden können.

Anschließend werden zwei homologe Nukleinsäureoligomere mit Hilfe eines DNA-Synthesizers hergestellt (Länge ca. 300 Basenpaare). Nach erfolgter Hybridisierung bildet sich das doppelsträngige DNA-Fragment mit der DNA-Sequenz des V3-Loops. Für die Herstellung des doppelsträngigen V3-Loop DNA-Fragments können verschiedene Standardmethoden aus der Molekularbiologie verwendet werden. Bei einer Methode werden die Variablen durch Inosine eingeführt und bei dem entsprechendem komplementären Strang werden die Variablen durch einen Nukleotiod-Mix (AGCT, AGC, AG, ...) eingeführt. Bei dieser Methode wird das DNA-Fragment ausschließlich chemisch hergestellt. Bei einer anderen Methode, einer Kombination aus chemischer und enzymatischer DNA-Synthese, werden die Variablen durch Nukleotid-Mischungen eingeführt. Die Hybridisierung der Oligonukleotide erfolgt dann an komplementären Enden einer Länge von 20-30 Basen, die nicht variabel sind. Die Synthese zum vollständig doppelsträngigen Molekül erfolgt enzymatisch mit Hilfe einer DNA-Polymerase. Dabei können sowohl isotherme DNA-Polymerasen (Klenow-Fragment) oder thermostabile DNA-Polymerasen verwendet werden (Taq-Polymerase). Verwendet man Taq-Polymerase, dann können größere Mengen für die Klonierung des V3-Loop DNA-Fragments auch mit Hilfe der Polymerase-Ketten-Reaktion hergestellt werden. Mit Hilfe dieser Methoden wird ein doppelsträngiges DNA-Fragment hergestellt, welches an bestimmten Positionen variabel ist. Diese degenerierte DNA-Sequenz kodiert für die entsprechende Vielzahl von V3-Loop Aminosäure-Sequenzen der herzustellenden GP120-Proteinmischung, der Protein-Vakzine.

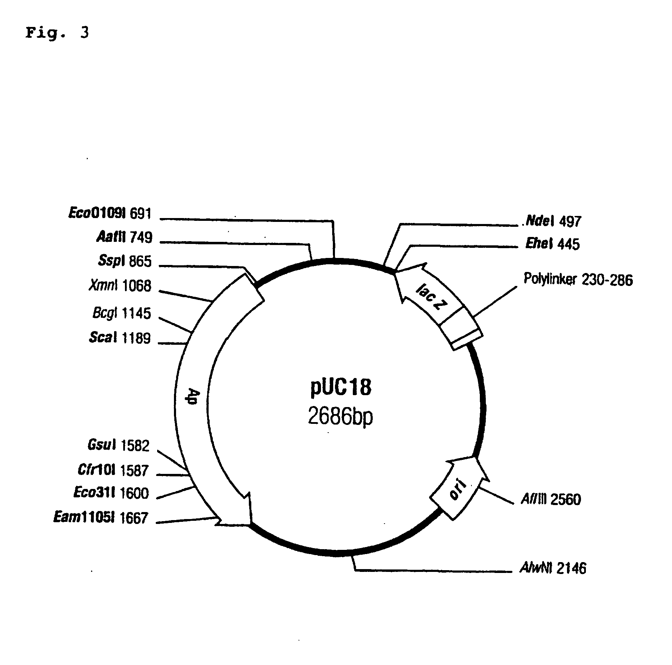

Die Mischung der synthetisierten V2-Fragmente besitzt am 5'-Ende eine PstI- und am 3'-Ende eine BclI-Schnittstelle. Die Mischung der synthetisierten V3-Fragmente besitzt am 5'-Ende eine BglII- und am 3'-Ende eine XbaI-Schnittstelle. Mit Hilfe der jeweiligen zwei Schnittstellen wird die Fragment-Mischung in einen Vektor, z.B. pUC18 delta-env oder pUC 18 BstEII-BamHI (Fig. 3), kloniert. Es entsteht nach dieser Klonierung eine Mischung oder ein Pool von Plasmid-DNAs des Vektors pUC 18, die alle das vollständige BstEII-BamHI env Fragment besitzen. Alle Plasmid-DNAs unterscheiden sich ausschließlich in der Sequenz des env V2-Loop oder V3-Loop. Durch Insertion der V2 bzw. der V3 Fragmente wird die Deletion (delta env) des *env*-Genabschitts im pUC18 Vektor aufgehoben.

Diese so hergestellte Mischung der Plasmid-DNAs mit variablen *env*-Fragmenten wird in *E*. *coli* transformiert und fermentiert. Dabei amplifiziert und repliziert *E*. *coli* diese Mischung an Plasmid-DNA. Dabei werden alle möglichen Variablen dieser V2-/V3-Loop-Plasmide erzeugt.

Anschließend wird dieser Plasmid Pool isoliert. Aus der Mischung der Plasmid-DNAs wird dann das *env*-Fragment durch BstEII- und BamHI-Verdau ausgeschnitten und direkt in den Vektor für die Expression des viralen *gp120* kloniert. Dieser Vektor hat den Vorteil, daß das GP120 auch im eukaryotischen Zellsystem exprimiert wird.

Dieser Pool an BSCenvATG-Vektor-DNA mit variablem *gp120-Kon*strukt wird nun in Cos- bzw. Chinese Hamster Ovary-Zellen (CHO-Zellen) transfiziert. Diese Eukaryonten exprimieren dann diesen Pool an Plasmiden, so daß statistisch das entsprechende Protein zu jeder Variablen translatiert und anschließend - je nachdem, welche Eukaryonten eingesetzt werden - entsprechend glykosyliert wird. Es schließt sich die Ernte der Proteine mit anschließender Aufreinigung bis zum Fertigprodukt (Protein-Mischung bzw. GP120-Mischung mit variabler Aminosäuresequenz) an.

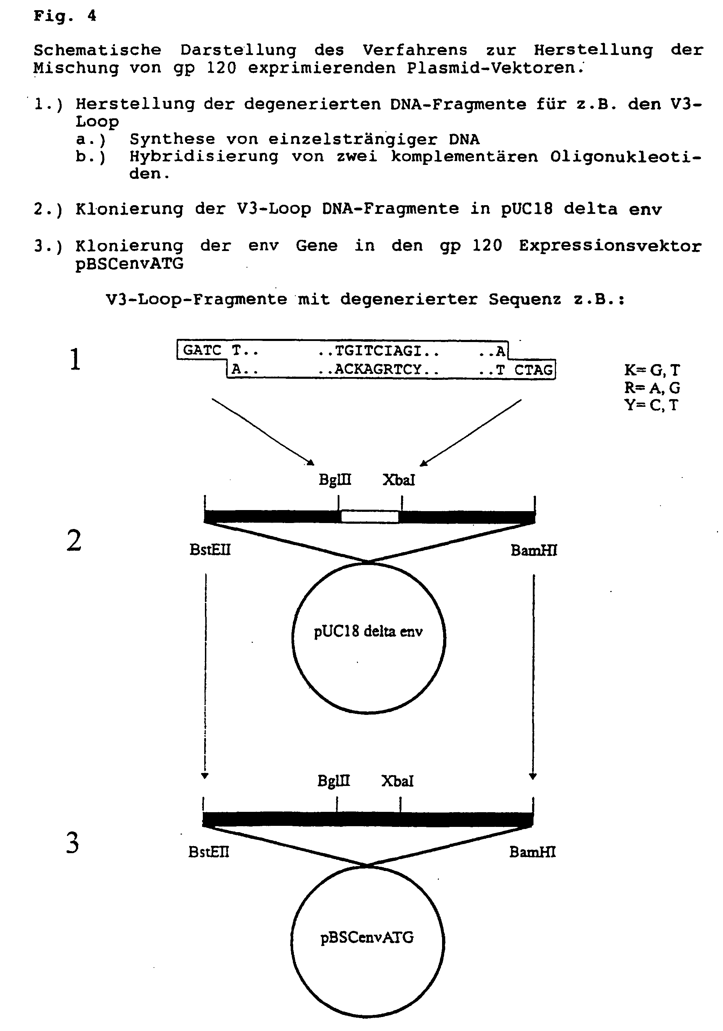

Zur Veranschaulichung wird das Verfahren zur Erzeugung der erfindungsgemäßen Protein-Vakzine in Fig. 4 übersichtlich dargestellt.

Wie bereits dargelegt, können Variationen im V2-Loop und/oder im V3-Loop vorgenommen werden. Die Variation des V2-Loops kann dabei nach demselben, für V3 beschriebenen Schema erfolgen.

Die Herstellung der erfindungsgemäßen Protein-Vakzine erfolgt, wie oben beschrieben, über verschiedene Schlüssel-Konstrukte, d.h. Nukleinsäure-Zwischenstufen und DNA-Konstrukte, die zur Ausführung der Erfindung wesentlich sind.

Wie oben bereits ausgeführt, wird die das GP120-Protein kodierende Nukleinsäuresequenz stückweise in pUC 18/19 hineinkloniert, wobei erfindungsgemäß von einer *gp120*-Sequenz ausgegangen wird, bei der die mehrfach vorhandenen Restriktionsspaltorte zunächst durch stille Mutationen so modifiziert werden, daß von den verbleibenden Restriktionsspaltorten jeder nur noch einmal in der Sequenz vorkommt. Diese Sequenzmodifikation ist notwendig, um die Expressionskassette, die zur Erzeugung der Sequenzvarianten erforderlich ist, gezielt an einer ganz bestimmten Stelle, die durch zwei, nur je einmal in der Sequenz vorkommende Restriktionsspaltorte begrenzt ist, einfügen zu können. Dem Fachmann sind Verfahren zur Einführung stiller Mutationen in einer Nukleinsäuresequenz wohlbekannt.

Für die vorliegende Erfindung wurde unter anderem eine Nukleinsäuresequenz erzeugt, die von der *env*-Sequenz in SEQ ID NO: 1 oder einem Fragment derselben abgeleitet ist, wobei sie derart modifiziert ist, daß sie ausschließlich monovalente Restriktionsspaltorte enthält. Vorzugsweise erfolgt die Modifikation durch Einführen stiller Mutationen. Gemäß einer bevorzugten Ausführungsform der Erfindung weist die Nukleinsäuresequenz die in SEQ ID NO: 9 dargestellte Sequenz auf. Die Sequenz der Genkassette kann derart modifiziert sein, daß sie das gesamte *env*-Gen oder Teile des env-Gens von Virusisolaten aus Patienten enthält. Vorzugsweise soll eine solche Genkassette die *env*-Sequenz des Pateientenisolats PI-932 (SEQ ID NO: 11) enthalten. Eine erfindungsgemäß bevorzugte Genkassette basierend auf der Sequenz PI-932 ist in SEQ ID NO: 12 dargestellt.

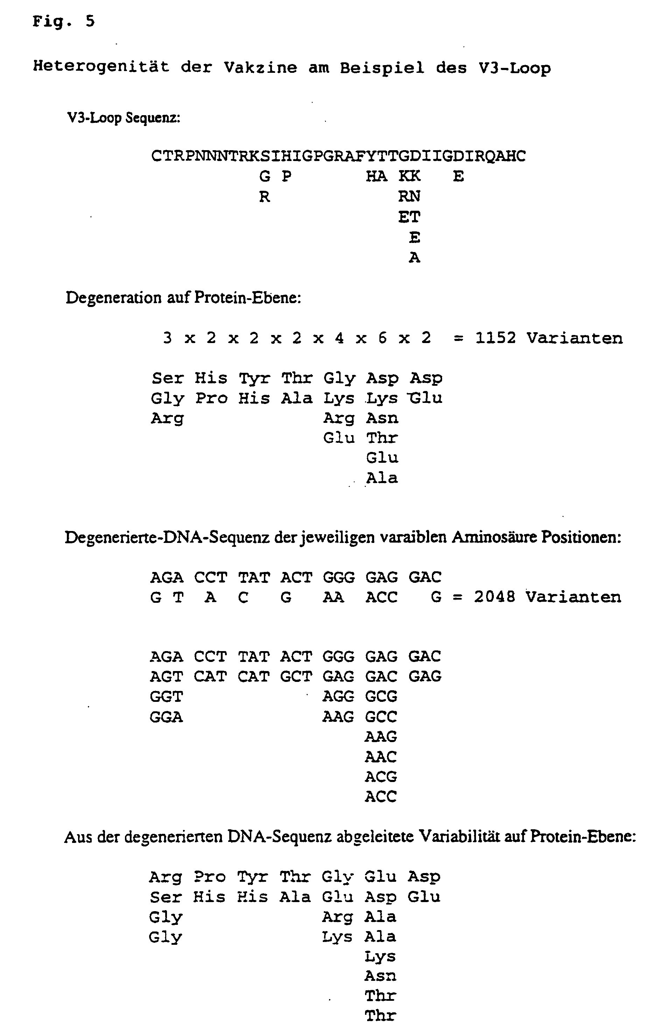

Gegenstand der vorliegenden Erfindung ist auch eine einzelsträngige Nukleinsäuresequenz, die den für den V3-Loop und/oder den für den V2-Loop kodierenden Bereich oder Fragmente bzw. Teile derselben enthält, wobei im Falle des V3-Loop ein BglII-XbaI (247 bp) oder auch ein BglII-NheI (283 bp) Fragment gegen ein verändertes Fragment welches an mindestens 6, vorzugsweise an 9 bis 20 Positionen, Nukleinsäureaustausche oder Mutationen trägt, und im Falle des V2-Loop ein PstI-BclI (139 bp) oder auch ein PstI-EcoRI (339 bp) Fragment gegen ein verändertes Fragment welches an mindestens 6, vorzugsweise an 9 bis 20 Positionen, Nukleinsäureaustausche oder Mutationen trägt, ausgetauscht sein kann. Dabei sind die Nukleotide innerhalb einer Nukleinsäuresequenz entweder jeweils durch Inosin oder jeweils durch eine Mischung von 2-4 Nukleotiden ersetzt. Am Beispiel in Fig. 5 ist dies erläutert. Sollen an 7 Aminosäurepositionen des V3-Loop 21 verschiedene Aminosäuren zu 1152 Varianten kombiniert werden, dann müssen an 11 Nukleinsäurepositionen jeweils zwei Nukleotide durch chemische Synthese in die Sequenz der einzelsträngigen Nukleinsäuren (Oligonukleotide) eingeführt werden (Fig. 5).

Die einzelsträngigen Nukleinsäuresequenzen werden - wie oben bereits erwähnt - zum Doppelstrang hybridisiert bzw. synthetisiert.

Die vorliegende Erfindung betrifft daher ferner doppelsträngige DNA, die Hybride der o.g. einzelsträngigen Nukleinsäuresequenzen umfaßt, wobei jeweils eine Nukleinsäuresequenz (5'-3'-Oligomer oder 3'-5'-Oligomer) an ein oder mehreren ausgewählten Positionen Inosine enthält und die andere Nukleinsäuresequenz (3'-5'-Oligomer oder 5'-3'-Oligomer) an den bei der späteren Hybridisierung entsprechenden komplementären Positionen jeweils zwei, drei oder vier der möglichen Nukleotide (Adenin, A; Thymin, T; Guanin, G; Cytosin, C) enthält. Dadurch werden Sequenzen erzeugt, bei denen die vier Basen zufallsverteilt an den jeweiligen Positionen der einzelsträngigen Nukleinsäuresequenz (DNA) vorhanden sind, so daß die gewünschten Kombinationen von A, T, G oder C für die entsprechenden gewünschten Aminosäure Codone (ein Codon wird aus einer Abfolge von drei Nukleotiden gebildet) erzeugt werden. Aufgrund der Variation von Nukleotidpositionen kommt es zu zufallsverteilten Sequenzkombinationen. Daher entstehen z.B. für die Kombination aus (A,C) (ACT) (ACGT) insgesamt 2x3x4=24 mögliche DNA-Sequenzen, die für verschiedene Aminosäuren kodieren. Die Anzahl der variablen Positionen bestimmt auch gleichzeitig die Anzahl und Position der eingeführten Inosine in der komplementären DNA-Sequenz. Die Berechnung der Heterogenität eines solchen Konstruktes ist in Fig. 5 dargestellt.

Da zur Bildung der doppelsträngigen DNA jeweils Inosine enthaltende einzelsträngige DNA mit Oligomeren hybridisiert wird, das an den entsprechenden Positionen jeweils zufallverteilt A, T, G oder C enthält, wird eine Mischung aus doppelsträngiger *gp120*-Sequenz oder eines Teils (Fragments) derselben erhalten, wobei die Nukleinsäuresequenzen jeweils von der *env*-Sequenz (SEQ ID NO: 1 oder 12) abgeleitet sind und wobei sich die Nukleinsäuresequenzen in dem für die V2-Schleife kodierenden Bereich und/oder in dem für die V3-Schleife kodierenden Bereich jeweils voneinander unterscheiden. Das heißt, daß sich die in der Mischung enthaltenen Nukleinsäuresequenzen derart voneinander unterscheiden, daß sie für eine Mischung von Proteinen kodieren, die in der V2-Scheife und/oder in der V3-Schleife jeweils voneinander verschiedene Aminosäuresequenzen aufweisen.

Im Rahmen der Erfindung können auf diese Weise mindestens 10², vorzugsweise mindestens 10³ und gemäß einer besonders bevorzugten Ausführungsform der Erfindung mindestens 10⁴ Sequenzvarianten auf Nukleinsäure-Ebene (DNA-Ebene) erhalten werden. Diese DNA-Mischung, die ≥ 10² DNA-Moleküle enthält, die sich in ihrer Nukleinsäuresequenz voneinander unterscheiden, wobei die Mischung aufgrund der Variation von Nukleotidpositionen zufallsverteilte Sequenzkombinationen aufweist, wird nachfolgend als DNA-Pool bezeichnet, bezogen auf die Varianten der *gp120*-Sequenz wird dieser nachfolgend als Pool mit variablem *gp120*-Konstrukt (*gp120*-Pool) bezeichnet.

Unter Sequenzvarianten eines DNA-Sequenz werden im Rahmen der vorliegenden Erfindung solche Moleküle verstanden, die eine gegenüber einer nativen viralen DNA-Molekül oder einem Teil (Fragment) desselben abgeleitete Nukleinsäuresequenz aufweisen, wobei sich die Varianten dadurch voneinander unterscheiden, daß mindestens ein Nukleotid an beliebigen Stellen der Sequenz ausgetauscht sein kann. Vorzugsweise weisen die Sequenzvarianten mehrere Nukleotidaustausche an verschiedenen Stellen der Sequenz auf, wobei die Zahl und die Lage der ausgetauschten Nukleinsäuren im wesentlichen von der Länge der Nukleinsäuresequenz abhängt.

Bei der Expression der env-Gen Varianten ausgehend von der hergestellten Plasmid-DNA-Mischung ist zu erwarten, daß aufgrund der Zufallsverteilung alle möglichen DNA Sequenzen exprimiert werden. Daher werden dann auch alle aufgrund der vorgegebenen DNA-Mischung möglichen Sequenzvarianten des *gp120*-Moleküls oder eines Teils desselben gebildet. Die Heterogenität des *gp120*-Gemisches berechnet sich einerseits anhand der Heterogenität der DNA-Sequenz und der Degeneration des genetischen Codes für die Aminosäuren (siehe auch Fig. 5).

Der Nachweis der Heterogenität der Plasmid-DNA Mischung erfolgt durch die DNA-Sequenzierung von einzelnen Klonen. Dazu wird die Mischung der Plasmid-DNA in *E. coli* transformiert und einzelne Klone werden zufällig ausgewählt und deren V2- bzw. V3-Loop-Sequenz bestimmt. Insgesamt sollen ca. 100-200 verschiedene Klone sequenziert werden. Aus der statistischen Verteilung der DNA-Sequenzen kann auf die Verteilung der gesamten Mischung zurückgerechnet werden. Die Methode entspricht weitestgehend der Methode der Stichproben-Entnahme, wie sie standardmäßig für die Qualitätskontrolle von verschiedensten Produkten eingesetzt wird.

Die direkte molekulare Analyse der Heterogenität der GP120-Protein Mischung gestaltet sich etwas schwieriger, da sich einzelne GP120-Moleküle aus der Mischung nicht abtrennen und nachweisen lassen. Dies liegt in der Natur der Sache, weil sich die einzelnen GP120-Varianten nur in wenigen Aminosäuren voneinander unterscheiden. Durch Gelelektrophoretische Auftrennung oder eine Massenspektroskopische Analyse kann festgestellt werden, ob es sich um eine Mischung oder eine einzelne Form des GP120-Moleküls handelt. Wenn die GP120-Mischung z.B. im Tier als Vakzine eingesetzt wird, ist die im Tier induzierte Immunantwort direkt von der Anzahl und der Zusammensetzung der GP120-Mischung abhängig. Die Immunantwort des Tieres, die neutralisierenden Antikörper kann dann in Virus-Neutralisations-Tests untersucht werden. Zur Kontrolle einer entsprechend Varianten-übergreifenden Immunantwort können verschiedene Patientenisolate des HIV-1 im Neutralisationtest eingesetzt werden. Bevorzugt werden Patientenisolate gewählt, die sich in der Fähigkeit unterscheiden, verschiedene Corezeptoren für die Infektion der Zielzellen benutzen zu können. Das Neutralisationspotential der GP120-Mischung dient dann als Qualitätsmaßstab.

Gegenstand der vorliegenden Erfindung ist daher ferner eine Protein-Mischung, die GP120-Proteine umfaßt, die in der V2-Scheife und/oder in der V3-Schleife jeweils voneinander verschiedene Aminosäuresequenzen aufweisen, wobei die Mischung mindestens 10² (vorzugsweise mindestens 10³ und gemäß einer besonders bevorzugten Ausführungsform der Erfindung mindestens 10⁴) Sequenzvarianten enthält und durch Expression einer Plasmid-DNA-Mischung erhältlich ist, die aufgrund der Variation von Nukleotidpositionen zufallsverteilte Sequenzkombinationen aufweist.

Wie bereits zuvor erwähnt, wird die Mischung doppelsträngiger DNA, die durch Hybridisierung von Inosine-enthaltender einzelsträngiger DNA mit zufallsverteilt A-, T-, G- und/oder C-enthaltender einzelsträngiger DNA erhalten werden kann, d.h. der Pool mit variablem GP120-Konstrukt, in prokaryontischen oder eukaryontischen Wirtszellen, vorzugsweise *E. coli*, transformiert und fermentiert.

Gegenstand der vorliegenden Erfindung sind daher Plasmide, die doppelsträngige DNA insertiert enthalten, die jeweils Hybride der einzelsträngigen, Inosin-enthaltenden Nukleinsäuresequenz (s.o.) mit der einzelsträngigen, eine Mischung aller vier Nukleotidvarianten (A, T, G, C) enthaltende Nukleinsäuresequenz (s.o.) umfassen. Ferner betrifft die Erfindung eine Vektor-Mischung, die eine Mischung dieser Plasmide, wobei sich die Nukleinsäuresequenzen der Plasmide in dem für die V3-Schleife kodierenden Bereich und/oder in dem für die V2-Schleife kodierenden Bereich jeweils voneinander unterscheiden, wobei die Vektor-Mischung mindestens 10² (vorzugsweise mindestens 10³ und gemäß einer besonders bevorzugten Ausführungsform der Erfindung mindestens 10⁴) der genannten Plasmide enthält und sie aufgrund der Variation von Nukleotidpositionen zufallsverteilte Sequenzkombinationen aufweist. Je nachdem, welche Wirtszellen zur Expression der Vektoren (Plasmide) verwendet werden sollen, kommen verschiedene, dem Fachmann wohlbekannte Expressionssysteme und Basisvektoren in Frage (vgl. Methods in Enzymology, Vol. 185, Gene Expression Technology, 1991, Herausgeber D.V. Goeddel, Academic Press, Inc.). So ist das Plasmid pUC 18/19 für die Expression in *E. coli* als Basisplasmid bevorzugt, in das die Nukleinsäuresequenz-Varianten hineinkloniert werden. Im Rahmen der vorliegenden Erfindung lassen sich die Vektor-Mischungen somit entweder in bakteriellen Wirtszellen, wie *E*. *coli*, oder in eukaryontischen Wirtszellen, vorzugsweise aus der Gruppe bestehend aus Cos-, CHO-, oder Baby Hamster Kidney-Zellen (BHK-Zellen), oder in anderen Wirtszellen exprimieren.

Die vorliegende Erfindung betrifft somit ferner *E. coli*-Wirtszellen oder eukaryontische Wirtszellen, die mit einer erfindungsgemäßen Vektor-Mischung transfiziert ist.

Da Wirtszellen mit dem DNA-Pool (der Vektor-Mischung) transfiziert werden, ist zu erwarten, daß eine ebenso große oder zumindest annähernd ebenso große Anzahl unterschiedlich transformierter Wirtszellen entsteht, wie Sequenzvarianten im DNA-Pool vorhanden sind. Bei der Herstellung der Vakzine ist besonderes Augenmerk auf die Ausbeuten bei den einzelnen Klonierungsschritten zu legen. Die Ausbeuten der Herstellung der doppelsträngigen DNA-Fragmente, der Ligation der V3-Loop und V2-Loop DNA-Fragmente in das env-Gen und die Transformation bzw. die Herstellung der Bakterien und Zellklone muß so gestaltet sein, daß die zu erzielende Heterogenität des Gemisches nicht eingeschränkt wird. An einem Beispiel soll dies verdeutlicht werden. Wenn z.B. aus einer Menge von Kugeln, die sich aufgrund zweier Farben unterscheiden, eine Anzahl Kugeln entnommen werden soll, aber beide Farben mit 99,9 % Wahrscheinlichkeit in dieser Auswahl vorhanden sein sollen, müßten ca. 13 Kugeln entnommen werden (G. Schreiber, Ein kombinatorisches Problem aus der Genetik; Bioengineering 1988, 2:32-35). Übertragen auf die Klonierungsschritte der HIV-Vakzine bedeutet dies: Wenn eine Heterogenität von ca. 6000 Virusvarianten erzeugt werden soll, müßten in jedem einzelnen Klonierungsschritt dreizehnmal so viele Klone erzeugt werden. Es muß also eine Genbank von ca. 80000 Klonen für den V3-Loop hergestellt werden. Ausgehend von dieser Genbank werden dann die Expressionsvektoren für die Transfektion der CHO-Zellen hergestellt. Bei der Transfektion der CHO-Zellen müßen dann demzufolge ca. 80000 Transfektionsereignisse erzielt werden. Eine solche Mischung von transfizierten CHO-Zellen erzeugt dann die gewünschte Menge von 6000 verschiedenen gp120 Varianten.

Die vorliegende Erfindung betrifft auch ein Verfahren zur Herstellung einer für ein virales Protein kodierenden Nukleinsäuresequenz (Expressionskassette), bei dem man die Sequenz derart modifiziert oder vorzugsweise so viele stille Mutationen einführt, daß sie anschließend mindestens zwei und vorzugsweise ausschließlich monovalente Restriktionsspaltorte enthält. Besonders bevorzugt enthält die Sequenz nur noch monovalente Restriktionsspaltorte. Vorzugsweise handelt es sich bei dem von der Nukleinsäuresequenz kodierten Protein um GP120, wobei man durch stille Mutationen die für das GP120 kodierende env-Wildtyp-Sequenz variiert.

Ferner ist Gegenstand der vorliegenden Erfindung ein verfahren zur Herstellung der erfindungsgemäßen vektor-Mischung, die vorzugsweise eine Mischung von Plasmiden enthält, deren Nukleinsäuresequenzen sich in dem für die V2-Schleife kodierenden Bereich und/oder in dem für die V3-Schleife kodierenden Bereich jeweils durch Zufallsverteilung der Basen an den variierten Nukleotidpositionen voneinander unterscheiden, wobei man die erfindungsgemäßen Plasmide in einen in Wirtszellen (vorzugsweise *E. coli*-, Cos-, CHO- oder BHK-Zellen) exprimierbaren (Basis-)Vektor hineinligiert. Bei dem (Basis-)Vektor handelt es sich vorzugsweise um den pUC 18-, den pUC 19- oder den BSCenvATG-Vektor.

Im Rahmen der vorliegenden Erfindung wird ferner ein Verfahren zur Herstellung/Erzeugung von Wirtszellen, vorzugsweise ausgewählt aus der Gruppe bestehend aus *E*. *coli*-, Cos-, BHK- oder CHO-Zellen, bei dem man die Wirtszellen mit einer erfindungsgemäßen Vektor-Mischung transformiert, die eine Mischung von Plasmiden enthält, deren Nukleinsäuresequenzen sich in dem für die V2-Schleife kodierenden Bereich und/oder in dem für die V3-Schleife kodierenden Bereich jeweils durch Zufallsverteilung der Basen an den variierten Nukleotidpositionen voneinander unterscheiden.

Schließlich wird erfindungsgemäß erstmals ein Verfahren zur Herstellung einer DNA-Vakzine zur Verfügung gestellt, bei dem man das erfindungsgemäße Verfahren zur Herstellung der Vektor-Mischung durchführt, wobei die erfindungsgemäßen Plasmide nach Applikation in Wirtszellen (z.B. menschliche und tierische Monocyten) die Mischung der *gp120*-Proteine exprimieren. Die DNA-Vakzine kann zur Applikation gegebenenfalls mit pharmazeutisch verträglichen Hilfs- und/oder Trägerstoffen formuliert sein und nach Verabreichung im Organismus die Produktion der Sequenzvarianten viraler Proteine ermöglichen.

Die vorliegende Erfindung betrifft somit auch eine pharmazeutische Zusammensetzung bzw. eine DNA-Vakzine, die für eine Mischung strukturell unterschiedlicher Virus-Proteine kodiert, wobei die Vakzine eine Mischung von Sequenzvarianten eines viralen DNA-Moleküls oder eines Teils desselben enthält, d.h. von DNA-Molekülen, deren Nukleinsäuresequenzen sich in dem für das Protein kodierenden Bereich, einem Teil oder einem Fragment desselben voneinander unterscheiden. Die Mischung enthält ≥ 10² DNA-Moleküle, die sich in ihrer Nukleinsäuresequenz voneinander unterscheiden, wobei die Mischung aufgrund der Variation von Nukleotidpositionen zufallsverteilte Sequenzkombinationen aufweist. Unter dem Begriff "strukturell unterschiedlichen Virus-Proteinen" werden erfindungsgemäß solche Proteine verstanden, deren Aminosäuresequenzen sich von der Wildtyp-Sequenz des entsprechenden Virus-Proteins ableiten, wobei die Aminosäuresequenzen insofern voneinander abweichen, als sie sich untereinander und im Vergleich zur Wildtyp-Sequenz durch ein oder mehrere ausgetauschte Aminosäuren an gleichen oder unterschiedlichen Positionen der Sequenz unterscheiden. Wie bereits ausgeführt, kodieren die Nukleinsäuresequenzen in der Vakzine erfindungsgemäß bevorzugt für eine Mischung strukturell unterschiedlicher GP120-Proteine von HIV (Sequenzvarianten von GP120), wobei eine Vakzine besonders bevorzugt ist, die eine Mischung von DNA-Molekülen enthält, deren Nukleinsäuresequenzen sich in dem für die V2-Schleife kodierenden Bereich und/oder in dem für die V3-Schleife kodierenden Bereich von *gp120* des HIV-1 voneinander unterscheiden. In diesem Zusammenhang wird auch auf Fig. 3 verwiesen, in der im V3-Loop von GP120 bekannte strukturelle Unterschiede bzw. Variationen dargestellt sind.

Die erfindungsgemäße DNA-Vakzine besitzt besondere Bedeutung im Rahmen einer Gentherapie.

Schließlich wird erfindungsgemäß erstmals ein Verfahren zur Herstellung einer pharmazeutischen Zusammensetzung bzw. einer Protein-Vakzine zur Verfügung gestellt, bei dem man die erfindungsgemäßen Wirtszellen, d.h. Wirtszellen, die mit einer erfindungsgemäßen Vektor-Mischung transformiert sind, wobei die Vektoren jeweils Plasmide enthalten, deren Nukleinsäuresequenzen sich in dem für die V2-Schleife kodierenden Bereich und/oder in dem für die V3-Schleife kodierenden Bereich jeweils durch Zufallsverteilung der Basen an den variierten Nukleotidpositionen voneinander unterscheiden, unter Bedingungen kultiviert, die die Expression der Mischung viraler Protein-Sequenzvarianten gestatten. Bei den Wirtszellen handelt es sich vorzugsweise um bakterielle Wirtszellen, wie *E. coli*, oder um eukaryontische Wirtszellen, vorzugsweise aus der Gruppe bestehend aus Cos-, Chinese Hamster Ovary- (CHO-), oder Baby Hamster Kidney-Zellen (BHK-Zellen).

Durch die vorliegende Erfindung ist es somit möglich, eine Vakzine aus variablen GP120-Proteinen zur Verfügung zu stellen, die bei der prophylaktischen und/oder therapeutischen Behandlung einer HIV-Infektion oder AIDS
1. das Immunsystem aktiviert,
2. die Bildung HIV-1-neutralisierender Antikörper induziert,
3. den Verlust von neutralisierenden Antikörpern verhindert und
4. die Kontrolle über die Stimulation der GP120-spezifischen Immunantwort durch den Wirkstoff übernommen wird.

Dies erfolgt vorzugsweise dadurch, daß bereits vor dem mit dem Ausbruch von AIDS im Zusammenhang stehenden Verlust HIV-neutralisierender Antikörper ausreichend Antigene zur Verfügung stehen, die aufgrund ihrer Vielfalt unterschiedlicher Aminosäuresequenzen (d.h. ihrer Sequenzvariationen) in der Lage sind, die Bildung derjenigen HIV-Antikörper zu induzieren, die normalerweise, d.h. ohne entsprechende Prophylaxe gemäß der vorliegenden Erfindung, bei Progression der Erkrankung verloren gehen oder in ihrer Konzentration abnehmen. Neben einer vorbeugenden Behandlung betrifft die vorliegende Erfindung auch die therapeutische Behandlung, wobei die GP120-Mischung (Protein-Vakzine) eingesetzt wird, um einer Abnahme bzw. einem Verlust HIV-neutralisierender Antikörpen entgegenzuwirken, indem das Immunsystem aktiviert und die Bildung neuer bzw. zusätzlicher HIV-1-neutralisierender Antikörper induziert wird.

Erfindungsgemäß wird daher die Mischung strukturell unterschiedlicher viraler Proteine, die vorgenannte Sequenzvarianten eines viralen Proteins (vorzugsweise von GP120) oder eines Teils desselben sind zur Herstellung einer Vakzine zur Prävention und/oder Therapie einer Virus-Infektion beim Menschen verwendet. Ferner betrifft die vorliegende Erfindung die Verwendung einer Mischung von DNA-Molekülen, die für ≥ 10² Sequenzvarianten eines viralen Proteins oder eines Teils desselben kodieren, wobei die Mischung aufgrund der Variation von Nukleotidpositionen zufallsverteilte Sequenzkombinationen aufweist, zur Herstellung einer Vakzine zur Prävention und/oder Therapie einer Virus-Infektion beim Menschen. Gemäß einer bevorzugten Ausführungsform betrifft die Erfindung die Verwendung zur Herstellung einer Vakzine zur Prävention und/oder Therapie einer HIV-Infektion beim Menschen.

Bei der Virus-Infektion kann es sich im Rahmen der vorliegenden Erfindung um jede beliebige Infektion handeln, bei der im Verlauf der Erkrankung sich replizierende Virusvarianten entstehen, wobei als ausgewählte Protein-Sequenzen oder proteinkodierende Nukleinsäuresequenzen diejenigen Aminosäuresequenzabschnitte oder für diese kodierende Nukleinsäureabschnitte erfindungsgemäß in Frage kommen, bei denen im Verlauf viraler Erkrankungen stets bzw. häufig Varianten, d.h Sequenzvariationen, beobachtet werden. Vorzugsweise handelt es sich vorliegend um Sequenzvarianten des GP120-Moleküls (auf Protein-Ebene) bzw. des für GP120 kodierenden Bereichs (auf DNA-Ebene) oder Teilen derselben, insbesondere um Sequenzvarianten im V3-Loop oder im V2-Loop, vorzugsweise sowohl im V3- als auch im V2-Loop.

Erfindungsgemäß werden folglich pharmazeutische Zusammensetzungen bzw. vakzinen zur Verfügung gestellt, die bei der immunrekonstitutiven Behandlung von Virus-Infizierten das Immunsystem in einer Art und Weise aktivieren, daß die natürlich erworbene Immunabwehr gegen das Virus regeneriert und neu stimuliert wird, um so die im Patienten replizierenden Virusvarianten an ihrer Vermehrung zu hindern. Gemäß einer bevorzugten Ausführungsform der Erfindung werden erstmals Vakzinen zur immunrekonstitutiven Behandlung von HIV-1--Infizierten bereitgestellt.

Im Rahmen der vorliegenden Erfindung können die Vakzinen entweder als solche, d.h. als DNA- und/oder Protein-Mischungen ohne weitere Zusätze, oder zusammen mit weiteren Wirkstoffen, wie z.B. Immunstimulantien wie Interleukin-2, CC- und CXC-Chemokine, und/oder pharmazeutisch verträglichen Hilfs- und Trägerstoffen formuliert bzw. verabreicht werden. In der Regel sind die erfindungsgemäßen Vakzinen zur intravenösen Applikation formuliert, wie z.B. intravenös, intramuskulär, subcutan. Außerdem können die Vakzinen zur oralen, mucosalen (intravaginalen, intrarektalen) und transdermalen Anwendung formuliert sein.

Die vorliegende Erfindung weist folgende Vorteile auf:

Durch eine gezielte gentechnische Manipulation der Nukleinsäuresequenz, mit Hilfe einer Genkassette, lassen sich beliebige Varianten eines Krankheitserregers oder eines Gens des Erregers herstellen. Die Variationen betreffen bevorzugt die Bereiche, gegen die neutralisierende Antikörper oder cytotoxische T-Zellen gebildet werden. Dadurch läßt sich ein Impfstoff in Form einer Mischung von Varianten des Krankheitserregers oder von Varianten der entsprechenden Antigene eines Krankheitserregers herstellen. Die Vorteile dieses Konzeptes in Bezug auf HIV als Krankheitserreger liegen in der Herstellung einer Mischung des HIV-GP120, dem äußeren Membranprotein des HIV, gegen welches sich die virusneutralisierende Immunantwort des Menschen richtet. Da gerade im Falle der HIV-Erkrankung jeder Patient einer Vielzahl von HIV-Varianten ausgesetzt ist, die sich alle in der Sequenz des GP120 Proteins unterscheiden, ist der Einsatz einer Vielzahl von GP120-Varianten als Impfstoff von besonderem Vorteil, wobei diese GP120-Mischung sowohl für die Immunisierung normaler gesunder Personen aber auch für die Therapie von bereits mit HIV infizierten Personen eingesetzt werden kann. Bei der Immunisierung mit der GP120-Mischung wird eine möglichst breite Immunantwort gegen möglichst viele Virusvarianten im Menschen induziert, die im idealen Falle gegen alle HIV-Varianten schützt. Bei der Therapie mit der GP120-Mischung kann der Verlust von neutralisierenden Antikörpern und der Verlust der HIV-spezifischen zellulären Immunantwort bekämpft werden.

Erfindungsgemäß ist es ferner erstmals möglich, *gp120*-Klone herzustellen, die in der Art bzw. Vielfalt der beobachteten Sequenzvariationen Plasmaisolaten von HIV-Infizierten bzw. an AIDS Erkrankten entsprechen (vgl. M. Schreiber et al., J. Virol. 68 No. 6 (1994) 3908-3916). In diesem Zusammenhang wird im Gegensatz zu verschiedenen bisher verfolgten Ansätzen kein isoliertes GP120-Molekül oder ein antigenes Fragment desselben zur Immunisierung verwendet, sondern eine Protein-Mischung, die aus einer Vielzahl von Sequenzvarianten besteht, die durch eine vollständige GP120-Sequenz charakterisiert sind, die darüber hinaus die korrekte, der natürlichen Faltung des GP120-Moleküls entsprechende Tertiärstruktur aufweisen. Die Konformation der in der Vakzine zur Verfügung gestellten Virusvarianten ist insofern von Bedeutung, als dadurch Sequenz- und Strukturvarianten bereitgestellt werden, die mit den im Verlauf der viruserkrankung nachweisbaren GP120-Varianten eine größtmögliche Übereinstimmung hinsichtlich der beobachteten Sequenzvariationen als auch der für die Bindung an CD4 und den Corezeptor erforderlichen Konformation erzielt wird, wodurch eine effektive Immunstimulation erzielt werden kann.

Durch die Erfindung wird eine Mischung von GP120-Proteinen bereitgestellt, die die Protein-Vakzine darstellt. Gleichzeitig wird durch die Erfindung eine Mischung der für diese Protein-Mischung kodierenden Gene bereitgestellt. Diese Gene können in einen Vektor überführt werden der sich für die direkte Anwendung am Menschen eignet (DNA-Vakzine) (Ulmer et al., 1995 Ann NY Acad Sci 772:117-125; Donnelly et al., 1995 Ann NY Acad Sci 1995 772:40-46). Eine DNA-Vakzine hat den Vorteil, daß die Heterogenität der Mischung der DNA-Vektoren höher sein kann als die Mischung der rekombinanten GP120-Proteine. Es ist leichter, eine hohe Heterogenität eines DNA-Vektorgemisches herzustellen. Eine solche DNA-Vakzine würde ein viel breiteres Spektrum an HIV-Varianten abdecken. Von der technischen Seite betrachtet ist die Herstellung der DNA-Mischung einfacher. Voraussetzung für die DNA-Vakzine ist ein *gp120*-Expressionsvektor, der die beiden Schnittstellen BstEII und BamHI trägt. Dies ermöglicht die Umsetzung der V3-Loop und V2-Loop Variablen *env*-Genfragmente in einen solchen Vektor.

Im Hinblick auf die GP120-Proteine ist zu beachten, daß sich im Bereich des V3-Loop 5 potentielle Stellen für die N-Glykosylierung befinden. Die Zuckerreste schützen den V3-Loop vor der Erkennung durch neutralisierende Antikörper. Virusvarianten, die einen glykosylierten V3-Loop aufweisen sind schlechter neutralisierbar als Virusvarianten bei denen die Glykosylierung unvollständig ist. Da sich Virusvarianten mit einer vollständigen V3-Loop-Glykosylierung der neutralisierenden Immunantwort entziehen können, sind sie für die Übertragung und für das Etablieren der Infektion von Bedeutung. Im Verlauf der Erkrankung, wenn ein Teil der neutralisierenden Antikörperantwort verlorengegangen ist, setzen sich Virusvarianten durch, deren V3-Loop nicht vollständig glykosyliert ist. Da der V3-Loop nicht mehr durch Zuckerreste verdeckt ist, replizieren diese Virusvarianten schneller als die vollständig glykosylierten V3-Loop Mutanten. Erfindungsgemäß ist es daher von Vorteil, bei der Konstruktion der GP120-Vakzine die unterschiedliche Glykosylierung des GP120 V3-Loop zu berücksichtigen.

Im Rahmen der vorliegenden Erfindung kann das an HIV dargestellte Prinzip auf andere Viren, die im Verlauf der viralen Erkrankung ebenfalls Varianten ausbilden, übertragen werden. Insbesondere können Vakzinen gegen eine Vielzahl von Viren bereitgestellt werden, die eine breite Immunantwort gegen viele Virusvarianten im Menschen induzieren, die im Idealfall gegen alle Varianten schützt. Bei der Therapie mit solchen Vakzinen kann der Verlust von neutralisierenden Antikörpern und der Verlust der HIV-spezifischen zellulären Immunantwort wirksam bekämpft werden. In diesem Zusammenhang ist es ferner möglich oder sogar ratsam, bei Erregertypen (Viren), die im Verlauf einer Erkrankung Sequenzvarianten mehrerer Proteine bilden, mehrere erfindungsgemäße Genkassetten bereitzustellen, die jeweils die für Varianten jedes dieser Proteine oder Fragmente derselben kodierende Nukleinsäuresequenz enthalten, um dem Verlust neutralisierender Antikörper gegen alle denkbaren Virusvarianten entgegenzuwirken.

Im Rahmen der vorliegenden Erfindung können die Vorteile einer Protein-Vakzine und einer DNA-Vakzine in vorteilhafter Weise kombiniert werden, um den Erfolg einer präventiven und/oder therapeutischen Behandlung einer viralen Erkrankung zu erhöhen. Es wird somit ferner eine pharmazeutische Zusammensetzung zur Prävention und/oder Therapie einer Virus-Infektion zur Verfügung gestellt, die eine Protein-Mischung und eine Nukleinsäuremischung umfaßt, wobei die Protein-Mischung ≥ 10² Sequenzvarianten eines viralen Proteins oder eines Teils desselben umfaßt und durch Expression einer Plasmid-DNA-Mischung erhältlich ist, die aufgrund der Variation von Nukleotidpositionen zufallsverteilte Sequenzkombinationen aufweist, und wobei die Nukleinsäuremischung ≥ 10² DNA-Moleküle umfaßt, die für Sequenzvarianten eines viralen Proteins oder eines Teils desselben kodieren, wobei die Nukleinsäuremischung aufgrund der Variation von Nukleotidpositionen zufallsverteilte Sequenzkombinationen aufweist. Insbesondere handelt es sich bei der pharmazeutischen Zusammensetzung um ein Kombinationspräparat, das sowohl eine der oben genannten Sequenzvarianten des GP120-Proteins umfassende Protein-Mischung als auch eine der oben genannten, von der env-Sequenz in SEQ ID NO: 1 oder SEQ ID NO: 11 oder einem Fragment derselben abgeleitete Nukleinsäuremischung umfaßt.

Die vorliegende Erfindung wird nachfolgend anhand von Beispielen, Figuren und einem Sequenzprotokoll erläutert.

### BEISPIELE

1. Allgemeine Beschreibung des Herstellungsverfahrens
   1.1 Klonierung von V3-loop kodierenden Oligonukleotiden
   1.2 Klonierung der V3-env-Varianten
   1.3 Analyse der Variabilität
2. Material und Methoden
   2.1 Abkürzungen
      2.1.1 Allgemeine Abkürzungen
      2.1.2 Nukleinsäuren
   2.2 Bakterienstämme
   2.3 Plasmide
   2.4 Enzyme
   2.5 Chemikalien
   2.6 Oligonukleotide
   2.7 Molekulargewichtsstandards
   2.8 Verwendete Reagenzien-Kits
   2.9 Medien
   2.10 Sterilisieren von Lösungen
   2.11 Anzucht und Lagerung von Bakterien
   2.12 Herstellung kompetenter Zellen
   2.13 Transformation in E.coli
   2.14 Plasmid-DNA Präparation
   2.15 Auftrennung von DNA in Agarosegelen
   2.16 Reinigung von DNA aus Agarosegelen
   2.17 Auftrennung von DNA in Polyacrylamidgelen
   2.18 Schneiden von DNA
   2.19 Ligation von DNA
   2.20 DNA-Sequenzierung
   2.21 Herstellung doppelsträngiger DNA mit Hilfe von Oligonukleotiden
   2.22 Transfektion von COS-Zellen und CHO-Zellen
   2.23 Chromatographische Reinigung der GP120-Mischung
   2.24 Herstellung der DNA-Vakzine

### 1. Allgemeine Beschreibung des Herstellungsverfahrens

### 1.1 Klonierung einer Mischung von V3-loop gp120-Varianten

Die Oligonukleotide werden wie unter 2.6 beschrieben synthetisiert. Als V3-loop-Sequenz ist beispielhaft eine mutierte Sequenz des HIV-1 Patientenisolates F1-01 (M. Schreiber et al., J. Virol. 68 Nr. 6 (1994) 3908-3916) angegeben. Jede andere Sequenz oder Mischung von Sequenzen ist ebenfalls möglich. Um viele verschiedene Varianten einer V3-loop-Sequenz zu klonieren, werden an bestimmten Positionen der Sequenz anstelle von reinen Nukleotidbausteinen Gemische verwendet. Auf diese Weise erhält man ein Gemisch von Oligonukleotiden, die sich alle in der Sequenz unterscheiden und somit für unterschiedliche V3-loops kodieren. Man bezeichnet solche Oligonukleotid-Mischungen auch als Oligonukleotide mit degenerierten Sequenzen. Ausgehend von chemisch synthetisierten Oligonukleotiden mit degenerierten Sequenzen wird der für die verschiedenen V3-loops kodierende Bereich dargestellt.

Ein Oligonukleotide in Leseraster-Orientierung (forward) wird für den env-Sequenzabschnitt von der BglII Schnittstelle bis zur ersten degenerierten Position synthetisiert. Ein zweites Oligonukleotid, in komplementärer Orientierung (reverse) wird entsprechend der Sequenz ab einer Position, die 15 Basen vor dem Beginn des variablen Bereiches liegt synthetisiert (Methode 2.6). Durch Überlappung der 3'-Region auf einer Länge von insgesamt 15 Basen erfolgt die Hybridisierung beider Oligonukleotide. In einer anschließenden Reaktion mit DNA-Polymerase (z.B. Taq DNA Polymerase oder Klenow-Fragment) entsteht aus den beiden hybridisierten Oligonukleotide ein vollständig doppelsträngiges DNA-Molekül (Methode 2.21).

Das so erhaltene DNA-Gemisch wird mit den Restriktionsendonucleasen BglII und XbaI verdaut. Die Klonierung des DNA-Gemisches erfolgt in den mit BglII und XbaI geschnittenen Expressionsvektor ΔV3-pBSCenvV3 (Methoden 2.15 und 2.16).

Dieser Vektor enthält die kodierende Sequenz des *gp160* des HIV-1 Stammes NL4-3 (IIIB). Das NL4-3 env Gen wurde so manipuliert, daß es die Restriktionschnittstellen BglII und XbaI sowie ApaLI, PstI und BclI nur jeweils einmal besitzt. Der für den V3 loop kodierende Bereich, der zwischen den Schnittstellen BglII und XbaI liegt, wurde entfernt und durch eine 15 Basenpaar Sequenz ersetzt, wodurch eine analytische Schnittstelle für das Enzym AscI eingeführt wird. In diesen Vektor wird das BglII und XbaI geschnittene V3-loop DNA-Gemisch kloniert (Methode 2.19). Der Verlust der AscI Schnittstelle im fertigen V3-loop pBSCenvV3 Vektor kann für die Selektion der V3-loop kodierenden Klone benutzt werden (Methode 2.18).

Das so entstandene Plasmidgemisch wird in DH5α-Bakterien transformiert (Methode 2.12), wobei eine Transformationsrate von >10⁵ erreicht werden soll. Dadurch erhält man eine Genbibliothek von V3-loop-Fragmenten mit einer Größe von ca. 10⁵ Klonen. Das Gemisch soll durch DNA-Sequenzierung analysiert werden (siehe 1.3). Mit Hilfe dieses Verfahrens erhält man ein Gemisch von Klonen, die alle für verschiedene V3-loop Varianten des GP120 Proteins kodieren. Dieses Gemisch von Vektoren dient als Ausgangsprodukt für die Herstellung des Proteingemisches für die Verwendung als Impfstoff und als Ausgangsprodukt für die Anwendung als DNA Vaccine.

Für die Herstellung der GP120-Proteinmischung werden die pBSCenvV3 Expressionvektoren in COS-Zellen und CHO-Zellen transfiziert (Methode 2.22). Die Reinigung der verschiedenen GP120-Proteine, der Wirkstoffmischung, wird, wie in der Literatur beschrieben, nach Methode 2.23 durchgeführt.

### 1.2 Klonierung einer Mischung von V2-loop gp120-Varianten

Varianten des V1-loop und V2-loop werden in gleicher Weise hergestellt. Der Expressionsvektor ΔV3-pBSCenvV3 besitzt dafür drei zusätzliche Restriktionsschnittstellen. Die Variation des V1-loop erfolgt durch Klonierung in die ApaLI und PstI Schnittstellen. Die Variation des V2-loop erfolgt durch Klonierung in die PstI und BclI Schnittstellen.

### 1.3 Analyse der Variabilität

Die Analyse der V3-loop-Gemische erfolgt durch DNA-Sequenzierung (Methode 2.20). Geplant ist die statistische Auswahl von ca. 100-200 Klonen, deren V1-, V2- und V3-loop Sequenzen bestimmt werden sollen. Sind alle diese Klone unterschiedlich, kann mit Hilfe einer statistischen Berechnung die Heterogenität der Genbibliothek und somit auch die Heterogenität der Proteinmischung bestimmt werden.

### 2. Material und Methoden

### 2.1 Abkürzungen

### 2.1.1 Allgemeine Abkürzungen

| | |
|---|---|
| µg | Mikrogramm |
| µl | Mikroliter |
| µmol | Mikromol |
| APS | Ammoniumpersulfat |
| bp | Basenpaare |
| dNTP | Desoxynukleosidtriphosphat |
| DNA | Desoxyribonukleinsäure |
| DTT | Dithiothreitol |
| EDTA | Ethylendiamintetraessigsäure |
| g | Gramm |
| GP | Glykoprotein |
| h | Stunde |
| HIV | Human Immunodeficiency Virus |
| IPTG | Isopropyl-β-D-thiogalaktopyranosid |
| MOPS | 3-Morpholinopropansulfonsäure |
| OD | Optische Dichte |
| PAGE | Poly-Acrylamid-Gel-Elektrophorese |
| PCR | Polymerase-Ketten-Reaktion |
| RT | Raumtemperatur |
| TEMED | N,N,N',N'-Tetramethylethylendiamin |

### 2.1.2 Nukleinsäuren

| | |
|---|---|
| A | Adenin |
| C | Cytosin |
| G | Guanin |
| T | Thymidin |

### 2.2 Bakterienstämme

| | |
|---|---|
| Escherichia coli DH5α: | F-, endA1, hsdr17, (rk-mk+), supE44, recA1, λ-, gyrA96, relA1, Φ80d lac z ΔM15 |

### 2.3 Plasmide

| | |
|---|---|
| pBSCenvATG | Eigenkonstruktion, die Sequenz ist in SEQ ID NO: 10 angegeben. |

### 2.4 Enzyme

| | |
|---|---|
| Restriktionsenzyme | MBI-Fermentas, Gibco-BRL, Biolabs |
| DNA-Polymerasen | MBI-Fermentas, Gibco-BRL |
| T4-DNA-Ligase | MBI-Fermentas |

### 2.5 Chemikalien

| | |
|---|---|
| [α-35S]dATP | Amersham Life Science |
| Agarose, ultra pure | GIBCO BRL |
| Ammoniumpersulfat | Merck |
| Ampicillin | US Biochemical |
| Bacto-Agar | Becton Dickinson |
| Bacto-Trypton | Becton Dickinson |
| Borsäure | Merck |
| Bromphenolblau | Merck |
| Calciumchlorid | Merck |
| Desoxyribonukleotide | MBI-Fermentas |
| Dithiothreitol | Biotechnik, St. Leon-Rot |
| Eisessig | Merck |
| Ethidiumbromid | Sigma |
| Ethylendiamintetraessigsäure | Merck |
| Glycerin | Merck |
| Harnstoff | ICN Biomedicals |
| Hefeextrakt | Becton Dickinson |
| Kaliumchlorid | Merck |
| Kaliumdihydrogenphosphat | Merck |
| Magnesiumchlorid | Merck |
| Acrylamid-Mix | Roth |
| Natriumacetat | Merck |
| Natriumchlorid | Merck |
| di-Natriumhydrogenphosphat | Merck |
| Natriumdihydrogenphosphat | Merck |
| 2-Propanol | Merck |
| Sigmacote (Chloriertes Polysiloxan) | Sigma |
| N,N,N',N'-Tetramethylethylendiamin | Merck |
| Tris(hydroxymethyl)-aminomethan | GIBCO BRL |

### 2.6 Oligonukleotide

Alle aufgeführten Oligonukleotide wurden mit dem Expedite™ Nucleic Acid Synthesis System der Firma PE Biosystems (Weiterstadt) hergestellt. Für die Klonierung von env Genen, die sich nur in der Sequenz für den V3 loop unterscheiden, werden Oligonukleotide verwendet. Am Beispiel der Klonierung der V3 loop Region für das HIV-1-Patienteisolat F1-01 sind die Sequenzen der Oligonukleotide aufgeführt.

V3-Loop: für die Klonierung in pNL4-3/BgIII-NheI, F1-01, forward:

V3-Loop: für die Klonierung in pNL4-3/BglII-NheI, F1-01:

Für die Sequenzierung von V3 loop-Klonen wurden die Oligonukleotide 7010, 7011 und die M13-Standard-Primer verwendet (M13, M13r).

### 2.7 Molekulargewichtsstandards

| | |
|---|---|
| 1 kb-Leiter | MBI-Fermentas |
| 100 bp Leiter | MBI Fermentas |

### 2.8 Verwendete Reagenzien-Kits

| | |
|---|---|
| T7-Sequenzier-Kit | Pharmacia |
| Qiaquick PCR Purification Kit | Qiagen |
| Qiagen Plasmid Kit | Qiagen |

### 2.9 Medien

| YT-Medium: | |
|---|---|
| 10 g | Trypton |
| 5 g | Hefeextrakt |
| 5 g | NaCl |

| dYT-Medium: | |
|---|---|
| 16 g | Trypton |
| 10 g | Hefeextrakt |
| 5 g | NaCl |

| dYT-Agarplatten: | |
|---|---|
| 10 g | Trypton |
| 5 g | Hefeextrakt |
| 5 g | NaCl |
| 15 g | Agar |

Die angegebenen Mengen beziehen sich auf jeweils 1000 ml deionisiertes Wasser. Autoklaviert wurden die Ansätze bei 121°C und 1,5 bar für 20 min. Sollten die Medien Antibiotika oder andere hitzeempfindliche Reagenzien enthalten, wurden die entsprechenden Mengen sterilfiltriert und nach dem Abkühlen des Mediums zugegeben.

| | |
|---|---|
| Ampicillin | 3,3 ml/l (60 mg/ml) |
| IPTG | 3 ml/l (100 mM) |
| xgal | 3 ml /l (2% in DMF, nicht filtrieren) |

### 2.10 Sterilisieren von Lösungen und Geräten

Lösungen und Medien sowie Pipettenspitzen, Eppendorfgefäße und Glasgeräte wurden 20-40 min bei 121°C und 1,5 bar autoklaviert. Hitzeempfindliche Lösungen wie z.B. Antibiotika- und IPTG-Lösungen wurden sterilfiltriert.

### 2.11 Anzucht und Lagerung von Bakterien

Bakterienkulturen wurden jeweils mit einer einzelnen Kolonie angeimpft. Zur Isolierung einer Einzelkolonie wurden Zellen einer Flüssigkultur auf einer Agarplatte ausgestrichen. Nach Inkubation bei 37°C über Nacht sind vereinzelte Kolonien gewachsen. Zur kurzfristigen Aufbewahrung der Bakterien wurden die Agar-Platten mit Parafilm versiegelt und bei 4°C-gelagert. Für eine dauerhafte Lagerung von Bakterienstämmen wurden 0,75 ml einer YT-Übernachtkultur mit 0,25 ml sterilem Glycerin vermischt, in flüssigem Stickstoff schockgefroren und bei -70°C aufbewahrt.

### 2.12 Herstellung kompetenter Zellen

100 ml YT-Medium wurden mit 100 µl einer Übernachtkultur angeimpft. Die Bakterien wurden bis zum Erreichen einer OD₅₆₀=0,4 bei 37°C im Schüttelinkubator inkubiert und anschließend 8 min bei 4°C und 1000 g zentrifugiert. Alle anschließenden Arbeiten erfolgten auf Eis unter Verwendung vorgekühlter Gefäße und 4°C kalter Lösungen. Die Zellen wurden in 50 ml 50 mM CaCl₂ resuspendiert und 30 min auf Eis inkubiert. Nach erneutem Zentrifugieren wurden die Bakterien in 2,5 ml sterilem TFBII-Puffer aufgenommen und in Portionen von jeweils 100 µl aufgeteilt. Die 100 µl Portionen der kompetenten Zellen konnten, soweit sie nicht für eine sofortige Transformation benötigt wurden, nach Schockgefrieren in flüssigem Stickstoff bei -70°C gelagert werden.

| TFBII-Puffer : | |
|---|---|
| 10 mM | MOPS pH 7,0 |
| 75 mM | CaCl₂ |
| 10 mM | KCl |
| 15% | Glycerin |

### 2.13 Transformation von E. coli

(Hanahan, J. Mol. Biol. 166 (1983) 557-580)
Eine 100 µl Portion kompetenter Zellen wurde im Eisbad aufgetaut und mit 1-100 ng Plasmid-DNA für 30 min bei 0°C inkubiert. Anschließend wurde der Transformationsansatz 1 min bei 42°C inkubiert. Danach wurde 1 ml YT-Medium zugegeben und bei 37°C für eine Stunde geschüttelt. Um eine Selektion der transformierten Zellen zu ermöglichen, wurden 100-500 µl des Ansatzes auf antibiotikahaltigen YT-Agarplatten ausgestrichen. Nach Inkubation über Nacht bei 37°C sind nur Kolonien gewachsen, die das plasmid-kodierte Resistenzgen tragen. Bei einer Transformation von Zellen, die keine funktionsfähige β-Galaktosidase enthalten (lacZΔM15 Mutation, z.B. DH5α), ermöglichen Vektoren wie der verwendete pUC über eine plasmid-kodierte β-Galaktosidase eine direkte Selektion (*blue white screening)* rekombinanter Bakterien. Für die Blau-Weiß-Selektion wurden die Bakterien auf Amp/IPTG/Xgal-YT-Agarplatten ausgestrichen. Das Substrat Xgal wird durch die β-Galaktosidase in einen blauen Farbstoff gespalten. Aufgrund der Zerstörung des Leserasters des lacZ-Gens durch die Insertion eines fremden DNA-Fragments in das lacZ-Gen werden weiße Kolonien erzeugt. Dagegen bedeuten blaue Kolonien, daß das lacZ-Gen bei der Klonierung und Ligation funktionsfähig geblieben ist und keine DNA inseriert wurde.

### 2.14 Plasmid-DNA Präparation

(Quiagen, Hilden)
Um Plasmid-DNA aus *E. coli* zu isolieren, wurde der QIAprep Spin Miniprep Kit der Firma Qiagen verwendet. Die DNA-Präparation erfolgte nach Anleitung des Herstellers (QIAprep Plasmid Handbook 03/95). Plasmidhaltige *E*. *coli*-Bakterien wurden in dYT-amp-Medium angeimpft und bei 37°C über Nacht geschüttelt. Drei ml der *E*. *coli*-Übernachtkultur wurden für die Plasmid Isolierung eingesetzt. Die Bakterein wurden geerntet (1min, 14.000 U/min, Heraeus Zentrifuge) und in 250 µl P1-Puffer aufgenommen. Durch alkalische Lyse wurden die Bakterien aufgeschlossen (250 µl, 0,2N NaOH/1%-SDS). Durch Zugabe von 3M Kaliumacetat, (350 µl, pH 5,5) wurde die Mischung neutralisiert. Die Aufreinigung der Plasmid-DNA basiert auf der selektiven Bindung von Plasmid-DNA an DEAE-Anionenaustauschersäulen. Bei den gewählten Salzkonzentrationen und pH-Bedingungen bindet die Plasmid-DNA an der DEAE-Matrix. Die DEAE-Matrix wurde gewaschen (750 µl, PE-Puffer, Qiagen). Die Plasmid-DNA wurde anschließend mit 50 µl H₂O eluiert und bei -20°C gelagert.

Um Plasmid-DNA in größerer Menge (ca. 100 µg) zu erhalten, wurden 25 ml Übernachtkultur eingesetzt. Für die Aufreinigung der DNA wurde der QIAGEN Plasmid Midi Kit verwendet. Die Aufreinigung beruht hier auf dem unter dem "QIAprep Spin Miniprep Kit" beschriebenen Prinzip und wurde nach Herstellerangaben (QIAGEN Plasmid Purification Handbook 01/97) durchgeführt.

### 2.15 Auftrennung von DNA in Agarosegelen

Die Trennung von DNA erfolgte durch Gelelektrophorese in Agarosegelen. In Abhängigkeit von der Größe der untersuchten Fragmente wurden 0,8-2% Agarose in TBE-Puffer (für analytische Agarosegele) oder TAE-Puffer (für präparative Agarosegele) durch Aufkochen gelöst. Nach dem Abkühlen auf ca. 60°C wurde die Gelflüssigkeit mit 1 µl Ethidiumbromid (10 mg/ml) versetzt und in ein vorbereitetes Gelbett mit eingesetztem Kamm gegossen. Das vollständig abgekühlte Gel wurde in einer Elektrophoresekammer mit TBE-, bzw. TAE-Puffer überschichtet und der Kamm entfernt. Die DNA-Proben wurden mit 1/5 Vol. Auftragspuffer gemischt und in die Probentaschen pipettiert. Die Fragmente wurden bei 5-10 Volt/cm Gellänge 0,5-2 h aufgetrennt. Zur Detektion wurde das Gel nach der Elektrophorese im UV-Durchlicht photographiert. Die Molekulargewichte der DNA-Banden wurden im Vergleich zu mitlaufenden DNA-Molekulargewichtsmarkern bestimmt.

| TBE-Puffer : | |
|---|---|
| 100 mM | Tris |
| 100 mM | Borsäure |
| 3 mM | EDTA |

| TAE-Puffer : | |
|---|---|
| 40 mM | Tris |
| 2mM | EDTA |
| 0,114% | Eisessig |

| Auftragspuffer : | |
|---|---|
| 0,25% | Bromphenolblau |
| 0,25% | Xylencyanol |
| 30% | Glycerin |
| 50 mM | EDTA |

### 2.16 Reinigung von DNA aus Agarosegelen

Für die Extraktion der DNA aus Agarosegelen wurde der "QIAquick Gel Extraction Kit" (Qiagen, Hilden) verwendet. Die Pufferbedingungen wurden so gewählt, daß die Nukleinsäuren an die Silica-Membran der Säulen binden, während niedermolekulare Bakterienbestandteile die Membran passieren (Methode 2.15). Es wurde nach dem "QIAquick Gel Extraction Kit Protokoll" des QIAqick Spin Handbooks 07/97 vorgegangen. Die gereinigte DNA wurde jeweils mit 50 µl H₂O von der Silica-Membran eluiert.

### 2.17 Auftrennung von DNA in Polyacrylamidgelen

Die Trennung von radioaktiv markierten DNA-Fragmenten zur DNA-Sequenzierung wurde unter Verwendung von denaturierenden Polyacrylamidgelen durchgeführt. Zwei gereinigte Glasplatten wurden mit Ethanol von Fettresten befreit und mit 1 ml Sigmacote pro Platte beschichtet, um eine hydrophobe Oberfläche zu erhalten. Durch die Beschichtung sollte ein späteres Zerreißen des Gels beim Abziehen von der Glasplatte vermieden werden. Zwischen die beiden Glasplatten wurden Abstandshalter (*Spacer*) gelegt, welche gleichzeitig zum seitlichen Abdichten dienten. Die ganze Apparatur wurde mit mehreren Klammern fixiert. Zur Herstellung des Sequenzgels wurden 21 g Harnstoff in etwa 20 ml Wasser gelöst. Nach Zugabe von 7,5 ml Acrylamid-Mix und 5 ml 10x TBE-Puffer (siehe 2.15) wurde der Ansatz mit Wasser auf 50 ml aufgefüllt. Die Polymerisation des Gels wurde durch Zugabe von 300 µl APS und 100 µl TEMED gestartet. Sofort danach wurde die Gellösung in die vorbereitete Gelapparatur gegossen, und durch Einsetzen des flachen Rückens des Sägezahnkamms der Taschenboden geformt. Bis zur vollständigen Polymerisation wurde das Gel waagerecht bei Raumtemperatur gelagert. Nach dem Einsetzen des Gels in die Gelkammer wurde diese mit TBE-Puffer gefüllt. Durch Umdrehen des Sägezahnkammes wurden die Auftragstaschen gebildet. Um das Gel auf die optimale Betriebstemperatur zu erwärmen, wurde ein Vorlauf von 20 min durchgeführt. Die Taschen wurden gründlich mit TBE-Puffer gespült und anschließend mit 4-5 µl der Proben gefüllt. Die Elektrophorese erfolgte bei 2 kV (150 Watt) für etwa 2-3 h. Das Gel wurde nach Beenden der Elektrophorese aus der Gelkammer genommen und eine der Glasplatten vorsichtig abgehoben. Durch Auflegen und leichtes Andrücken wurde das Gel auf Schleicher & Schuell-Papier (Whatman, England) übertragen. Nach dem Trocknen bei 80°C unter Vakuum wurde das Gel 1-3 Tage bei RT auf einem Röntgen-Film (Kodak BioMax MR-1, Sigma Deisenhofen) exponiert.

| Acrylamid-Mix: | |
|---|---|
| 40% | Polyacrylamid |
| 0,8% | Bisacrylamid |

### 2.18 Schneiden von DNA

Restriktionsendonukleasen erkennen und hydrolysieren enzymspezifische palindrome DNA-Sequenzen von meist 4-8 Nukleotiden Länge. Bei der Hydrolyse entstehen je nach Art des Enzyms stumpfe *(blunt ends)* oder überhängende Enden einzelsträngiger DNA (*sticky ends*). Für einen Restriktionsverdau wurden 0,1-60 µg DNA mit 2-120 Einheiten (*Units*) eines Restriktionsenzyms in dem vom Hersteller angegebenen Puffer für 2-5 h bei 37°C inkubiert. Das Gesamtvolumen betrug zwischen 10 µl für analytische und 300 µl für präparative Ansätze. Für Restriktionen mit zwei verschiedenen Enzymen wurde ein Puffer gewählt, in dem beide Enzyme eine ausreichende Aktivität besitzen, z.B. den EcoRI-Puffer für einen EcoRI/BamHI-Verdau, oder es wurden nach Inkubation mit dem ersten Enzym die Bedingungen für das zweite Enzym eingestellt.

### 2.19 Ligation von DNA

DNA-Ligasen katalysieren die Verknüpfung von DNA-Molekülen unter Verbrauch von NAD⁺ oder ATP durch Bildung einer Phosphodiesterbindung zwischen einer freien 5'-Phosphatgruppe und einer 3'-Hydroxylgruppe. Ein drei- bis fünffacher Überschuß an DNA-Fragment wurde mit 100-500 ng geschnittenen Plasmids und 5 Einheiten T4-DNA-Ligase sowie 10 nmol ATP über Nacht bei 12°C in Ligationspuffer inkubiert. Es wurden nur Ligationen kohäsiver Enden durchgeführt.

| Ligationspuffer: | |
|---|---|
| 40 mM | Tris-HCl pH 7,8 |
| 10 mM | MgCl2 |
| 10 mM | DTT |
| 0,5 mM | ATP |

### 2.20 DNA-Sequenzierung

(Sanger et al., Proc. Natl. Acad. Sci. USA 74 (1977) 5463-5467)
Die Sequenzierung von DNA wurde nach der Kettenabbruchmethode durchgeführt. Als Substrat diente doppelsträngige Plasmid-DNA. Aus Einzelstrang-DNA kann ausgehend von einem Oligonukleotidprimer in Anwesenheit von dNTPs durch eine DNA-Polymerase doppelsträngige DNA synthetisiert werden. Ist im Nukleotidgemisch ein geringer Anteil Didesoxynukleotide (ddNTPs) enthalten, führt dies zu einem zufällig verteiltem Einbau des ddNTPs, da die DNA-Polymerase nicht zwischen dNTPs und ddNTPs unterscheiden kann. Der Einbau führt aufgrund der fehlenden 3'-Hydroxylgruppe der ddNTPs zu einem Abbruch der Doppelstrangsynthese und, da er statistisch verteilt erfolgt, zu unterschiedlich langen DNA-Einzelsträngen. Da der Syntheseansatz viergeteilt ist und in jeder Probe nur eines der vier ddNTPs vorhanden ist, kommt es in den jeweiligen Ansätzen zu basenspezifischen Kettenabbrüchen. Zur Markierung des synthetisierten Einzelstranges wird der Reaktion α-³⁵S-dATP zugegeben. Nach Denaturierung und Trennung der DNA über Polyacrylamidgelelek-trophorese lassen sich die Banden autoradiographisch detektieren und die Sequenz des DNA-Stranges direkt ablesen.

Durchgeführt wurden die Sequenzierungen mit dem T7-Sequenzier-Kit (Pharmacia) nach der Anleitung des Herstellers. Als Oligonukleotidprimer wurde dabei entweder der M13-Universal-Primer (Pharmacia) oder Primer M13r verwendet. 32 µl (ca. 2 µg) gereinigter doppelsträngiger Plasmid-DNA wurden mit 8 µl 2 M NaOH für 10 min bei RT denaturiert. Nach Zugabe von 7 µl 3 M Natriumacetat pH 4,8, 4 µl H20 und 120 µl -20°C Ethanol wurde die DNA 20 min bei -70°C gefällt. Durch Zentrifugieren (15 min, 4°C) wurde die DNA isoliert, zweimal mit -20°C kaltem 70% Ethanol gewaschen und in einer Vakuumzentrifuge getrocknet. Das DNA-Sediment wurde anschließend in 10 µl H₂O resuspendiert und nach Zugabe von 2 µl Annealing-Puffer und 2 µl Primerlösung (10 pmol in Wasser) bei 65°C für 5 min, 37°C für 10 min und 5min bei RT mit dem Oligonukleotidprimer hybridisiert. Direkt darauf wurden zwecks Primerelongation und radioaktiver Markierung 3 µl *Labelling*-Mix, 1 µl α-³⁵S-dATP (1000 Ci/mmol, 10 µCi/µl) und 2 µl T7-Polymeraselösung (mit *Enzyme Dilution* Puffer 1:5 verdünnt) zugegeben und 5 min bei RT inkubiert. Jeweils 4,5 µl dieses Ansatzes wurden auf eine, auf 37°C vorgewärmte *MicroSample Plate* (Greiner) gegeben, in der je 2,5 µl der vier verschiedenen dNTP/ddNTP-Mixe vorlagen. Nach fünfminütiger Inkubation bei 37°C, die der weiteren Elongation und basenspezifischen Termination diente, wurden die Reaktionen durch Zugabe von 5 µl Stoplösung beendet. Vor dem Auftrag auf das Polyacrylamidgel wurden die Proben für 2 min bei 80°C denaturiert. Gelagert wurden die Proben bei -20°C.

| Annealing-Puffer: | |
|---|---|
| 1 M | Tris-HCl pH 7,6 |
| 100 mM | MgCl₂ |
| 160 mM | DTT |

| Labelling-Mix: | |
|---|---|
| 1,375 µM | dCTP |
| 1,375 µM | dGTP |
| 1,375 µM | dTTP |
| 333,5 mM | NaCl |

| Enzyme Dilution Puffer: | |
|---|---|
| 20 mM | Tris-HCl pH 7,5 |
| 5 mM | DTT |
| 100 µg/ml | BSA |
| 5% | Glycerin |

| Stoplösung: | |
|---|---|
| 10 mM | EDTA |
| 97,5% | Formamid |
| 0,3% | Bromphenolblau |
| 0,3% | Xylencyanol |

| | | | |
|---|---|---|---|
| A-Mix | 840 µM dCTP | C-Mix | 840 µM dATP |
| | 840 µM dGTP | | 840 µM dGTP |
| | 840 µM dTTP | | 840 µM dTTP |
| | 93,5 µM dATP | | 93,5 µM dCTP |
| | 14 µM ddATP | | 14 µM ddCTP |
| | 40 mM Tris-HCl pH 7,6 | | 40 mM Tris-HCl pH 7,6 |
| | 50 mM NaCl | | 50 mM NaCl |

| | | | |
|---|---|---|---|
| G-Mix | 840 µM dATP | T-Mix | 840 µM dATP |
| | 840 µM dCTP | | 840 µM dCTP |
| | 840 µM dTTP | | 840 µM dGTP |
| | 93,5 µM dGTP | | 93,5 µM dTTP |
| | 14 µM ddGTP | | 14 µM ddTTP |
| | 40 mM Tris-HCl pH 7,6 | | 40 mM Tris-HCl pH 7,6 |
| | 50 mM NaCl | | 50 mM NaCl |

### 2.21 Herstellung doppelsträngiger DNA mit Hilfe von Oligonukleotiden

Für die Herstellung des DNA-Gemisches wurde die Oligonukleotide bei 60°C hybridisiert. Je Oligonukleotid wurden 100 pmol eingesetzt. Von der hybridisierten Probe wurden 1 pmol für die PCR oder 100 pmol für die Klenow-Reaktion eingesetzt.

Für die PCR wurden folgende Zyklen durchgeführt:

| | | |
|---|---|---|
| Am Anfang | 10 min | 94°C |
| 30 Zyklen | 1 min | 94°C |
| | 1 min | 45°C |
| | 1 min | 72°C |
| Am Schluß | 10 min | 72°C |

Als DNA-*template* für die PCR wurden 1 pmol hybridisierte Oligonukleotide eingesetzt. PCR-Primer wurden bei allen durchgeführten PCR-Reaktionen in einer Endkonzentration von 0,1 pmol/µl eingesetzt. Von der Taq-Polymerase wurden 1 Unit pro 50 µl des Reaktionsansatzes benutzt. Die Konzentration der dNTPs im PCR-Ansatz wurde auf 0,1 mM eingestellt.

Für die Herstellung des DNA-Gemisches mit Hilfe der Klenow-Reaktion wurden 100 pmol der hybridisierten Oligonukleotide in Klenow-Puffer (50 mM Tris-HCl, pH 8,0; 50 mM MgCl₂, 10 mM DTT, 0,05 mM dNTP) gelöst. Die Reaktion wurde durch Zugabe von 5 Units DNA-Polymerase I (Klenow-Fragment) gestartet. Nach 30 min bei 37°C wurde die Reaktion durch Erhitzen auf 75°C (10 min) gestoppt.

### 2.22 Transfektion von COS-Zellen und CHO-Zellen

5-10 µg linearisierte Plasmid-DNA werden in 150 µl Zellkulturmedium (ohne FKS und Antibiotika) gelöst. Der Lösung werden 30 µl des Transfektionsmittels zugegeben (SuperFect, Qiagen, Hilden). Die Mischung wird 10 min bei RT inkubiert und nach Zugabe von 1 ml Medium auf die Zellen gegeben. Die COS-Zellen (80% konfluent gewachsen) wurden in 60 mm-Platten gezüchtet und kurz vor der Transfektion mit PBS gewaschen. Nach einer Inkubation von 3 h bei 37°C und 5% CO₂ wurde das Transfektionsmedium entfernt. Anschließend wurden die Zellen 4x mit PBS gewaschen und in Selektionsmedium (DMEM, 5% FKS, 600µg/ml Geneticin) aufgenommen.

### 2.23 Chromatographische Reinigung der GP120-Mischung

Die chromatographische Reinigung erfolgt nach Standardmethoden (Techniques in HIV Research, Aldovini & Walker, Stockton Press, 1990; S.W. Pyle et al. Purification of 120,00 dalton envelope glycoprotein from culture fluids of human immunodeficiency virus (HIV)-infected H9 cells. AIDS Res Hum Retroviruses 1987, 3:387-400). Zellextrakte und Zellkulturüberstände von GP120-exprimierenden COS-Zellen werden für die Isolierung der GP120-Mischung eingesetzt. Nach Zentrifugation (25 000 g) wird das Lysat wie folgt aufgereinigt:
1. Gelfiltration (Sephadex G-20)
2. Immunoaffinitätschromatographie
   (anti-GP120-Antikörper gebunden an CH-Sepharose 4B)
3. Anreicherung durch Proteinfällung
4. Anreicherung durch Dialyse

### 2.24 Herstellung der DNA-Vakzine

Ausgangsmaterial für die Herstellung der DNA-Vakzine ist die Mischung der BstEII-BamHI DNA-Fragmente des *env*-Gens, die für die Herstellung der GP120-Protein Mischung verwendet wurden. Ein eukaryotischer Expressionsvektor für das HIV-1 GP120 der für DNA-Vakzinierungen zugelassen ist (vgl. z.B. J.D. Boyer et al. , J Infect Dis 1997, 176:1501-1509; J.D. Boyer et al., Nat Med 1997, 3:526-532; M.L. Bagarazzi et al., J Med Primatol 1997, 26:27-33) wird so verändert, daß sich im *env*-Gen die Schnittstellen für BstEII und BamHI an identischen Stellen des *gp120*-Leserasters befinden. In einen solchen Vektor werden die mutierten, variablen *env*-Genfragmente (BstEII-BamHI) kloniert. Die Veränderung der *env*-Gene im V2-Loop- und V3-Loop-Bereich und deren Klonierung in den DNA-Vakzine-Vektor erfolgt analog der Klonierungsschritte die für die Herstellung der gp120-Mischung durchgeführt werden.

## Patentansprüche

1. Protein-Vakzine, die eine Mischung viraler Protein-Moleküle umfaßt, **dadurch gekennzeichnet, daß** die Moleküle Sequenzvarianten eines einzigen viralen Proteins oder eines Teils desselben sind, wobei die Mischung ≥ 10² Sequenzvarianten enthält und durch Expression einer Plasmid-DNA-Mischung erhältlich ist, die aufgrund der Variation von Nukleotidpositionen zufallsverteilte Sequenzkombinationen aufweist.

2. Protein-Vakzine nach Anspruch 1, **dadurch gekennzeichnet, daß** die Mischung ≥ 10³ und vorzugsweise ≥ 10⁴ Sequenzvarianten enthält.

3. Protein-Vakzine nach Anspruch 1 oder 2, **dadurch gekennzeichnet, daß** sie eine Mischung von GP120-Proteinen von HIV umfaßt, die sich jeweils in ihrer Aminosäuresequenz im Bereich der V2-Scheife und/oder der V3-Schleife voneinander unterscheiden.

4. DNA-Vakzine, die für eine Mischung strukturell unterschiedlicher Virus-Proteine kodiert, **dadurch gekennzeichnet, daß** die Vakzine eine Mischung von Sequenzvarianten eines viralen DNA-Moleküls oder eines Teils desselben enthält, wobei die Mischung ≥ 10² DNA-Moleküle enthält, die sich in ihrer Nukleinsäuresequenz voneinander unterscheiden, wobei die Mischung aufgrund der Variation von Nukleotidpositionen zufallsverteilte Sequenzkombinationen aufweist.

5. DNA-Vakzine nach Anspruch 4, **dadurch gekennzeichnet, daß** sie eine Mischung von DNA-Molekülen enthält, die Sequenzvarianten eines viralen Proteins oder eines Teils kodieren.

6. DNA-Vakzine nach Anspruch 4 oder 5, **dadurch gekennzeichnet, daß** die Mischung ≥ 10³ und vorzugsweise ≥ 10⁴ DNA-Moleküle enthält, die sich in ihrer Nukleinsäuresequenz voneinander unterscheiden.

7. DNA-Vakzine nach einem der Ansprüche 4 bis 6, **dadurch gekennzeichnet, daß** sie für eine Mischung strukturell unterschiedlicher GP120-Proteine von HIV kodiert, wobei die Vakzine eine Mischung von DNA Molekülen enthält, deren Nukleinsäuresequenzen sich in dem für die V2-Schleife kodierenden Bereich und/oder in dem für die V3-Schleife kodierenden Bereich voneinander unterscheiden.

8. DNA-Vakzine nach Anspruch 7, **dadurch gekennzeichnet, daß** sie eine Mischung von DNA-Molekülen enthält, die sich in ihrer Nukleinsäuresequenz derart voneinander unterscheiden, daß sie für eine Mischung von GP120-Proteinen kodieren, die in der V2-Scheife und/oder in der V3-Schleife jeweils voneinander verschiedene Aminosäuresequenzen aufweisen.

9. Nukleinsäuresequenz, die von der in SEQ ID NO: 1 dargestellten *env*-Sequenz oder einem Fragment derselben abgeleitet ist, **dadurch gekennzeichnet, daß** sie derart modifiziert ist, daß sie zehn monovalente Restriktionschnittstellen in Abständen von ca. 150 Basenpaaren enthält.

10. Nukleinsäuresequenz nach Anspruch 9, die von der in SEQ ID NO: 1 dargestellten *env*-Sequenz oder einem Fragment derselben abgeleitet ist, **dadurch gekennzeichnet, daß** sie derart modifiziert ist, daß sie monovalente Restriktionsspaltorte enthält, die den spezifischen Austausch der V2- und V3-Region ermöglichen.

11. Nukleinsäuresequenz nach Anspruch 9 oder 10, **dadurch gekennzeichnet, daß** die Sequenz durch Einführen stiller Mutationen modifiziert ist.

12. Nukleinsäuresequenz nach den Ansprüchen 9 bis 11, **dadurch gekennzeichnet, daß** sie die in SEQ ID NO: 9 angegebene Sequenz aufweist.

13. Nukleinsäuresequenz, **dadurch gekennzeichnet, daß** sie die in SEQ ID NO: 11 angegebene Sequenz aufweist.

14. Nukleinsäuresequenz, **dadurch gekennzeichnet, daß** sie die in SEQ ID NO: 12 angegebene Sequenz aufweist.

15. Einzelsträngige Nukleinsäuresequenz, die den für die V3-Schleife und/oder den für die V2-Schleife von GP120 kodierenden Bereich enthält, **dadurch gekennzeichnet, daß**
a) in der V3-Schleife ein 247 bp langes BglII-XbaI-Fragment oder ein 283 bp langes BglII-NheI-Fragment gegen ein verändertes Fragment ausgetauscht ist, das gegenüber dem ursprünglichen Fragment jeweils an mindestens 6 Positionen Inosin, einen Nukleinsäureaustausch oder eine Mutation aufweist und/oder
b) in der V2-Schleife ein 139 bp langes PstI-BclI-Fragment oder ein 339 bp langes PstI-EcoRI-Fragment gegen ein verändertes Fragment ausgetauscht ist, das gegenüber dem ursprünglichen Fragment jeweils an mindestens 6 Positionen Inosin, einen Nukleinsäureaustausch oder eine Mutation aufweist.

16. Nukleinsäuresequenz, **dadurch gekennzeichnet, daß** sie zu der in SEQ ID NO: 12 angegebenen Sequenz komplementär ist.

17. Nukleinsäuresequenz nach Anspruch 15 oder 16, **dadurch gekennzeichnet, daß** das Fragment/die Fragmente an 9 bis 20 Positionen Inosin, einen Nukleinsäureaustausch oder eine Mutation aufweist/aufweisen.

18. Doppelsträngige DNA, die Hybride der einzelsträngigen Nukleinsäuresequenz nach Anspruch 15 oder 17 mit der einzelsträngigen Nukleinsäuresequenz nach Anspruch 16 oder 17 umfaßt.

19. Nukleinsäure-Mischung, die doppelsträngige DNAs umfaßt, deren Nukleinsäuresequenzen von der env-Sequenz in SEQ ID NO: 1 oder SEQ ID NO: 11 oder einem Fragment derselben abgeleitet sind, **dadurch gekennzeichnet, daß** sich die Nukleinsäuresequenzen in dem für die V2-Schleife kodierenden Bereich und/oder in dem für die V3-Schleife kodierenden Bereich jeweils voneinander unterscheiden, wobei die Mischung aufgrund der Variation von Nukleotidpositionen zufallsverteilte Sequenzkombinationen aufweist, und wobei die Nukleinsäuresequenzen für eine Protein-Mischung kodieren, die ≥ 10² Sequenzvarianten enthält.

20. Nukleinsäure-Mischung nach Anspruch 19, **dadurch gekennzeichnet, daß** die Mischung ≥ 10³ und vorzugsweise ≥ 10⁴ Sequenzvarianten enthält.

21. Protein-Mischung, die Sequenzvarianten des GP120-Proteins umfaßt, **dadurch gekennzeichnet, daß** es eine Mischung von Proteinen ist, die in der V2-Scheife und/oder in der V3-Schleife jeweils voneinander verschiedene Aminosäuresequenzen aufweisen, wobei die Mischung ≥ 10² Sequenzvarianten enthält und durch Expression einer Plasmid-DNA-Mischung erhältlich ist, die aufgrund der Variation von Nukleotidpositionen zufallsverteilte Sequenzkombinationen aufweist.

22. Protein-Mischung nach Anspruch 21, **dadurch gekennzeichnet, daß** die Mischung ≥ 10³ und vorzugsweise ≥ 10⁴ Sequenzvarianten enthält.

23. Plasmid, welches eine doppelsträngige DNA nach Anspruch 18 insertiert enthält.

24. Expressionsvektor, **dadurch gekennzeichnet, daß** er eine Nukleinsäuresequenz nach den Ansprüchen 9 bis 14 insertiert enthält.

25. Expressionsvektor nach Anspruch 24, **dadurch gekennzeichnet, daß** er die in SEQ ID NO: 10 angegebene Sequenz aufweist.

26. Expressionsvektor, **dadurch gekennzeichnet, daß** er DSM 12612 entspricht.

27. Vektor-Mischung, die eine Mischung von Plasmiden nach Anspruch 23 enthält, **dadurch gekennzeichnet, daß** sich die Nukleinsäuresequenzen der Plasmide in dem für die V2-Schleife kodierenden Bereich und/oder in dem für die V3-Schleife kodierenden Bereich jeweils voneinander unterscheiden, wobei die Plasmid-Mischung ≥ 10² Plasmide enthält und sie aufgrund der Variation von Nukleotidpositionen zufallsverteilte Sequenzkombinationen aufweist.

28. Vektor-Mischung nach Anspruch 27, **dadurch gekennzeichnet, daß** die Mischung ≥ 10³ und vorzugsweise ≥ 10⁴ Plasmide enthält, die sich in ihrer Nukleinsäuresequenz voneinander unterscheiden.

29. Vektor-Mischung nach Anspruch 27 oder 28, **dadurch gekennzeichnet, daß** sich sich die Plamide in *E*. *coli* als Wirtszelle exprimieren lassen.

30. Vektor-Mischung nach Anspruch 27 oder 28, **dadurch gekennzeichnet, daß** sich die Plasmide in Eukaryontenzellen, vorzugsweise in Cos-, CHO- oder BHK-Zellen, als Wirtszellen exprimieren lassen.

31. *E. coli*-Wirtszellen, die mit einer Vektor-Mischung nach Anspruch 29 transfiziert sind.

32. Eukaryontische Wirtszellen, die mit einer Vektor-Mischung nach Anspruch 30 transfiziert sind.

33. Eukaryontische Wirtszellen nach Anspruch 32, **dadurch gekennzeichnet, daß** sie Wirtszellen aus der Gruppe bestehend aus Cos-, BHK- oder CHO-Zellen sind.

34. Verfahren zur Herstellung der Nukleinsäuresequenz nach Anspruch 10, **dadurch gekennzeichnet, daß** man in eine für ein virales Protein kodierende Nukleinsäuresequenz so viele stille Mutationen einführt, daß die dadurch erhaltene Nukleinsäuresequenz monovalente Restriktionsspaltorte enthält, die den Austausch der V2- und V3-Region ermöglichen.

35. Verfahren zur Herstellung der Nukleinsäuresequenz nach Anspruch 10, **dadurch gekennzeichnet, daß** man in eine für ein virales Protein kodierende Nukleinsäuresequenz so viele stille Mutationen einführt, daß die dadurch erhaltene Nukleinsäuresequenz zehn monovalente Restriktionschnittstellen in Abständen von ca. 150 Basenpaaren enthält.

36. Verfahren nach Anspruch 34 oder 35, **dadurch gekennzeichnet, daß** die für ein virales Protein kodierende Nukleinsäuresequenz die Sequenz gemäß SEQ ID NO: 1 oder SEQ ID NO: 11 oder ein Fragment derselben ist.

37. Verfahren zur Herstellung der Vektor-Mischung nach den Ansprüchen 29 und 30, **dadurch gekennzeichnet, daß** man Plasmide, deren Nukleinsäuresequenzen sich in dem für die V2-Schleife kodierenden Bereich und/oder in dem für die V3-Schleife kodierenden Bereich jeweils durch Zufallsverteilung der Basen an den variierten Nukleotidpositionen voneinander unterscheiden, in einen in Wirtszellen exprimierbaren Vektor hineinligiert.

38. Verfahren nach Anspruch 37, **dadurch gekennzeichnet, daß** die Wirtszellen *E. coli-*, Cos-, CHO- oder BHK-Zellen sind.

39. Verfahren zur Herstellung der Wirtszellen nach Anspruch 31 oder 32, **dadurch gekennzeichnet, daß** man die Wirtszellen mit einer Vektor-Mischung nach Anspruch 27 oder 28 transformiert.

40. Verfahren zur Herstellung einer Protein-vakzine nach einem der Ansprüche 1 bis 3, **dadurch gekennzeichnet, daß** man die Wirtszellen nach einem der Ansprüche 31 bis 33 unter Bedingungen kultiviert, die die Expression der Mischung viraler Protein-Sequenzvarianten gestatten.

41. Verfahren zur Herstellung einer DNA-Vakzine nach einem der Ansprüche 4 bis 8, **dadurch gekennzeichnet, daß** man das Verfahren nach Anspruch 37 oder 38 durchführt, wobei man die erfindungsgemäßen Plasmide in einen Vektor hineinligiert, der in Wirtszellen des zu vakzinierenden Organismus exprimierbar ist.

42. Verwendung einer Mischung strukturell unterschiedlicher viraler Proteine, die Sequenzvarianten eines viralen Proteins oder eines Teils desselben sind, wobei die Mischung ≥ 10² Sequenzvarianten enthält und durch Expression einer Plasmid-DNA-Mischung erhältlich ist, die aufgrund der Variation von Nukleotidpositionen zufallsverteilte Sequenzkombinationen aufweist, zur Herstellung einer Vakzine zur Prävention und/oder Therapie einer Virus-Infektion beim Menschen.

43. Verwendung einer Protein-Mischung nach Anspruch 21 oder 22 zur Herstellung einer Vakzine zur Prävention und/oder Therapie einer HIV-Infektion beim Menschen.

44. Verwendung einer Mischung von DNA-Molekülen, die für ≥ 10² Sequenzvarianten eines viralen Proteins oder eines Teils desselben kodieren, wobei die Mischung aufgrund der Variation von Nukleotidpositionen zufallsverteilte Sequenzkombinationen aufweist, zur Herstellung einer Vakzine zur Prävention und/oder Therapie einer Virus-Infektion beim Menschen.

45. Verwendung einer Nukleinsäure-Mischung nach Anspruch 19 oder 20 zur Herstellung einer Vakzine zur Prävention und/oder Therapie einer Virus-Infektion beim Menschen.

46. Verwendung der Nukleinsäure-Mischung nach Anspruch 19 oder 20zur Herstellung einer Vektor-Mischung nach Anspruch 27 oder 28 die in Wirtszellen exprimierbar ist, wobei die Wirtszellen aus der Gruppe bestehend aus *E. coli*-, Cos-, CHO- und BHK-Zellen ausgewählt ist.

47. Verwendung der Vektor-Mischung nach Anspruch 27 oder 28 zur Expression einer Protein-Mischung nach Anspruch 21 oder 22.

48. Verwendung der Wirtszelle nach einem der Ansprüche 31 bis 33 zur Herstellung einer Protein-Mischung nach Anspruch 21 oder 22.

49. Pharmazeutische Zusammensetzung zur Prävention und/oder Therapie einer Virus-Infektion, **dadurch gekennzeichnet, daß** sie eine Protein-Mischung und eine Nukleinsäuremischung umfaßt, wobei die Protein-Mischung ≥ 10² Sequenzvarianten eines viralen Proteins oder eines Teils desselben umfaßt und durch Expression einer Plasmid-DNA-Mischung erhältlich ist, die aufgrund der Variation von Nukleotidpositionen zufallsverteilte Sequenzkombinationen aufweist, und wobei die Nukleinsäuremischung ≥ 10² DNA-Moleküle umfaßt, die für Sequenzvarianten eines viralen Proteins oder eines Teils desselben kodieren, wobei die Nukleinsäuremischung aufgrund der Variation von Nukleotidpositionen zufallsverteilte Sequenzkombinationen aufweist.

50. Pharmazeutische Zusammensetzung nach Anspruch 49, **dadurch gekennzeichnet, daß** sie eine Protein-Mischung nach Anspruch 21 oder 22 und eine Nukleinsäuremischung nach Anspruch 19 oder 20 umfaßt.

## Claims

1. Protein vaccine which comprises a mixture of viral protein molecules, **characterized in that** the molecules are sequence variants of a single viral protein or of part of same, the mixture containing ≥ 10² sequence variants and being obtainable by expression of a plasmid-DNA mixture which comprises randomly distributed sequence combinations owing to variation in nucleotide positions.

2. Protein vaccine according to Claim 1, **characterized in that** the mixture contains ≥ 10³ and preferably ≥ 10⁴ sequence variants.

3. Protein vaccine according to Claim 1 or 2, **characterized in that** it comprises a mixture of GP120 proteins of HIV, which in each case differ from each other in their amino acid sequence in the region of the V2 loop and/or of the V3 loop.

4. DNA vaccine which codes for a mixture of structurally different virus proteins, **characterized in that** the vaccine contains a mixture of sequence variants of a viral DNA molecule or of part of same, the mixture containing ≥ 10² DNA molecules which differ from each other in their nucleic acid sequence, the mixture comprising randomly distributed sequence combinations owing to variation in nucleotide positions.

5. DNA vaccine according to Claim 4, **characterized in that** it contains a mixture of DNA molecules which code for sequence variants of a viral protein or of part.

6. DNA vaccine according to Claim 4 or 5, **characterized in that** the mixture contains ≥ 10³ and preferably ≥ 10⁴ DNA molecules which differ from each other in their nucleic acid sequence.

7. DNA vaccine according to one of Claims 4 to 6, **characterized in that** it codes for a mixture of structurally different GP120 proteins of HIV, in which the vaccine contains a mixture of DNA molecules, the nucleic acid sequences of which differ from each other in the region coding for the V2 loop and/or in the region coding for the V3 loop.

8. DNA vaccine according to Claim 7, **characterized in that** it contains a mixture of DNA molecules which differ from each other in their nucleic acid sequence such that they code for a mixture of GP120 proteins which contain amino acid sequences which differ from each other in the V2 loop and/or in the V3 loop.

9. Nucleic acid sequence which is derived from the env sequence represented in SEQ ID NO: 1 or from a fragment of same, **characterized in that** it is modified such that it contains ten monovalent restriction sites at intervals of about 150 base pairs.

10. Nucleic acid sequence according to Claim 9 which is derived from the env sequence represented in SEQ ID NO: 1 or from a fragment thereof, **characterized in that** it is modified such that it contains monovalent restriction sites which enable the specific exchange of the V2 and B3 regions.

11. Nucleic acid sequence according to Claim 9 or 10, **characterized in that** the sequence is modified by the introduction of silent mutations.

12. Nucleic acid sequence according to Claims 9 to 11, **characterized in that** it contains the sequence given in SEQ ID NO: 9.

13. Nucleic acid sequence, **characterized in that** it contains the sequence given in SEQ ID NO: 11.

14. Nucleic acid sequence, **characterized in that** it contains the sequence given in SEQ ID NO: 12.

15. Single-stranded nucleic acid sequence, which contains the region coding for the V3 loop and/or for the V2 loop of GP120, **characterized in that**
a) in the V3 loop a 247 bp-long BglII-XbaI fragment or a 283 bp-long BglII-NheI fragment is exchanged for a modified fragment which, compared with the original fragment, comprises in either case inosine, a nucleic acid exchange or a mutation at 6 or more positions, and/or
b) in the V2 loop a 139 bp-long PstI-BclI fragment or a 339 bp-long PstI-EcoRI fragment is exchanged for a modified fragment which, compared with the original fragment, comprises in either case inosine, a nucleic acid exchange or a mutation at 6 or more positions.

16. Nucleic acid sequence, **characterized in that** it is complementary to the sequence given in SEQ ID NO: 12.

17. Nucleic acid sequence according to Claim 15 or 16, **characterized in that** each or every fragment comprises inosine, a nucleic acid exchange or a mutation at 9 to 20 positions.

18. Double-stranded DNA which comprises hybrids of the single-stranded nucleic acid sequence according to Claim 15 or 17 with the single-stranded nucleic acid sequence according to Claim 16 or 17.

19. Nucleic acid mixture which comprises double-stranded DNAs, the nucleic acid sequences of which are derived from the env sequence in SEQ ID NO: 1 or SEQ ID NO: 11 or a fragment of same, **characterized in that** the nucleic acid sequences in each case differ from each other in the region coding for the V2 loop and/or in the region coding for the V3 loop, the mixture comprising randomly-distributed sequence combinations owing to variation in nucleotide positions and the nucleic acid sequences coding for a protein mixture which contains ≥ 10² sequence variants.

20. Nucleic acid mixture according to Claim 19, **characterized in that** the mixture contains ≥ 10³ and preferably ≥ 10⁴ sequence variants.

21. Protein mixture which comprises sequence variants of the GP120 protein, **characterized in that** it is a mixture of proteins which contain amino acid sequences which in each case differ from each other in the V2 loop and/or in the V3 loop, the mixture containing ≥ 10² sequence variants and being obtainable by expression of a plasmid-DNA mixture which comprises randomly distributed sequence combinations owing to variation in nucleotide positions.

22. Protein mixture according to Claim 21, **characterized in that** the mixture contains ≥ 10³ and preferably ≥ 10⁴ sequence variants.

23. Plasmid which contains an inserted double-stranded DNA according to Claim 18.

24. Expression vector, **characterized in that** it contains an inserted nucleic acid sequence according to Claims 9 to 14.

25. Expression vector according to Claim 24, **characterized in that** it contains the sequence given in SEQ ID NO: 10.

26. Expression vector, **characterized in that** it corresponds to DSM 12612.

27. Vector mixture which contains a mixture of plasmids according to Claim 23, **characterized in that** the nucleic acid sequences of the plasmids differ in each case from each other in the region coding for the V2 loop and/or in the region coding for the V3 loop, the plasmid mixture containing ≥ 10² plasmids and comprises randomly distributed sequence combinations owing to variation in nucleotide positions.

28. Vector mixture according to Claim 27, **characterized in that** the mixture contains ≥ 10³ and preferably ≥ 10⁴ plasmids which differ from each other in their nucleic acid sequence.

29. Vector mixture according to Claim 27 or 28, **characterized in that** the plasmids can be expressed in E. coli as host cell.

30. Vector mixture according to Claim 27 or 28, **characterized in that** the plasmids can be expressed in eukaryotic cells, preferably in Cos, CHO or BHK cells, as host cells.

31. E. coli host cells which are transfected with a vector mixture according to Claim 29.

32. Eukaryotic host cells which are transfected with a vector mixture according to Claim 30.

33. Eukaryotic host cells according to Claim 32, **characterized in that** they are host cells from the group consisting of Cos, BHK or CHO cells.

34. Process for the preparation of the nucleic acid sequence according to Claim 10, **characterized in that** so many silent mutations are introduced into a nucleic acid sequence coding for a viral protein that the nucleic acid sequence obtained as a result contains monovalent restriction sites which enable exchange of the V2 and V3 regions.

35. Process for the preparation of the nucleic acid sequence according to Claim 10, **characterized in that** so many silent mutations are introduced into a nucleic acid sequence coding for a viral protein that the nucleic acid sequence obtained as a result contains ten monovalent restriction sites at intervals of about 150 base pairs.

36. Process according to Claim 34 or 35, **characterized in that** the nucleic acid sequence coding for a viral protein is the sequence as per SEQ ID NO: 1 or SEQ ID NO: 11 or a fragment thereof.

37. Process for the preparation of the vector mixture according to Claims 29 and 30, **characterized in that** plasmids, the nucleic acid sequences of which in each case differ from each other in the region coding for the V2 loop and/or in the region coding for the V3 loop through random distribution of the bases at the varied nucleotide positions, are ligated into a vector which can be expressed in host cells.

38. Process according to Claim 37, **characterized in that** the host cells are E. coli, Cos, CHO or BHK cells.

39. Process for the preparation of the host cells according to Claim 31 or 32, **characterized in that** the host cells are transformed with a vector mixture according to Claim 27 or 28.

40. Process for the preparation of a protein vaccine according to one of Claims 1 to 13, **characterized in that** the host cells are cultivated according to one of Claims 31 to 33 under conditions which allow the expression of the mixture of viral protein sequence variants.

41. Process for the preparation of a DNA vaccine according to one of Claims 4 to 8, **characterized in that** the process is carried out according to Claim 37 or 38, the plasmids according to the invention being ligated into a vector which can be expressed in host cells of the organism to be vaccinated.

42. Use of a mixture of structurally different viral proteins which are sequence variants of a viral protein or of part of same, the mixture containing ≥ 10² sequence variants and being obtainable by expression of a plasmid-DNA mixture which comprises randomly distributed sequence combinations owing to variation in nucleotide positions, for the preparation of a vaccine for the prevention and/or therapy of a virus infection in humans.

43. Use of a protein mixture according to Claim 21 or 22 for the preparation of a vaccine for the prevention and/or therapy of an HIV infection in humans.

44. Use of a mixture of DNA molecules which code for ≥ 10² sequence variants of a viral protein or of part of same, the mixture comprising randomly distributed sequence combinations owing to variation in nucleotide positions, for the preparation of a vaccine for the prevention and/or therapy of a virus infection in humans.

45. Use of a nucleic acid mixture according to Claim 19 or 20 for the preparation of a vaccine for the prevention and/or therapy of a virus infection in humans.

46. Use of the nucleic acid mixture according to Claim 19 or 20 for the preparation of a vector mixture according to Claim 27 or 28 which can be expressed in host cells, the host cells being selected from the group consisting of E. coli, Cos, CHO and BHK cells.

47. Use of the vector mixture according to Claim 27 or 28 for the expression of a protein mixture according to Claim 21 or 22.

48. Use of the host cell according to one of Claims 31 to 33 for the preparation of a protein mixture according to Claim 21 or 22.

49. Pharmaceutical composition for the prevention and/or therapy of a virus infection, **characterized in that** it comprises a protein mixture and a nucleic acid mixture, the protein mixture comprising ≥ 10² sequence variants of a viral protein or of part of same and being obtainable by expression of a plasmid-DNA mixture which comprises randomly distributed sequence combinations owing to variation in nucleotide positions, and the nucleic acid mixture comprising ≥ 10² DNA molecules which code for sequence variants of a viral protein or of part of same, the nucleic acid mixture comprising randomly distributed sequence combinations owing to variation in nucleotide positions.

50. Pharmaceutical composition according to Claim 49, **characterized in that** it comprises a protein mixture according to Claim 21 or 22 and a nucleic acid mixture according to Claim 19 or 20.

## Revendications

1. Vaccin protéique qui comprend un mélange de molécules virales protéiques, **caractérisé en ce que** les molécules sont des variantes de séquences d'une protéine virale unique ou d'une partie de celle-ci, dans lequel le mélange contient ≥ 10² variantes de séquences, est obtenu par l'expression d'un mélange d'ADN - plasmide qui présente des combinaisons de séquences réparties de manière aléatoire sur la base de la variation des positions de nucléotides.

2. Vaccin protéique selon la revendication 1, **caractérisé en ce que** le mélange comprend ≥ 10³ et de préférence, ≥ 10⁴ variantes de séquences.

3. Vaccin protéique selon la revendication 1 ou 2, **caractérisé en ce qu'**il comprend un mélange de protéines GP120 du VIH qui se distinguent respectivement les unes des autres dans leur séquence d'acides aminés dans la zone de la boucle V2 et/ou de la boucle V3.

4. Vaccin à ADN qui code pour un mélange de protéines virales structuralement différentes, **caractérisé en ce que** le vaccin contient un mélange de variantes de séquences d'une molécule d'ADN viral ou d'une partie de celle-ci, dans lequel le mélange contient ≥ 10² molécules d'ADN qui se distinguent les unes des autres dans leur séquence d'acides nucléiques, le mélange présentant des combinaisons de séquences réparties de manière aléatoire sur la base de la variation des positions de nucléotides.

5. Vaccin à ADN selon la revendication 4, **caractérisé en ce qu'**il comprend un mélange de molécules d'ADN qui codent pour les variantes de séquences d'une protéine virale ou d'une partie de celle-ci.

6. Vaccin à ADN selon la revendication 4 ou 5, **caractérisé en ce que** le mélange contient ≥ 10³ et de préférence, ≥ 10⁴ molécules d'ADN qui se distinguent les unes des autres dans leur séquence d'acides nucléiques.

7. Vaccin à ADN selon l'une quelconque des revendications 4 à 6, **caractérisé en ce qu'**il code pour un mélange de protéines GP120 de VIH structuralement différentes, dans lequel le vaccin contient un mélange de molécules d'ADN dont les séquences d'acides nucléiques se distinguent les unes des autres dans le domaine codant pour la boucle V2 et/ou le domaine codant pour la boucle V3.

8. Vaccin à ADN selon la revendication 7, **caractérisé en ce qu'**il contient un mélange de molécules d'ADN qui se distinguent les unes des autres dans leur séquence d'acides nucléiques de telle sorte qu'elles codent pour un mélange de protéines GP120 qui présentent respectivement des séquences d'acides aminés différentes dans la boucle V2 et/ou dans la boucle V3.

9. Séquence d'acides nucléiques qui est dérivée de la séquence *env* ou d'un fragment de celle-ci représenté(e) dans le SEQ ID N° : 1, **caractérisée en ce qu'**elle est modifiée de telle sorte qu'elle contient dix sites de coupure de restriction monovalents à intervalles d'environ 150 paires de bases.

10. Séquence d'acides nucléiques selon la revendication 9 qui est dérivée de la séquence *env* ou d'un fragment de celle-ci représenté(e) dans le SEQ ID N°: 1, **caractérisée en ce qu'**elle est modifiée de telle sorte qu'elle contient des sites de clivage de restriction monovalents qui permettent l'échange spécifique de la région V2 ou de la région V3.

11. Séquence d'acides nucléiques selon la revendication 9 ou 10, **caractérisée en ce que** la séquence est modifiée par l'introduction de mutations silencieuses.

12. Séquence d'acides nucléiques selon les revendications 9 à 11, **caractérisée en ce qu'**elle présente la séquence indiquée dans le SEQ ID N°: 9.

13. Séquence d'acides nucléiques, **caractérisée en ce qu'**elle présente la séquence indiquée dans le SEQ ID N°: 11.

14. Séquence d'acides nucléiques, **caractérisée en ce qu'**elle présente la séquence indiquée par SEQ ID N° : 12.

15. Séquence d'acides nucléiques à brin simple qui contient le domaine codant pour la boucle V2 de GP120, **caractérisée en ce que**
a) un fragment BglII-XbaI d'une longueur de 247 pb ou un fragment BgIII-NheI d'une longueur de 283 pb est échangé dans une boucle V3 contre un fragment modifié qui présente par rapport au fragment d'origine, respectivement une inosine sur au moins 6 positions, un échange d'acide nucléique ou une mutation et/ou
b) un fragment PstI-BclI d'une longueur de 139 pb ou un fragment PstI-EcoRI d'une longueur de 339 pb est échangé contre un fragment modifié qui présente par rapport au fragment d'origine, respectivement une inosine sur au moins 6 positions, un échange d'acide nucléique ou une mutation.

16. Séquence d'acides nucléiques, **caractérisée en ce qu'**elle est complémentaire à la séquence indiquée dans le SEQ ID N°: 12.

17. Séquence d'acides nucléiques selon la revendication 15 ou 16, **caractérisée en ce que** le fragment/ les fragments présente(nt) une inosine sur 9 à 20 positions, un échange d'acide nucléique ou une mutation.

18. ADN à double brin qui comprend des hybrides de la séquence d'acides nucléiques à brin simple selon la revendication 15 ou 17, avec la séquence d'acides nucléiques à brin simple selon la revendication 16 ou 17.

19. Mélange d'acides nucléiques qui comprend des ADN à double brin dont les séquences d'acides nucléiques sont dérivées de la séquence *env* dans le SEQ ID N : 1 ou la SEQ ID N : 11 ou d'un fragment de celle-ci, **caractérisé en ce que** les séquences d'acides nucléiques dans le domaine codant pour la boucle V2 et/ou dans le domaine codant pour la boucle V3 se distinguent respectivement les unes des autres, dans lequel le mélange présente des combinaisons de séquences réparties de manière aléatoire sur la base de la variation des positions de nucléotides et dans lequel les séquences d'acides nucléiques codent pour un mélange protéique qui contient ≥ 10² variantes de séquences.

20. Mélange d'acides nucléiques selon la revendication 19, **caractérisé en ce que** le mélange contient ≥ 10³ et de préférence, ≥ 10⁴ variantes de séquences.

21. Mélange protéique qui comprend des variantes de séquences de la protéine GP120, **caractérisé en ce qu'**il est un mélange de protéines qui présentent des séquences d'acides aminés respectivement différentes dans la boucle V2 et/ou dans la boucle V3, dans lequel le mélange contient ≥ 10² variantes de séquences et est obtenu par l'expression d'un mélange d'ADN - plasmides qui présente des combinaisons de séquences réparties de manière aléatoire sur la base de la variation des positions de nucléotides.

22. Mélange protéique selon la revendication 21, **caractérisé en ce que** le mélange contient ≥ 10³ et de préférence, ≥ 10⁴ variantes de séquences.

23. Plasmide qui contient un ADN à double brin inséré selon la revendication 18.

24. Vecteur d'expression **caractérisé en ce qu'**il contient une séquence d'acides nucléiques insérée selon les revendications 9 à 14.

25. Vecteur d'expression selon la revendication 24, **caractérisé en ce qu'**il présente la séquence indiquée dans le SEQ ID N°: 10.

26. Vecteur d'expression, **caractérisé en ce qu'**il correspond à DSM 12612.

27. Mélange vecteur qui contient un mélange de plasmides selon la revendication 23, **caractérisé en ce que** les séquences d'acides nucléiques se distinguent respectivement les unes des autres des plasmides dans leur domaine codant pour la boucle V2 et/ou dans leur domaine codant pour la boucle V3, dans lequel le mélange de plasmides contient ≥ 10² plasmides et présente des combinaisons de séquences réparties de manière aléatoire sur la base de la variation des positions de nucléotides.

28. Mélange vecteur selon la revendication 27, **caractérisé en ce que** le mélange contient ≥ 10³ et de préférence, ≥ 10⁴ plasmides qui se distinguent les uns des autres dans leur séquence d'acides nucléiques.

29. Mélange vecteur selon la revendication 27 ou 28, **caractérisé en ce que** les plasmides sont exprimés dans E. coli comme cellule hôte.

30. Mélange vecteur selon la revendication 27 ou 28, **caractérisé en ce que** les plasmides sont exprimés dans des cellules eucaryotes, de préférence dans les cellules Cos, CHO ou BHK en tant que cellules hôtes.

31. Cellules hôtes de E. coli qui sont transfectées avec un mélange vecteur selon la revendication 29.

32. Cellules hôtes eucaryotes qui sont transfectées avec un mélange vecteur selon la revendication 30.

33. Cellule hôte eucaryote selon la revendication 32, **caractérisée en ce qu'**elle est une cellule hôte issue du groupe constitué de cellules Cos, BHK ou CHO.

34. Procédé de préparation de la séquence d'acides nucléiques selon la revendication 10, **caractérisé en ce que** l'on introduit dans une séquence d'acides nucléiques codant pour une protéine virale, un nombre de mutations silencieuses tel que la séquence d'acides nucléiques ainsi obtenue contienne des sites de clivage de restriction monovalents qui permettent l'échange de la région V2 et de la région V3.

35. Procédé de préparation de la séquence d'acides nucléiques selon la revendication 10, **caractérisé en ce que** l'on introduit dans une séquence d'acides nucléiques codant pour une protéine virale, un nombre de mutations silencieuses tel que la séquence d'acides nucléiques ainsi obtenue contienne dix sites de coupure de restriction monovalents à intervalles d'environ 150 paires de bases.

36. Procédé selon la revendication 34 ou 35, **caractérisé en ce que** la séquence d'acides nucléiques codant pour une protéine virale est la séquence selon le SEQ ID N° : 1 ou le SEQ ID N° : 11 ou un fragment de celle-ci.

37. Procédé de préparation du mélange vecteur selon les revendications 29 et 30, **caractérisé en ce que** l'on lie des plasmides dont les séquences d'acides nucléiques se distinguent respectivement les unes des autres dans leur domaine codant pour la boucle V2 et/ou dans leur domaine codant pour la boucle V3 par la répartition aléatoire des bases sur les positions de nucléotides variées, dans un vecteur exprimable dans des cellules hôtes.

38. Procédé selon la revendication 37, **caractérisé en ce que** les cellules hôtes sont des cellules de E. coli, Cos, CHO ou BHK.

39. Procédé de préparation de cellules hôtes selon la revendication 31 ou 32, **caractérisé en ce que** l'on transforme les cellules hôtes avec un mélange vecteur selon la revendication 27 ou 28.

40. Procédé de préparation d'un vaccin protéique selon les revendications 1 à 3, **caractérisé en ce que** l'on cultive les cellules hôtes selon l'une quelconque des revendications 31 à 33 dans des conditions qui permettent l'expression du mélange des variantes de séquences de la protéine virale.

41. Procédé de préparation d'un vaccin protéique selon les revendications 4 à 8, **caractérisé en ce que** l'on réalise le procédé selon la revendication 37 ou 38, sachant que l'on lie les plasmides selon la présente invention dans un vecteur qui est exprimable dans des cellules hôtes de l'organisme à vacciner.

42. Utilisation d'un mélange de protéines virales structuralement différentes qui sont des variantes de séquences d'une protéine virale ou d'une partie de celle-ci, dans lequel le mélange contient ≥ 10² variantes de séquences et est obtenu par l'expression d'un mélange ADN - plasmides qui présente des combinaisons de séquences réparties de manière aléatoire sur la base de la variation des positions de nucléotides, pour produire un vaccin destiné à la prévention et/ou au traitement d'une infection virale chez l'être humain.

43. Utilisation d'un mélange protéique selon la revendication 21 ou 22 pour la production d'un vaccin destiné à la prévention et/ou au traitement d'une infection au VIH chez l'être humain.

44. Utilisation d'un mélange de molécules d'ADN qui codent pour ≥ 10² variantes de séquences d'une protéine virale ou d'une partie de celles-ci, dans lequel le mélange présente des combinaisons de séquences réparties de manière aléatoire sur la base de la variation des positions de nucléotides, pour la production d'un vaccin destiné à la prévention et/ou au traitement d'une infection virale chez l'être humain.

45. Utilisation d'un mélange d'acides nucléiques selon la revendication 19 ou 20 pour la production d'un vaccin destiné à la prévention et/ou au traitement d'une infection au VIH chez l'être humain.

46. Utilisation d'un mélange d'acides nucléiques selon la revendication 19 ou 20 pour la préparation d'un mélange vecteur selon la revendication 27 ou 28 qui est exprimable dans des cellules hôtes, dans lequel les cellules hôtes sont sélectionnées dans le groupe constitué des cellules de E. coli, Cos, CHO et BHK.

47. Utilisation du mélange vecteur selon la revendication 27 ou 28 pour l'expression d'un mélange protéique selon la revendication 21 ou 22.

48. Utilisation de cellules hôtes selon l'une quelconque des revendications 31 à 33 pour la préparation d'un mélange protéique selon la revendication 21 ou 22.

49. Composition pharmaceutique pour la prévention et/ou le traitement d'une infection virale, **caractérisée en ce qu'**elle comprend un mélange protéique et un mélange d'acides nucléiques, dans laquelle le mélange protéique comprend ≥ 10² variantes de séquences d'une protéine virale ou d'une partie de celles-ci et qui est obtenue par l'expression d'un mélange d'ADN - plasmides qui présente des combinaisons de séquences réparties de manière aléatoire sur la base des positions de nucléotides et dans laquelle le mélange d'acides nucléiques comprend ≥ 10² molécules d'ADN qui codent pour des variantes de séquences d'une protéine virale ou une partie de celles-ci, dans laquelle le mélange d'acides nucléiques présente des combinaisons de séquences réparties de manière aléatoire sur la base de la variation des positions de nucléotides.

50. Composition pharmaceutique selon la revendication 49, **caractérisée en ce qu'**elle comprend un mélange protéique selon la revendication 21 ou 22 et un mélange d'acides aminés selon la revendication 19 ou 20.


### SEQUENZPROTOKOLL

<110> Strathmann AG & Co.
<120> Virus-Vakzine
<130> P052348
<140>
   <141>
<150> 199 07 485.2
   <151> 1999-02-12
<160> 12
<170> PatentIn Ver. 2.1
<210> 1
   <211> 9709
   <212> DNA
   <213> Human immunodeficiency virus
<400> 1
   tggaagggct aatttggtcc caaaaaagac aagagatcct tgatctgtgg atctaccaca 60
   cacaaggcta cttccctgat tggcagaact acacaccagg gccagggatc agatatccac 120
   tgacctttgg atggtgcttc aagttagtac cagttgaacc agagcaagta gaagaggcca 180
   aataaggaga gaagaacagc ttgttacacc ctatgagcca gcatgggatg gaggacccgg 240
   agggagaagt attagtgtgg aagtttgaca gcctcctagc atttcgtcac atggcccgag 300
   agctgcatcc ggagtactac aaagactgct gacatcgagc tttctacaag ggactttccg 360
   ctggggactt tccagggagg tgtggcctgg gcgggactgg ggagtggcga gccctcagat 420
   gctacatata agcagctgct ttttgcctgt actgggtctc tctggttaga ccagatctga 480
   gcctgggagc tctctggcta actagggaac ccactgctta agcctcaata aagcttgcct 540
   tgagtgctca aagtagtgtg tgcccgtctg ttgtgtgact ctggtaacta gagatccctc 600
   agaccctttt agtcagtgtg gaaaatctct agcagtggcg cccgaacagg gacttgaaag 660
   cgaaagtaaa gccagaggag atctctcgac gcaggactcg gcttgctgaa gcgcgcacgg 720
   caagaggcga ggggcggcga ctggtgagta cgccaaaaat tttgactagc ggaggctaga 780
   aggagagaga tgggtgcgag agcgtcggta ttaagcgggg gagaattaga taaatgggaa 840
   aaaattcggt taaggccagg gggaaagaaa caatataaac taaaacatat agtatgggca 900
   agcagggagc tagaacgatt cgcagttaat cctggccttt tagagacatc agaaggctgt 960
   agacaaatac tgggacagct acaaccatcc cttcagacag gatcagaaga acttagatca 1020
   ttatataata caatagcagt cctctattgt gtgcatcaaa ggatagatgt aaaagacacc 1080
   aaggaagcct tagataagat agaggaagag caaaacaaaa gtaagaaaaa ggcacagcaa 1140
   gcagcagctg acacaggaaa caacagccag gtcagccaaa attaccctat agtgcagaac 1200
   ctccaggggc aaatggtaca tcaggccata tcacctagaa ctttaaatgc atgggtaaaa 1260
   gtagtagaag agaaggcttt cagcccagaa gtaataccca tgttttcagc attatcagaa 1320
   ggagccaccc cacaagattt aaataccatg ctaaacacag tggggggaca tcaagcagcc 1380
   atgcaaatgt taaaagagac catcaatgag gaagctgcag aatgggatag attgcatcca 1440
   gtgcatgcag ggcctattgc accaggccag atgagagaac caaggggaag tgacatagca 1500
   ggaactacta gtacccttca ggaacaaata ggatggatga cacataatcc acctatccca 1560
   gtaggagaaa tctataaaag atggataatc ctgggattaa ataaaatagt aagaatgtat 1620
   agccctacca gcattctgga cataagacaa ggaccaaagg aaccctttag agactatgta 1680
   gaccgattct ataaaactct aagagccgag caagcttcac aagaggtaaa aaattggatg 1740
   acagaaacct tgttggtcca aaatgcgaac ccagattgta agactatttt aaaagcattg 1800
   ggaccaggag cgacactaga agaaatgatg acagcatgtc agggagtggg gggacccggc 1860
   cataaagcaa gagttttggc tgaagcaatg agccaagtaa caaatccagc taccataatg 1920
   atacagaaag gcaattttag gaaccaaaga aagactgtta agtgtttcaa ttgtggcaaa 1980
   gaagggcaca tagccaaaaa ttgcagggcc cctaggaaaa agggctgttg gaaatgtgga 2040
   aaggaaggac accaaatgaa agattgtact gagagacagg ctaatttttt agggaagatc 2100
   tggccttccc acaagggaag gccagggaat tttcttcaga gcagaccaga gccaacagcc 2160
   ccaccagaag agagcttcag gtttggggaa gagacaacaa ctccctctca gaagcaggag 2220
   ccgatagaca aggaactgta tcctttagct tccctcagat cactctttgg cagcgacccc 2280
   tcgtcacaat aaagataggg gggcaattaa aggaagctct attagataca ggagcagatg 2340
   atacagtatt agaagaaatg aatttgccag gaagatggaa accaaaaatg atagggggaa 2400
   ttggaggttt tatcaaagta ggacagtatg atcagatact catagaaatc tgcggacata 2460
   aagctatagg tacagtatta gtaggaccta cacctgtcaa cataattgga agaaatctgt 2520
   tgactcagat tggctgcact ttaaattttc ccattagtcc tattgagact gtaccagtaa 2580
   aattaaagcc aggaatggat ggcccaaaag ttaaacaatg gccattgaca gaagaaaaaa 2640
   taaaagcatt agtagaaatt tgtacagaaa tggaaaagga aggaaaaatt tcaaaaattg 2700
   ggcctgaaaa tccatacaat actccagtat ttgccataaa gaaaaaagac agtactaaat 2760
   ggagaaaatt agtagatttc agagaactta ataagagaac tcaagatttc tgggaagttc 2820
   aattaggaat accacatcct gcagggttaa aacagaaaaa atcagtaaca gtactggatg 2880
   tgggcgatgc atatttttca gttcccttag ataaagactt caggaagtat actgcattta 2940
   ccatacctag tataaacaat gagacaccag ggattagata tcagtacaat gtgcttccac 3000
   agggatggaa aggatcacca gcaatattcc agtgtagcat gacaaaaatc ttagagcctt 3060
   ttagaaaaca aaatccagac atagtcatct atcaatacat ggatgatttg tatgtaggat 3120
   ctgacttaga aatagggcag catagaacaa aaatagagga actgagacaa catctgttga 3180
   ggtggggatt taccacacca gacaaaaaac atcagaaaga acctccattc ctttggatgg 3240
   gttatgaact ccatcctgat aaatggacag tacagcctat agtgctgcca gaaaaggaca 3300
   gctggactgt caatgacata cagaaattag tgggaaaatt gaattgggca agtcagattt 3360
   atgcagggat taaagtaagg caattatgta aacttcttag gggaaccaaa gcactaacag 3420
   aagtagtacc actaacagaa gaagcagagc tagaactggc agaaaacagg gagattctaa 3480
   aagaaccggt acatggagtg tattatgacc catcaaaaga cttaatagca gaaatacaga 3540
   agcaggggca aggccaatgg acatatcaaa tttatcaaga gccatttaaa aatctgaaaa 3600
   caggaaaata tgcaagaatg aagggtgccc acactaatga tgtgaaacaa ttaacagagg 3660
   cagtacaaaa aatagccaca gaaagcatag taatatgggg aaagactcct aaatttaaat 3720
   tacccataca aaaggaaaca tgggaagcat ggtggacaga gtattggcaa gccacctgga 3780
   ttcctgagtg ggagtttgtc aatacccctc ccttagtgaa gttatggtac cagttagaga 3840
   aagaacccat aataggagca gaaactttct atgtagatgg ggcagccaat agggaaacta 3900
   aattaggaaa agcaggatat gtaactgaca gaggaagaca aaaagttgtc cccctaacgg 3960
   acacaacaaa tcagaagact gagttacaag caattcatct agctttgcag gattcgggat 4020
   tagaagtaaa catagtgaca gactcacaat atgcattggg aatcattcaa gcacaaccag 4080
   ataagagtga atcagagtta gtcagtcaaa taatagagca gttaataaaa aaggaaaaag 4140
   tctacctggc atgggtacca gcacacaaag gaattggagg aaatgaacaa gtagatgggt 4200
   tggtcagtgc tggaatcagg aaagtactat ttttagatgg aatagataag gcccaagaag 4260
   aacatgagaa atatcacagt aattggagag caatggctag tgattttaac ctaccacctg 4320
   tagtagcaaa agaaatagta gccagctgtg ataaatgtca gctaaaaggg gaagccatgc 4380
   atggacaagt agactgtagc ccaggaatat ggcagctaga ttgtacacat ttagaaggaa 4440
   aagttatctt ggtagcagtt catgtagcca gtggatatat agaagcagaa gtaattccag 4500
   cagagacagg gcaagaaaca gcatacttcc tcttaaaatt agcaggaaga tggccagtaa 4560
   aaacagtaca tacagacaat ggcagcaatt tcaccagtac tacagttaag gccgcctgtt 4620
   ggtgggcggg gatcaagcag gaatttggca ttccctacaa tccccaaagt caaggagtaa 4680
   tagaatctat gaataaagaa ttaaagaaaa ttataggaca ggtaagagat caggctgaac 4740
   atcttaagac agcagtacaa atggcagtat tcatccacaa ttttaaaaga aaagggggga 4800
   ttggggggta cagtgcaggg gaaagaatag tagacataat agcaacagac atacaaacta 4860
   aagaattaca aaaacaaatt acaaaaattc aaaattttcg ggtttattac agggacagca 4920
   gagatccagt ttggaaagga ccagcaaagc tcctctggaa aggtgaaggg gcagtagtaa 4980
   tacaagataa tagtgacata aaagtagtgc caagaagaaa agcaaagatc atcagggatt 5040
   atggaaaaca gatggcaggt gatgattgtg tggcaagtag acaggatgag gattaacaca 5100
   tggaaaagat tagtaaaaca ccatatgtat atttcaagga aagctaagga ctggttttat 5160
   agacatcact atgaaagtac taatccaaaa ataagttcag aagtacacat cccactaggg 5220
   gatgctaaat tagtaataac aacatattgg ggtctgcata caggagaaag agactggcat 5280
   ttgggtcagg gagtctccat agaatggagg aaaaagagat atagcacaca agtagaccct 5340
   gacctagcag accaactaat tcatctgcac tattttgatt gtttttcaga atctgctata 5400
   agaaatacca tattaggacg tatagttagt cctaggtgtg aatatcaagc aggacataac 5460
   aaggtaggat ctctacagta cttggcacta gcagcattaa taaaaccaaa acagataaag 5520
   ccacctttgc ctagtgttag gaaactgaca gaggacagat ggaacaagcc ccagaagacc 5580
   aagggccaca gagggagcca tacaatgaat ggacactaga gcttttagag gaacttaaga 5640
   gtgaagctgt tagacatttt cctaggatat ggctccataa cttaggacaa catatctatg 5700
   aaacttacgg ggatacttgg gcaggagtgg aagccataat aagaattctg caacaactgc 5760
   tgtttatcca tttcagaatt gggtgtcgac atagcagaat aggcgttact cgacagagga 5820
   gagcaagaaa tggagccagt agatcctaga ctagagccct ggaagcatcc aggaagtcag 5880
   cctaaaactg cttgtaccaa ttgctattgt aaaaagtgtt gctttcattg ccaagtttgt 5940
   ttcatgacaa aagccttagg catctcctat ggcaggaaga agcggagaca gcgacgaaga 6000
   gctcatcaga acagtcagac tcatcaagct tctctatcaa agcagtaagt agtacatgta 6060
   atgcaaccta taatagtagc aatagtagca ttagtagtag caataataat agcaatagtt 6120
   gtgtggtcca tagtaatcat agaatatagg aaaatattaa gacaaagaaa aatagacagg 6180
   ttaattgata gactaataga aagagcagaa gacagtggca atgagagtga aggagaagta 6240
   tcagcacttg tggagatggg ggtggaaatg gggcaccatg ctccttggga tattgatgat 6300
   ctgtagtgct acagaaaaat tgtgggtcac agtctattat ggggtacctg tgtggaagga 6360
   agcaaccacc actctatttt gtgcatcaga tgctaaagca tatgatacag aggtacataa 6420
   tgtttgggcc acacatgcct gtgtacccac agaccccaac ccacaagaag tagtattggt 6480
   aaatgtgaca gaaaatttta acatgtggaa aaatgacatg gtagaacaga tgcatgagga 6540
   tataatcagt ttatgggatc aaagcctaaa gccatgtgta aaattaaccc cactctgtgt 6600
   tagtttaaag tgcactgatt tgaagaatga tactaatacc aatagtagta gcgggagaat 6660
   gataatggag aaaggagaga taaaaaactg ctctttcaat atcagcacaa gcataagaga 6720
   taaggtgcag aaagaatatg cattctttta taaacttgat atagtaccaa tagataatac 6780
   cagctatagg ttgataagtt gtaacacctc agtcattaca caggcctgtc caaaggtatc 6840
   ctttgagcca attcccatac attattgtgc cccggctggt tttgcgattc taaaatgtaa 6900
   taataagacg ttcaatggaa caggaccatg tacaaatgtc agcacagtac aatgtacaca 6960
   tggaatcagg ccagtagtat caactcaact gctgttaaat ggcagtctag cagaagaaga 7020
   tgtagtaatt agatctgcca atttcacaga caatgctaaa accataatag tacagctgaa 7080
   cacatctgta gaaattaatt gtacaagacc caacaacaat acaagaaaaa gtatccgtat 7140
   ccagagggga ccagggagag catttgttac aataggaaaa ataggaaata tgagacaagc 7200
   acattgtaac attagtagag caaaatggaa tgccacttta aaacagatag ctagcaaatt 7260
   aagagaacaa tttggaaata ataaaacaat aatctttaag caatcctcag gaggggaccc 7320
   agaaattgta acgcacagtt ttaattgtgg aggggaattt ttctactgta attcaacaca 7380
   actgtttaat agtacttggt ttaatagtac ttggagtact gaagggtcaa ataacactga 7440
   aggaagtgac acaatcacac tcccatgcag aataaaacaa tttataaaca tgtggcagga 7500
   agtaggaaaa gcaatgtatg cccctcccat cagtggacaa attagatgtt catcaaatat 7560
   tactgggctg ctattaacaa gagatggtgg taataacaac aatgggtccg agatcttcag 7620
   acctggagga ggcgatatga gggacaattg gagaagtgaa ttatataaat ataaagtagt 7680
   aaaaattgaa ccattaggag tagcacccac caaggcaaag agaagagtgg tgcagagaga 7740
   aaaaagagca gtgggaatag gagctttgtt ccttgggttc ttgggagcag caggaagcac 7800
   tatgggctgc acgtcaatga cgctgacggt acaggccaga caattattgt ctgatatagt 7860
   gcagcagcag aacaatttgc tgagggctat tgaggcgcaa cagcatctgt tgcaactcac 7920
   agtctggggc atcaaacagc tccaggcaag aatcctggct gtggaaagat acctaaagga 7980
   tcaacagctc ctggggattt ggggttgctc tggaaaactc atttgcacca ctgctgtgcc 8040
   ttggaatgct agttggagta ataaatctct ggaacagatt tggaataaca tgacctggat 8100
   ggagtgggac agagaaatta acaattacac aagcttaata cactccttaa ttgaagaatc 8160
   gcaaaaccag caagaaaaga atgaacaaga attattggaa ttagataaat gggcaagttt 8220
   gtggaattgg tttaacataa caaattggct gtggtatata aaattattca taatgatagt 8280
   aggaggcttg gtaggtttaa gaatagtttt tgctgtactt tctatagtga atagagttag 8340
   gcagggatat tcaccattat cgtttcagac ccacctccca atcccgaggg gacccgacag 8400
   gcccgaagga atagaagaag aaggtggaga gagagacaga gacagatcca ttcgattagt 8460
   gaacggatcc ttagcactta tctgggacga tctgcggagc ctgtgcctct tcagctacca 8520
   ccgcttgaga gacttactct tgattgtaac gaggattgtg gaacttctgg gacgcagggg 8580
   gtgggaagcc ctcaaatatt ggtggaatct cctacagtat tggagtcagg aactaaagaa 8640
   tagtgctgtt aacttgctca atgccacagc catagcagta gctgagggga cagatagggt 8700
   tatagaagta ttacaagcag cttatagagc tattcgccac atacctagaa gaataagaca 8760
   gggcttggaa aggattttgc tataagatgg gtggcaagtg gtcaaaaagt agtgtgattg 8820
   gatggcctgc tgtaagggaa agaatgagac gagctgagcc agcagcagat ggggtgggag 8880
   cagtatctcg agacctagaa aaacatggag caatcacaag tagcaataca gcagctaaca 8940
   atgctgcttg tgcctggcta gaagcacaag aggaggaaga ggtgggtttt ccagtcacac 9000
   ctcaggtacc tttaagacca atgacttaca aggcagctgt agatcttagc cactttttaa 9060
   aagaaaaggg gggactggaa gggctaattc actcccaaag aagacaagat atccttgatc 9120
   tgtggatcta ccacacacaa ggctacttcc ctgattggca gaactacaca ccagggccag 9180
   gggtcagata tccactgacc tttggatggt gctacaagct agtaccagtt gagccagata 9240
   aggtagaaga ggccaataaa ggagagaaca ccagcttgtt acaccctgtg agcctgcatg 9300
   gaatggatga ccctgagaga gaagtgttag agtggaggtt tgacagccgc ctagcatttc 9360
   atcacgtggc ccgagagctg catccggagt acttcaagaa ctgctgacat cgagcttgct 9420
   acaagggact ttccgctggg gactttccag ggaggcgtgg cctgggcggg actggggagt 9480
   ggcgagccct cagatgctgc atataagcag ctgctttttg cctgtactgg gtctctctgg 9540
   ttagaccaga tctgagcctg ggagctctct ggctaactag ggaacccact gcttaagcct 9600
   caataaagct tgccttgagt gcttcaagta gtgtgtgccc gtctgttgtg tgactctggt 9660
   aactagagat ccctcagacc cttttagtca gtgtggaaaa tctctagca            9709
<210> 2
   <211> 854
   <212> PRT
   <213> Human immunodeficiency virus
<220>
   <223> Envelope Polyprotein
<400> 2
   Met Arg Val Lys Glu Lys Tyr Gln His Leu Trp Arg Trp Gly Trp Lys
   1               5                   10                  15
   Trp Gly Thr Met Leu Leu Gly Ile Leu Met Ile Cys Ser Ala Thr Glu
               20                  25                  30
   Lys Leu Trp Val Thr Val Tyr Tyr Gly Val Pro Val Trp Lys Glu Ala
           35                  40                  45
   Thr Thr Thr Leu Phe Cys Ala Ser Asp Ala Lys Ala Tyr Asp Thr Glu
       50                  55                  60
   Val His Asn Val Trp Ala Thr His Ala Cys Val Pro Thr Asp Pro Asn
   65                  70                  75                  80
   Pro Gln Glu Val Val Leu Val Asn Val Thr Glu Asn Phe Asn Met Trp
                   85                  90                  95
   Lys Asn Asp Met Val Glu Gln Met His Glu Asp Ile Ile Ser Leu Trp
               100                 105                 110
   Asp Gln Ser Leu Lys Pro Cys Val Lys Leu Thr Pro Leu Cys Val Ser
           115                 120                 125
   Leu Lys Cys Thr Asp Leu Lys Asn Asp Thr Asn Thr Asn Ser Ser Ser
       130                 135                 140
   Gly Arg Met Ile Met Glu Lys Gly Glu Ile Lys Asn Cys Ser Phe Asn
   145                 150                 155                 160
   Ile Ser Thr Ser Ile Arg Asp Lys Val Gln Lys Glu Tyr Ala Phe Phe
                   165                 170                 175
   Tyr Lys Leu Asp Ile Val Pro Ile Asp Asn Thr Ser Tyr Arg Leu Ile
               180                 185                 190
   Ser Cys Asn Thr Ser Val Ile Thr Gln Ala Cys Pro Lys Val Ser Phe
           195                 200                 205
   Glu Pro Ile Pro Ile His Tyr Cys Ala Pro Ala Gly Phe Ala Ile Leu
       210                 215                 220
   Lys Cys Asn Asn Lys Thr Phe Asn Gly Thr Gly Pro Cys Thr Asn Val
   225                 230                 235                 240
   Ser Thr Val Gln Cys Thr His Gly Ile Arg Pro Val Val Ser Thr Gln
                   245                 250                 255
   Leu Leu Leu Asn Gly Ser Leu Ala Glu Glu Asp Val Val Ile Arg Ser
               260                 265                 270
   Ala Asn Phe Thr Asp Asn Ala Lys Thr Ile Ile Val Gln Leu Asn Thr
           275                 280                 285
   Ser Val Glu Ile Asn Cys Thr Arg Pro Asn Asn Asn Thr Arg Lys Ser
       290                 295                 300
   Ile Arg Ile Gln Arg Gly Pro Gly Arg Ala Phe Val Thr Ile Gly Lys
   305                 310                 315                 320
   Ile Gly Asn Met Arg Gln Ala His Cys Asn Ile Ser Arg Ala Lys Trp
                   325                 330                 335
   Asn Ala Thr Leu Lys Gln Ile Ala Ser Lys Leu Arg Glu Gln Phe Gly
               340                 345                 350
   Asn Asn Lys Thr Ile Ile Phe Lys Gln Ser Ser Gly Gly Asp Pro Glu
           355                 360                 365
   Ile Val Thr His Ser Phe Asn Cys Gly Gly Glu Phe Phe Tyr Cys Asn
       370                 375                 380
   Ser Thr Gln Leu Phe Asn Ser Thr Trp Phe Asn Ser Thr Trp Ser Thr
   385                 390                 395                 400
   Glu Gly Ser Asn Asn Thr Glu Gly Ser Asp Thr Ile Thr Leu Pro Cys
                   405                 410                 415
   Arg Ile Lys Gln Phe Ile Asn Met Trp Gln Glu Val Gly Lys Ala Met
               420                 425                 430
   Tyr Ala Pro Pro Ile Ser Gly Gln Ile Arg Cys Ser Ser Asn Ile Thr
           435                 440                 445
   Gly Leu Leu Leu Thr Arg Asp Gly Gly Asn Asn Asn Asn Gly Ser Glu
       450                 455                 460
   Ile Phe Arg Pro Gly Gly Gly Asp Met Arg Asp Asn Trp Arg Ser Glu
   465                 470                 475                 480
   Leu Tyr Lys Tyr Lys Val Val Lys Ile Glu Pro Leu Gly Val Ala Pro
                   485                 490                 495
   Thr Lys Ala Lys Arg Arg Val Val Gln Arg Glu Lys Arg Ala Val Gly
               500                 505                 510
   Ile Gly Ala Leu Phe Leu Gly Phe Leu Gly Ala Ala Gly Ser Thr Met
           515                 520                 525
   Gly Cys Thr Ser Met Thr Leu Thr Val Gln Ala Arg Gln Leu Leu Ser
       530                 535                 540
   Asp Ile Val Gln Gln Gln Asn Asn Leu Leu Arg Ala Ile Glu Ala Gln
   545                 550                 555                 560
   Gln His Leu Leu Gln Leu Thr Val Trp Gly Ile Lys Gln Leu Gln Ala
                   565                 570                 575
   Arg Ile Leu Ala Val Glu Arg Tyr Leu Lys Asp Gln Gln Leu Leu Gly
               580                 585                 590
   Ile Trp Gly Cys Ser Gly Lys Leu Ile Cys Thr Thr Ala Val Pro Trp
           595                 600                 605
   Asn Ala Ser Trp Ser Asn Lys Ser Leu Glu Gln Ile Trp Asn Asn Met
       610                 615                 620
   Thr Trp Met Glu Trp Asp Arg Glu Ile Asn Asn Tyr Thr Ser Leu Ile
   625                 630                 635                 640
   His Ser Leu Ile Glu Glu Ser Gln Asn Gln Gln Glu Lys Asn Glu Gln
                   645                 650                 655
   Glu Leu Leu Glu Leu Asp Lys Trp Ala Ser Leu Trp Asn Trp Phe Asn
               660                 665                 670
   Ile Thr Asn Trp Leu Trp Tyr Ile Lys Leu Phe Ile Met Ile Val Gly
           675                 680                 685
   Gly Leu Val Gly Leu Arg Ile Val Phe Ala Val Leu Ser Ile Val Asn
       690                 695                 700
   Arg Val Arg Gln Gly Tyr Ser Pro Leu Ser Phe Gln Thr His Leu Pro
   705                 710                 715                 720
   Ile Pro Arg Gly Pro Asp Arg Pro Glu Gly Ile Glu Glu Glu Gly Gly
                   725                 730                 735
   Glu Arg Asp Arg Asp Arg Ser Ile Arg Leu Val Asn Gly Ser Leu Ala
               740                 745                 750
   Leu Ile Trp Asp Asp Leu Arg Ser Leu Cys Leu Phe Ser Tyr His Arg
           755                 760                 765
   Leu Arg Asp Leu Leu Leu Ile Val Thr Arg Ile Val Glu Leu Leu Gly
       770                 775                 780
   Arg Arg Gly Trp Glu Ala Leu Lys Tyr Trp Trp Asn Leu Leu Gln Tyr
   785                 790                 795                 800
   Trp Ser Gln Glu Leu Lys Asn Ser Ala Val Asn Leu Leu Asn Ala Thr
                   805                 810                 815
   Ala Ile Ala Val Ala Glu Gly Thr Asp Arg Val Ile Glu Val Leu Gln
               820                 825                 830
   Ala Ala Tyr Arg Ala Ile Arg His Ile Pro Arg Arg Ile Arg Gln Gly
           835                 840                 845
   Leu Glu Arg Ile Leu Leu
       850
<210> 3
   <211> 107
   <212> DNA
   <213> Kuenstliche Sequenz
<220>
   <223> Beschreibung der kuenstlichen Sequenz: Oligonukleotid fuer Klonierung
<400> 3
   aagatgtagt aattagatct gccaatttca cagacaatgc taaaaccata atagtacagc 60
   tgaacacatc gttagaaatt aattgtacaa gacccaacaa caataca               107
<210> 4
   <211> 120
   <212> DNA
   <213> Kuenstliche Sequenz
<220>
   <223> Beschreibung der kuenstlichen Sequenz: Oligonukleotid fuer Klonierung
<220>
   <221> misc_feature
   <222> (97)..(99)
   <223> Sequenz an dieser Position: (GA)(AT)(GATC), d.h. Base an Position 97 kann G oder A sein, Base an Position 98 kann A oder T sein, und Base an Position 99 kann G, A, T oder C sein.
<400> 4
   ttttgctcta gaaatgttac aatgtgcttg tcttatgtct cctgttgcag cttctgttgc 60
   atgaaatgct ctccctggtc cgatatggat actatgrwnt tttcttgtat tgttgttggg 120
<210> 5
   <211> 17
   <212> DNA
   <213> Kuenstliche Sequenz
<220>
   <223> Beschreibung der kuenstlichen Sequenz: Sequenzierungsprimer
<400> 5
   ccatgtacaa atgtcag 17
<210> 6
   <211> 17
   <212> DNA
   <213> Kuenstliche Sequenz
<220>
   <223> Beschreibung der kuenstlichen Sequenz: Sequenzierungsprimer
<400> 6
   aaaactgtgc gttacaa 17
<210> 7
   <211> 17
   <212> DNA
   <213> Kuenstliche Sequenz
<220>
   <223> Beschreibung der kuenstlichen Sequenz: Sequenzierungsprimer
<400> 7
   gtaaaacgac ggccagt 17
<210> 8
   <211> 17
   <212> DNA
   <213> Kuenstliche Sequenz
<220>
   <223> Beschreibung der kuenstlichen Sequenz: Sequenzierungsprimer
<400> 8
   caggaaacag ctatgac 17
<210> 9
   <211> 2148
   <212> DNA
   <213> Kuenstliche Sequenz
<220>
   <223> Beschreibung der kuenstlichen Sequenz: Synthetische DNA
<220>
   <221> misc_feature
   <222> (3)..(9)
   <223> BstEII-Schnittstelle
<220>
   <221> misc_feature
   <222> (2143)..(2148)
   <223> BamHI-Schnittstelle
<400> 9
   tgggtcaccg tctattatgg ggtgcctgtg tggaaggaag caaccaccac tctattttgt 60
   gcatcagatg ctaaagcata tgatacagag gtacataatg tttgggccac acatgcctgt 120
   gtacccacag accccaaccc acaagaagta gtattggtaa atgtgacaga aaattttaac 180
   atgtggaaaa atgacatggt agaacagatg catgaggata taatcagttt atgggatcaa 240
   agccttaagc catgtgtaaa attaacccca ctctgtgtta gtttaaagtg cactgatttg 300
   aagaatgata ctaataccaa tagtagtagc gggagaatga taatggagaa aggagagata 360
   aaaaactgca gcttcaatat cagcacaagc ataagagata aggtgcagaa agaatatgca 420
   ttcttttata aacttgatat agtaccaata gataatacca gctataggtt gataagttgt 480
   aacacctcag tgatcacaca ggcctgtcca aaggtatcct ttgagccaat tcccatacat 540
   tattgtgccc cggctggttt tgcgattcta aaatgtaata ataagacgtt caatggaaca 600
   ggaccatgta caaatgtcag cacagtacaa tgtacacatg gaattcgacc agtagtatca 660
   actcaactgc tgttaaatgg cagtctagca gaagaagatg tagtaattag atctgccaat 720
   ttcacagaca atgctaaaac cataatagta cagctgaaca catctgtaga aattaattgt 780
   acaagaccca acaacaatac aagaaaaagt atccgtatcc agaggggacc agggagagca 840
   tttgttacaa taggaaaaat aggaaatatg agacaagcac attgtaacat ttctagagca 900
   aaatggaatg ccactttaaa acagatagct agcaaattaa gagaacaatt tggaaataat 960
   aaaacaataa tctttaagca gtcatccgga ggggacccag aaattgtaac gcacagtttt 1020
   aattgtggag gggaattttt ctactgtaat tcaacacaac tgtttaatag tacttggttt 1080
   aatagtactt ggagtactga agggtcaaat aacactgaag gaagtgacac aatcacactc 1140
   ccatgcagaa taaaacaatt tataaacatg tggcaggaag taggaaaagc aatgtatgcc 1200
   cctcccatca gtggccaaat tagatgttca tcaaatatta ctgggctgct attaactcga 1260
   gatggtggta ataacaacaa tgggtccgag attttcagac ctggaggagg cgatatgagg 1320
   gataattgga gaagtgaatt atataaatat aaagtagtaa aaattgaacc attaggagta 1380
   gcacccacca aggcaaagag acgcgtggtg cagagagaaa agcgcgcagt gggaatagga 1440
   gctctgttcc ttgggttctt gggagcagca ggaagcacta tgggcgcagc gtcaatgacg 1500
   ctgacggtac aggccagaca attattgtct gatatagtgc agcagcagaa caatttgctg 1560
   agggcaattg aggcgcaaca gcatctgttg caactcacag tctggggcat caaacagctc 1620
   caggcaagaa tcctggctgt ggaaagatac ctaaaggatc aacagctcct ggggatttgg 1680
   ggttgctctg gaaaactcat ttgcaccact gctgtgcctt ggaatgctag ttggagtaat 1740
   aaatctctgg aacagatttg gaataacatg acctggatgg agtgggacag agaaattaac 1800
   aattacacaa gcttaataca ctccttaatt gaagaatcgc aaaaccagca agaaaagaat 1860
   gaacaagaat tattggaatt agataaatgg gcaagtttgt ggaattggtt taacataaca 1920
   aattggctgt ggtatataaa attattcata atgatagtag gaggcttggt aggtttaaga 1980
   atagtttttg ctgtactttc tatagtgaat agagttaggc agggatattc accattatcg 2040
   tttcagaccc acctcccaat cccgagggga cccgacaggc ccgaaggaat agaagaagaa 2100
   ggtggagaga gagacagaga cagatccatt cgattagtga acggatcc             2148
<210> 10
   <211> 6229
   <212> DNA
   <213> Kuenstliche Sequenz
<220>
   <223> Beschreibung der kuenstlichen Sequenz: Synthetische DNA
<220>
   <221> sig_peptide
   <222> (1293)..(1295)
   <223> env ATG
<220>
   <221> misc_feature
   <222> (1377)..(1379)
   <223> env AGT, gp120 Anfang
<220>
   <221> misc_feature
   <222> (1397)..(1403)
   <223> BstEII-Schnittstelle
<220>
   <221> misc_feature
   <222> (3537)..(3542)
   <223> BamHI-Schnittstelle
<220>
   <221> misc_feature
   <222> (3855)..(3857)
   <223> env TAA, Stop
<400> 10
   ctgacgcgcc ctgtagcggc gcattaagcg cggcgggtgt ggtggttacg cgcagcgtga 60
   ccgctacact tgccagcgcc ctagcgcccg ctcctttcgc tttcttccct tcctttctcg 120
   ccacgttcgc cggctttccc cgtcaagctc taaatcgggg gctcccttta gggttccgat 180
   ttagtgcttt acggcacctc gaccccaaaa aacttgatta gggtgatggt tcacgtagtg 240
   ggccatcgcc ctgatagacg gtttttcgcc ctttgacgtt ggagtccacg ttctttaata 300
   gtggactctt gttccaaact ggaacaacac tcaaccctat ctcggtctat tcttttgatt 360
   tataagggat tttgccgatt tcggcctatt ggttaaaaaa tgagctgatt taacaaaaat 420
   ttaacgcgaa ttttaacaaa atattaacgc ttacaatttc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gtctagttat taatagtaat caattacggg gtcattagtt 660
   catagcccat atatggagtt ccgcgttaca taacttacgg taaatggccc gcctggctga 720
   ccgcccaacg acccccgccc attgacgtca ataatgacgt atgttcccat agtaacgcca 780
   atagggactt tccattgacg tcaatgggtg gagtatttac ggtaaactgc ccacttggca 840
   gtacatcaag tgtatcatat gccaagtacg ccccctattg acgtcaatga cggtaaatgg 900
   cccgcctggc attatgccca gtacatgacc ttatgggact ttcctacttg gcagtacatc 960
   tacgtattag tcatcgctat taccatggtg atgcggtttt ggcagtacat caatgggcgt 1020
   ggatagcggt ttgactcacg gggatttcca agtctccacc ccattgacgt caatgggagt 1080
   ttgttttggc accaaaatca acgggacttt ccaaaatgtc gtaacaactc cgccccattg 1140
   acgcaaatgg gcggtaggcg tgtacggtgg gaggtctata taagcagagc tcgtttagtg 1200
   aaccgtcaga tcgcctggag acgccatcca cgctgttttg acctccatag aagacaccgg 1260
   gacaattcga gctcggtacc gtcgacgcca ccatgagagt gaaggagaag tatcagcact 1320
   tgtggagatg ggggtggaaa tggggcacca tgctccttgg gatattgatg atctgtagtg 1380
   ctacagaaaa attgtgggtc accgtctatt atggggtacc tgtgtggaag gaagcaacca 1440
   ccactctatt ttgtgcatca gatgctaaag catatgatac agaggtacat aatgtttggg 1500
   ccacacatgc ctgtgtaccc acagacccca acccacaaga agtagtattg gtaaatgtga 1560
   cagaaaattt taacatgtgg aaaaatgaca tggtagaaca gatgcatgag gatataatca 1620
   gtttatggga tcaaagccta aagccatgtg taaaattaac cccactctgt gttagtttaa 1680
   agtgcactga tttgaagaat gatactaata ccaatagtag tagcgggaga atgataatgg 1740
   agaaaggaga gataaaaaac tgctctttca atatcagcac aagcataaga gataaggtgc 1800
   agaaagaata tgcattcttt tataaacttg atatagtacc aatagataat accagctata 1860
   ggttgataag ttgtaacacc tcagtcatta cacaggcctg tccaaaggta tcctttgagc 1920
   caattcccat acattattgt gccccggctg gttttgcgat tctaaaatgt aataataaga 1980
   cgttcaatgg aacaggacca tgtacaaatg tcagcacagt acaatgtaca catggaatca 2040
   ggccagtagt atcaactcaa ctgctgttaa atggcagtct agcagaagaa gatgtagtaa 2100
   ttagatctgc caatttcaca gacaatgcta aaaccataat agtacagctg aacacatctg 2160
   tagaaattaa ttgtacaaga cccaacaaca atacaagaaa aagtatccgt atccagaggg 2220
   gaccagggag agcatttgtt acaataggaa aaataggaaa tatgagacaa gcacattgta 2280
   acattagtag agcaaaatgg aatgccactt taaaacagat agctagcaaa ttaagagaac 2340
   aatttggaaa taataaaaca ataatcttta agcaatcctc aggaggggac ccagaaattg 2400
   taacgcacag ttttaattgt ggaggggaat ttttctactg taattcaaca caactgttta 2460
   atagtacttg gtttaatagt acttggagta ctgaagggtc aaataacact gaaggaagtg 2520
   acacaatcac actcccatgc agaataaaac aatttataaa catgtggcag gaagtaggaa 2580
   aagcaatgta tgcccctccc atcagtggac aaattagatg ttcatcaaat attactgggc 2640
   tgctattaac aagagatggt ggtaataaca acaatgggtc cgagatcttc agacctggag 2700
   gaggcgatat gagggacaat tggagaagtg aattatataa atataaagta gtaaaaattg 2760
   aaccattagg agtagcaccc accaaggcaa agagaagagt ggtgcagaga gaaaaaagag 2820
   cagtgggaat aggagctttg ttccttgggt tcttgggagc agcaggaagc actatgggct 2880
   gcacgtcaat gacgctgacg gtacaggcca gacaattatt gtctgatata gtgcagcagc 2940
   agaacaattt gctgagggct attgaggcgc aacagcatct gttgcaactc acagtctggg 3000
   gcatcaaaca gctccaggca agaatcctgg ctgtggaaag atacctaaag gatcaacagc 3060
   tcctggggat ttggggttgc tctggaaaac tcatttgcac cactgctgtg ccttggaatg 3120
   ctagttggag taataaatct ctggaacaga tttggaataa catgacctgg atggagtggg 3180
   acagagaaat taacaattac acaagcttaa tacactcctt aattgaagaa tcgcaaaacc 3240
   agcaagaaaa gaatgaacaa gaattattgg aattagataa atgggcaagt ttgtggaatt 3300
   ggtttaacat aacaaattgg ctgtggtata taaaattatt cataatgata gtaggaggct 3360
   tggtaggttt aagaatagtt tttgctgtac tttctatagt gaatagagtt aggcagggat 3420
   attcaccatt atcgtttcag acccacctcc caatcccgag gggacccgac aggcccgaag 3480
   gaatagaaga agaaggtgga gagagagaca gagacagatc cattcgatta gtgaacggat 3540
   ccttagcact tatctgggac gatctgcgga gcctgtgcct cttcagctac caccgcttga 3600
   gagacttact cttgattgta acgaggattg tggaacttct gggacgcagg gggtgggaag 3660
   ccctcaaata ttggtggaat ctcctacagt attggagtca ggaactaaag aatagtgctg 3720
   ttaacttgct caatgccaca gccatagcag tagctgaggg gacagatagg gttatagaag 3780
   tattacaagc agcttataga gctattcgcc acatacctag aagaataaga cagggcttgg 3840
   aaaggatttt gctataagat gggtggcaag tggtcaaaaa gtagtgtgat tggatggcct 3900
   gctgtaaggg aaagaatgag acgagctgag ccagcagcag atggggtggg agcagtatct 3960
   cgagatctag actagaacta gcttcgatcc agacatgata agatacattg atgagtttgg 4020
   acaaaccaca actagaatgc agtgaaaaaa atgctttatt tgtgaaattt gtgatgctat 4080
   tgctttattt gtaaccatta taagctgcaa taaacaagtt aacaacaaca attgcattca 4140
   ttttatgttt caggttcagg gggaggtgtg ggaggttttt taaagcaagt aaaacctcta 4200
   caaatgtggt atggctgatt atgatcctgc ctcgcgcgtt tcggtgatga cggtgaaaac 4260
   ctctgacaca tgcagctccc ggagacggtc acagcttgtc tgtaagcgga tgccgggagc 4320
   agacaagccc gtcagggcgc gtcagcgggt gttggcgggt gtcggggcgc agccatgacc 4380
   cagtcacgta gcgatagcgg agtgtatact ggcttaacta tgcggcatca gagcagattg 4440
   tactgagagt gcaccatatg tcgggccgcg ttgctggcgt ttttccatag gctccgcccc 4500
   cctgacgagc atcacaaaaa tcgacgctca agtcagaggt ggcgaaaccc gacaggacta 4560
   taaagatacc aggcgtttcc ccctggaagc tccctcgtgc gctctcctgt tccgaccctg 4620
   ccgcttaccg gatacctgtc cgcctttctc ccttcgggaa gcgtggcgct ttctcatagc 4680
   tcacgctgta ggtatctcag ttcggtgtag gtcgttcgct ccaagctggg ctgtgtgcac 4740
   gaaccccccg ttcagcccga ccgctgcgcc ttatccggta actatcgtct tgagtccaac 4800
   ccggtaagac acgacttatc gccactggca gcagccactg gtaacaggat tagcagagcg 4860
   aggtatgtag gcggtgctac agagttcttg aagtggtggc ctaactacgg ctacactaga 4920
   aggacagtat ttggtatctg cgctctgctg aagccagtta ccttcggaaa aagagttggt 4980
   agctcttgat ccggcaaaca aaccaccgct ggtagcggtg gtttttttgt ttgcaagcag 5040
   cagattacgc gcagaaaaaa aggatctcaa gaagatcctt tgatcttttc tacggggtct 5100
   gacgctcagt ggaacgaaaa ctcacgttaa gggattttgg tcatgagatt atcaaaaagg 5160
   atcttcacct agatcctttt aaattaaaaa tgaagtttta aatcaatcta aagtatatat 5220
   gagtaaactt ggtctgacag ttaccaatgc ttaatcagtg aggcacctat ctcagcgatc 5280
   tgtctatttc gttcatccat agttgcctga ctccccgtcg tgtagataac tacgatacgg 5340
   gagggcttac catctggccc cagtgctgca atgataccgc gagacccacg ctcaccggct 5400
   ccagatttat cagcaataaa ccagccagcc ggaagggccg agcgcagaag tggtcctgca 5460
   actttatccg cctccatcca gtctattaat tgttgccggg aagctagagt aagtagttcg 5520
   ccagttaata gtttgcgcaa cgttgttgcc attgctacag gcatcgtggt gtcacgctcg 5580
   tcgtttggta tggcttcatt cagctccggt tcccaacgat caaggcgagt tacatgatcc 5640
   cccatgttgt gcaaaaaagc ggttagctcc ttcggtcctc cgatcgttgt cagaagtaag 5700
   ttggccgcag tgttatcact catggttatg gcagcactgc ataattctct tactgtcatg 5760
   ccatccgtaa gatgcttttc tgtgactggt gagtactcaa ccaagtcatt ctgagaatag 5820
   tgtatgcggc gaccgagttg ctcttgcccg gcgtcaatac gggataatac cgcgccacat 5880
   agcagaactt taaaagtgct catcattgga aaacgttctt cggggcgaaa actctcaagg 5940
   atcttaccgc tgttgagatc cagttcgatg taacccactc gtgcacccaa ctgatcttca 6000
   gcatctttta ctttcaccag cgtttctggg tgagcaaaaa caggaaggca aaatgccgca 6060
   aaaaagggaa taagggcgac acggaaatgt tgaatactca tactcttcct ttttcaatat 6120
   tattgaagca tttatcaggg ttattgtctc atgagcggat acatatttga atgtatttag 6180
   aaaaataaac aaataggggt tccgcgcaca tttccccgaa aagtgccac             6229
<210> 11
   <211> 860
   <212> DNA
   <213> Human immunodeficiency virus
<220>
   <221> misc_feature
   <222> (1)..(860)
   <223> PI-932 Originalsequenz V1-V2-V3-Loop
<400> 11
   tgtgtaccca cagaccccaa cccacaaaag gtagtattgg aaaatgtgac agaaaatttt 60
   aacatgtgga aaaatgacat ggtagaacag atgcatgagg atataatcaa tttatgggat 120
   caaagcctaa agccatgtgt aaaactaacc ccactctgtg ttactttaaa ttgcactgat 180
   gctgatttaa attgcaataa tactgattta aattgcacta aagctaattt ggggaaaaat 240
   actcataaca atactattag tgggaaaata atagagaaag tagaaataaa aaactgctct 300
   ttcaaggtca ccacaggcat aagggataag atgcaaaaag aatatgcact tttgaataaa 360
   cttgatatag taccaataga taatgataag aataatacta actttatatt gataagttgt 420
   aacacctcga ccattacaca ggcctgtcca aaggtatcct ttgagccaat tcccatacat 480
   ttttgtgccc cggctggttt tgcgattcta aagtgtaatg aaaagagtta cagtggaaaa 540
   ggaccatgta aaaatgtcag cacagtacaa tgtacacatg gaattaggcc agtagtgtca 600
   actcaactgc tgttgaatgg cagtctagca gaaaaagaag tagtaattag atctgagaat 660
   ttcacagaca atgctaaaac cataatagta cagctgaagg aatctgtaaa cattacttgt 720
   ataagacccc acaacactgt aacagacagg atacatatag ggccagggag atcatttcat 780
   acaacaagaa aaataaaagg agatataaga caagcacatt gtagccttag gagaaaagat 840
   tggaataaca ctttacaaga                                             860
<210> 12
   <211> 870
   <212> DNA
   <213> Kuenstliche Sequenz
<220>
   <223> Beschreibung der kuenstlichen Sequenz: PI-932 Genkassette, beinhaltet die Schnittstellen fuer die Restriktionsenzyme BspT1, PstI, BclI, EcoRI, BglII, PvuII, XbaI, NheI
<400> 12
   tgtgtaccca cagaccccaa cccacaaaag gtagtattgg aaaatgtgac agaaaatttt 60
   aacatgtgga aaaatgacat ggtagaacag atgcatgagg atataatcaa tttatgggat 120
   caaagcctta agccatgtgt aaaactaacc ccactctgtg ttactttaaa ttgcactgat 180
   gctgatttaa attgcaataa tactgattta aattgcacta aagctaattt ggggaaaaat 240
   actcataact gcagtattag tgggaaaata atagagaaag tagaaataaa aaactgctct 300
   ttcaaggtca ccacaggcat aagggataag atgcaaaaag aatatgcact tttgaataaa 360
   cttgatatag taccaataga taatgataag aataatacta actttatatt gataagttgt 420
   aacacctcgg tgatcacaca ggcctgtcca aaggtatcct ttgagccaat tcccatacat 480
   ttttgtgccc cggctggttt tgcgattcta aagtgtaatg aaaagagtta cagtggaaaa 540
   ggaccatgta aaaatgtcag cacagtacaa tgtacacatg gaattcggcc agtagtgtca 600
   actcaactgc tgttgaatgg cagtctagca gaaaaagaag tagtaattag atctgagaat 660
   ttcacagaca atgctaaaac cataatagta cagctgaagg aatctgtaaa cattacttgt 720
   ataagacccc acaacactgt aacagacagg atacatatag ggccagggag atcatttcat 780
   acaacaagaa aaataaaagg agatataaga caagcacatt gtagcctttc tagaaaagat 840
   tggaataaca ctttacaaga gatagctagc                                  870